# EUROPÄISCHE PATENTANMELDUNG

(11) **EP 3 770 241 A1**
(43) Veröffentlichungstag der Anmeldung: **27.01.2021**
(21) Anmeldenummer: 19187520.2
(22) Anmeldetag: 22.07.2019
(51) Int. Cl.: C11D 17/00, C11D 11/00, C11D 3/386

(54) **REINIGUNGSMITTEL MIT PROTEASE ZUR AUTOMATISCHEN DOSIERUNG**

(71) Anmelder: Henkel AG & Co. KGaA, 40589 Düsseldorf (DE)
(72) Erfinder: Doering, Thomas, 41540 Dormagen (DE); Weber, Thomas, 35096 Weimar (Lahn) (DE); Degering, Christian, 40699 Erkrath (DE); Wieland, Susanne, 41541 Zons/Dormagen (DE); Kempen, Brigitte, 40593 Düsseldorf (DE); Mussmann, Nina, 47877 Willich (DE)

(57) **Zusammenfassung**

Die Erfindung betrifft eine flüssige Reinigungsmittelzubereitung, welche 30 Gew.-% und weniger, vorzugsweise 25 Gew.-% und weniger, insbesondere 15 Gew.-% und weniger Wasser, und mindestens eine geeignete Protease umfasst, sowie Reinigungsmittelkombinationen, umfassend diese.

## Beschreibung

Die Erfindung betrifft eine flüssige Reinigungsmittelzubereitung, welche 30 Gew.-% und weniger, vorzugsweise 25 Gew.-% und weniger, insbesondere 15 Gew.-% und weniger Wasser, und mindestens eine geeignete Protease umfasst, sowie Reinigungsmittelkombinationen, umfassend diese.

Die Konfektions- und Angebotsformen von Reinigungsmitteln unterliegen immer neuen Änderungen. Ein Hauptaugenmerk liegt dabei seit geraumer Zeit auf der bequemen Dosierung von Reinigungsmitteln und der Vereinfachung der zur Durchführung eines Reinigungsverfahrens notwendigen Arbeitsschritte.

Insbesondere Vorrichtungen zur Mehrfachdosierung von Reinigungsmitteln werden vom Verbraucher gewünscht. Dabei können Vorrichtungen in zwischen in die Geschirrspülmaschine integrierten Dosierbehältern einerseits und eigenständigen, von der Geschirrspülmaschine unabhängigen Vorrichtungen andererseits unterschieden werden. Mittels dieser Vorrichtungen, welche die mehrfache der für die Durchführung eines Reinigungsverfahrens notwendigen Reinigungsmittelmenge enthalten, werden Reinigungsmittelportionen in automatischer oder halbautomatischer Weise im Verlauf mehrerer aufeinander folgender Reinigungsverfahren in den Innenraum der Reinigungsmaschine dosiert. Für den Verbraucher entfällt damit die Notwendigkeit der Dosierung des Reinigungsmittels vor Beginn jedes einzelnen Reinigungsgangs. Beispiele für derartige Vorrichtungen werden in der europäischen Patentanmeldung EP 1 759 624 A2 (Reckitt Benckiser) oder in der deutschen Patentanmeldung DE 10 2005 062 479 A1 (BSH Bosch und Siemens Hausgeräte GmbH) beschrieben.

Insbesondere wünschen Verbraucher mit eher geringem Spül-/Wäscheaufkommen eine Lösung, die unkompliziert und einfach zu bedienen ist.

Unabhängig von der exakten Bauart der im Innenraum von Geschirrspülmaschinen eingesetzten Dosiervorrichtungen sind die in diesen Vorrichtungen zur Mehrfachdosierung enthaltenen Reinigungsmittel darüber hinaus über eine längere Zeitdauer insbesondere wechselnden Temperaturen ausgesetzt, wobei diese Temperaturen in erster Näherung den zur Durchführung der Reinigungsverfahren eingesetzten Wassertemperaturen gleichen. Diese Temperaturen können bis zu 95°C betragen, wobei im Bereich der maschinellen Geschirrreinigung üblicherweise nur Temperaturen zwischen 50 und 75°C erreicht werden. Ein in einer zur Mehrfachdosierung vorgesehenen Vorrichtung enthaltenes Reinigungsmittel wird demnach im Verlaufe mehrerer Reinigungsverfahren wiederholt auf Temperaturen deutlich oberhalb der zum Transport und zur Lagerung üblichen Temperaturen erwärmt, wobei insbesondere temperaturempfindliche Aktivsubstanzen in Mitleidenschaft gezogen werden.

Insbesondere Verbraucher, die die Geschirrspülmaschine über mehrere Tage mit Geschirr beladen und bei denen das verschmutzte Spülgut einige Zeit ungereinigt im Inneren der Geschirrspülmaschine verbleibt, bevor ein Spülzyklus durchgeführt wird, haben das Problem, dass die Reinigungsleistung der Produkte, insbesondere bei hartnäckigen , insbesondere aufgetrockneten Speiseresten auf dem Spülgut, beispielsweise Haferflocken oder Tee, im Vergleich zum zeitnahen Spülen nach Benutzung des Spülguts zu wünschen übrig lässt. Das Abspülen von Speiseresten vor Einsortierung in die Spülmaschine ist aber aus ökologischen und zeitlichen Gründen sowie hinsichtlich der zusätzlichen Wasserkosten nicht vorteilhaft.

Die Aufgabe der vorliegenden Anmeldung bestand demnach in der Bereitstellung eines Reinigungsmittels / einer Reinigungsmittelangebotsform, umfassend ein solches, welches eine gute Reinigungsleistung, insbesondere auf schwierigen eingetrockneten Speiseresten, insbesondere Hackfleisch und/oder Eigelb und/oder Tee, liefert, auch wenn mehrere Tage zwischen den einzelnen Nutzungszyklen liegen. Dieses System soll insbesondere für den Verbraucher wenig aufwendig sein.

Ein erster Gegenstand der vorliegenden Anmeldung ist daher eine flüssige Reinigungsmittelzubereitung (bei 20°C, 1013 bar), bevorzugt Geschirrspülmittelzubereitung, insbesondere maschinelles Geschirrspülmittelzubereitung, bevorzugt phosphatfreie maschinelle Geschirrspülmittelzubereitung, welche 30 Gew.-% und weniger, vorzugsweise 25 Gew.-% und weniger, insbesondere 15 Gew.-% und weniger Wasser, und mindestens eine Protease umfasst, bevorzugt eine von Subtilisin abgeleitete Protease, und/oder insbesondere eine Protease, die ausgewählt ist aus der Gruppe von Proteasen gemäß (i) bis (v), wobei die mindestens eine Protease
(i) eine Aminosäuresequenz umfasst, die mindestens 70% Sequenzidentität mit der in SEQ ID NO:1 angegebenen Aminosäuresequenz über deren Gesamtlänge aufweist und die eine Aminosäuresubstitution an mindestens einer der Positionen, die den Positionen 12, 43, 122, 127, 154, 156, 160, 211, 212 oder 222, jeweils bezogen auf die Nummerierung gemäß SEQ ID NO:1, entsprechen, aufweist, insbesondere wobei die mindestens eine Aminosäuresubstitution aus der Gruppe bestehend aus Q12L, I43V, M122L, D127P, N154S, T156A, G160S, M211N, M211L, P212D, P212H oder A222S, jeweils bezogen auf die Nummerierung gemäß SEQ ID NO:1, ausgewählt ist, und/oder insbesondere bevorzugt eine der folgenden Aminosäuresubstitutionsvarianten, jeweils bezogen auf die Nummerierung gemäß SEQ ID NO:1, aufweist:
   a. I43V;
   b. M122L, N154S und T156A;
   c. M211N und P212D;
   d. M211L und P212D;
   e. G160S;
   f. D127P, M211L und P212D;
   g. P212H; oder
   h. Q12L, M122L und A222S;
(ii) eine Aminosäuresequenz umfasst, die ein Subtilisin 309 aus *Bacillus lentus* umfasst, insbesondere eine, die eine Aminosäuresequenz umfasst, die zu der in SEQ ID NO:2 angegebenen Aminosäuresequenz über deren Gesamtlänge zu mindestens 80%, bevorzugt zu mindestens 90 %, insbesondere zu 100 % identisch ist und mindestens eine Aminosäuresubstitution an einer der Positionen 9, 15, 66, 212 und 239 in der Zählung gemäß SEQ ID NO:2 aufweist, vorzugsweise ausgewählt aus der Gruppe bestehend aus S9R, A15T, V66A, N212D und Q239R;
iii) eine Aminosäuresequenz umfasst, die ein Subtilisin 309 aus *Bacillus lentus* umfasst, insbesondere eine, die eine Aminosäuresequenz umfasst, die zu der in SEQ ID NO:2 angegebenen Aminosäuresequenz über deren Gesamtlänge zu mindestens 80% bevorzugt zu mindestens 90 %, insbesondere zu 100 % identisch ist und eine Aminosäuresubstitution an der Position 97 und eine Insertion von einer Aminosäure zwischen den Aminosäuren an Positionen 97 und 98 in der Zählung gemäß SEQ ID NO:2 aufweist, vorzugsweise ausgewählt aus S97A und/oder S97AD;
iv) eine Aminosäuresequenz umfasst, die eine zu der in SEQ ID NO:3 angegebenen Aminosäuresequenz über deren Gesamtlänge zu mindestens 80%, bevorzugt zu mindestens 90 %, insbesondere zu 100 % identisch ist und die optional mindestens eine Aminosäuresubstitution an einer der folgenden Positionen 32, 33, 48-54, 58-62, 94-107, 116, 123-133, 150, 152-156, 158-161, 164, 169, 175-186, 197, 198, 203-216 in der Zählung gemäß SEQ ID NO:3 aufweist, insbesondere mindestens eine Aminosäuresubstitution an einer, zwei, drei oder vier der folgenden Positionen 116, 126, 127, 128 und 160 in der Zählung gemäß SEQ ID NO:3, vorzugsweise mindestens eine, noch bevorzugter mehrere, am bevorzugtesten jede der Aminosäuresubstitution G116V, S126L, P127Q und/oder S128A; oder
v) eine Aminosäuresequenz umfasst, die eine alkalische Protease aus Bacillus lentus DSM 5483 umfasst, insbesondere eine, die eine Aminosäuresequenz aufweist, die zu der in SEQ ID NO:4 angegebenen Aminosäuresequenz über deren Gesamtlänge zu mindestens 80%, bevorzugt zu mindestens 90 %, insbesondere zu 100 % identisch ist und die optional mindestens eine Aminosäuresubstitution an einer, zwei, drei oder vier der folgenden Positionen 3, 4, 99 und 199 in der Zählung gemäß SEQ ID NO:4 aufweist, insbesondere die Aminosäuresubstitution R99E oder R99D, sowie optional zusätzlich mindestens eine oder zwei, vorzugsweise alle drei der Aminosäuresubstitutionen S3T, V4I und V199I.

Gemäß einer bevorzugten Ausführungsform beträgt der Gewichtsanteil aller Proteasen, bezogen auf den Gehalt an aktivem Enzymprotein, am Gesamtgewicht der Reinigungsmittelzubereitung, 0,0005 bis 10,0 Gew.-%, bevorzugt 0,001 bis 7,0 Gew.-%, weiter bevorzugt 0,01 bis 4,0 Gew.-% und noch weiter bevorzugt 0,1 bis 2,0 Gew.-%.

Unter den Proteasen sind solche vom Subtilisin-Typ bevorzugt. Beispiele hierfür sind die Subtilisine BPN' und Carlsberg sowie deren weiterentwickelte Formen, die Protease PB92, die Subtilisine 147 und 309, die Alkalische Protease aus *Bacillus lentus,* Subtilisin DY und die den Subtilasen, nicht mehr jedoch den Subtilisinen im engeren Sinne zuzuordnenden Enzyme Thermitase, Proteinase K und die Proteasen TW3 und TW7.

Überraschenderweise wurde gefunden, dass bestimmte, insbesondere die Proteasen, die sich von bestimmten Subtilisinen ableiten, besonders für den Einsatz in wasserarmen flüssigen Reinigungsmitteln geeignet sind. Insbesondere können sie in Reinigungsmittelangebotsformen, die mehrere Teilmengen an Zubereitungen enthalten und so bevorzugt zur automatischen Dosierung geeignet sind, eingesetzt werden. Dabei sind diese Proteasen in der Lage, mehrfach hintereinander die Temperaturerhöhungen in einer Geschirrspülmaschine bei Durchführung mehrere Spülzyklen zu überstehen.

In einer ganz besonders bevorzugten Ausführungsform der Erfindung umfasst die erfindungsgemäß eingesetzte Protease eine Aminosäuresequenz, die zu der in SEQ ID NO:1 angegebenen Aminosäuresequenz über deren Gesamtlänge zu mindestens 70% und zunehmend bevorzugt mindestens 71%, 72%, 73%, 74%, 75%, 76%, 77%, 78%, 79%, 80%, 81%, 82%, 83%, 84%, 85%, 86%, 87%, 88%, 89%, 90%, 90,5%, 91%, 91,5%, 92%, 92,5%, 93%, 93,5%, 94%, 94,5%, 95%, 95,5%, 96%, 96,5%, 97%, 97,5%, 98%, 98,5% und 98,8% identisch ist, und die bevorzugt an mindestens einer der Positionen, die den Positionen 12, 43, 122, 127, 154, 156, 160, 211, 212 oder 222 in der Zählung gemäß SEQ ID NO:1 entsprechen, eine oder mehrere der Aminosäuresubstitutionen 12L, 43V, 122L, 127P, 154S, 156A, 160S, 211N, 211L, 212D, 212H oder 222S aufweist.

In einer weiteren Ausführungsform der Erfindung umfasst die erfindungsgemäß eingesetzte Protease eine Aminosäuresequenz, die zu der in SEQ ID NO:2 angegebenen Aminosäuresequenz über deren Gesamtlänge zu mindestens 81% und zunehmend bevorzugt mindestens 82%, 83%, 84%, 85%, 86%, 87%, 88%, 89%, 90%, 90,5%, 91%, 91,5%, 92%, 92,5%, 93%, 93,5%, 94%, 94,5%, 95%, 95,5%, 96%, 96,5%, 97%, 97,5%, 98%, 98,5% und 98,8% identisch ist, und die bevorzugt mindestens eine Aminosäuresubstitution an einer der Positionen 9, 15, 66, 212 und 239 in der Zählung gemäß SEQ ID NO:2 entsprechen, eine oder mehrere Aminosäuresubstitionen aufweisen, vorzugsweise ausgewählt aus der Gruppe bestehend aus 9R, 15T, 66A, 212D oder 239R.

In einer weiteren Ausführungsform der Erfindung umfasst die erfindungsgemäß eingesetzte Protease eine Aminosäuresequenz, die zu der in SEQ ID NO:2 angegebenen Aminosäuresequenz über deren Gesamtlänge zu mindestens 81% und zunehmend bevorzugt mindestens 82%, 83%, 84%, 85%, 86%, 87%, 88%, 89%, 90%, 90,5%, 91%, 91,5%, 92%, 92,5%, 93%, 93,5%, 94%, 94,5%, 95%, 95,5%, 96%, 96,5%, 97%, 97,5%, 98%, 98,5% und 98,8% identisch ist, und die eine Aminosäuresubstitution an der Position 97 und eine Insertion von einer Aminosäure zwischen den Aminosäuren an Positionen 97 und 98 in der Zählung gemäß SEQ ID NO:2 aufweist, vorzugsweise ausgewählt aus 97A und/oder 97AD.

In einer weiteren Ausführungsform der Erfindung umfasst die erfindungsgemäß eingesetzte Protease eine Aminosäuresequenz, die zu der in SEQ ID NO:3 angegebenen Aminosäuresequenz über deren Gesamtlänge zu mindestens 81% und zunehmend bevorzugt mindestens 82%, 83%, 84%, 85%, 86%, 87%, 88%, 89%, 90%, 90,5%, 91%, 91,5%, 92%, 92,5%, 93%, 93,5%, 94%, 94,5%, 95%, 95,5%, 96%, 96,5%, 97%, 97,5%, 98%, 98,5% und 98,8% identisch ist, und die bevorzugt an mindestens einer der Positionen, die den Positionen 32, 33, 48-54, 58-62, 94-107, 116, 123-133, 150, 152-156, 158-161, 164, 169, 175-186, 197, 198, 203-216 in der Zählung gemäß SEQ ID NO:3 entsprechen, insbesondere mindestens eine Aminosäuresubstitution an einer, zwei, drei oder vier der folgenden Positionen 116, 126, 127, 128 und 160 in der Zählung gemäß SEQ ID NO:3, vorzugsweise mindestens eine, noch bevorzugter mehrere, am bevorzugtesten jede der Aminosäuresubstitution 116V, 126L, 127Q und/oder 128A aufweist.

In einer weiteren Ausführungsform der Erfindung umfasst die erfindungsgemäß eingesetzte Protease eine Aminosäuresequenz, die zu der in SEQ ID NO:4 angegebenen Aminosäuresequenz über deren Gesamtlänge zu mindestens 81% und zunehmend bevorzugt mindestens 82%, 83%, 84%, 85%, 86%, 87%, 88%, 89%, 90%, 90,5%, 91%, 91,5%, 92%, 92,5%, 93%, 93,5%, 94%, 94,5%, 95%, 95,5%, 96%, 96,5%, 97%, 97,5%, 98%, 98,5% und 98,8% identisch ist, und die bevorzugt an mindestens einer der Positionen, die den Positionen 3, 4, 99 und 199 in der Zählung gemäß SEQ ID NO:4 insbesondere die Aminosäuresubstitution 99E oder 99D, sowie optional zusätzlich mindestens eine oder zwei, vorzugsweise alle drei der Aminosäuresubstitutionen 3T, 4I und 199I aufweist.

Besonders bevorzugt umfasst ist (ggf. neben weiteren Enzymen) eine Protease, die eine Aminosäuresequenz umfasst, die mindestens 70% Sequenzidentität mit der in SEQ ID NO:1 (B.gibsonii wt) angegebenen Aminosäuresequenz über deren Gesamtlänge aufweist und die eine Aminosäuresubstitution an mindestens einer der Positionen, die den Positionen 12, 43, 122, 127, 154, 156, 160, 211, 212 oder 222, jeweils bezogen auf die Nummerierung gemäß SEQ ID NO:1, entsprechen, aufweist, insbesondere wobei die mindestens eine Aminosäuresubstitution aus der Gruppe bestehend aus Q12L, I43V, M122L, D127P, N154S, T156A, G160S, M211N, M211L, P212D, P212H oder A222S, jeweils bezogen auf die Nummerierung gemäß SEQ ID NO:1, ausgewählt ist, und/oder insbesondere bevorzugt ist eine Protease, welche eine der folgenden Aminosäuresubstitutionsvarianten, jeweils bezogen auf die Nummerierung gemäß SEQ ID NO:1, aufweist:
(i) I43V;
(ii) M122L, N154S und T156A;
(iii) M211N und P212D;
(iv) M211L und P212D;
(v) G160S;
(vi) D127P, M211L und P212D;
(vii) P212H; oder
(viii) Q12L, M122L und A222S.

Ein weiterer Gegenstand der vorliegenden Erfindung ist eine Reinigungsmittelkombination bzw. Reinigungsmittelangebotsform, umfassend
a) eine flüssige (20°C) Reinigungsmittelzubereitung A, enthaltend Gerüststoff;
b) eine flüssige (20°C) von der Reinigungsmittelzubereitung A verschiedene Reinigungsmittelzubereitung B enthaltend
   b1) mindestens eine Protease,
      die bevorzugt eine von Subtilisinen abgeleitete Protease ist, und/oder insbesondere eine Protease gemäß (i) bis (v), wobei die mindestens eine Protease
      (i) eine Aminosäuresequenz umfasst, die mindestens 70% Sequenzidentität mit der in SEQ ID NO:1 angegebenen Aminosäuresequenz über deren Gesamtlänge aufweist und die eine Aminosäuresubstitution an mindestens einer der Positionen, die den Positionen 12, 43, 122, 127, 154, 156, 160, 211, 212 oder 222, jeweils bezogen auf die Nummerierung gemäß SEQ ID NO:1, entsprechen, aufweist, insbesondere wobei die mindestens eine Aminosäuresubstitution aus der Gruppe bestehend aus Q12L, I43V, M122L, D127P, N154S, T156A, G160S, M211N, M211L, P212D, P212H oder A222S, jeweils bezogen auf die Nummerierung gemäß SEQ ID NO:1, ausgewählt ist, und/oder insbesondere bevorzugt eine der folgenden Aminosäuresubstitutionsvarianten, jeweils bezogen auf die Nummerierung gemäß SEQ ID NO:1, aufweist:
         a. I43V;
         b. M122L, N154S und T156A;
         c. M211N und P212D;
         d. M211L und P212D;
         e. G160S;
         f. D127P, M211L und P212D;
         g. P212H; oder
         h. Q12L, M122L und A222S;
      (ii) eine Aminosäuresequenz umfasst, die ein Subtilisin 309 aus *Bacillus lentus* umfasst, insbesondere eine, die eine Aminosäuresequenz umfasst, die zu der in SEQ ID NO:2 angegebenen Aminosäuresequenz über deren Gesamtlänge zu mindestens 80%, bevorzugt zu mindestens 90 %, insbesondere zu 100 % identisch ist und mindestens eine Aminosäuresubstitution an einer der Positionen 9, 15, 66, 212 und 239 in der Zählung gemäß SEQ ID NO:2 aufweist, vorzugsweise ausgewählt aus der Gruppe bestehend aus S9R, A15T, V66A, N212D und Q239R;
      iii) eine Aminosäuresequenz umfasst, die ein Subtilisin 309 aus *Bacillus lentus* umfasst, insbesondere eine, die eine Aminosäuresequenz umfasst, die zu der in SEQ ID NO:2 angegebenen Aminosäuresequenz über deren Gesamtlänge zu mindestens 80% bevorzugt zu mindestens 90 %, insbesondere zu 100 % identisch ist und eine Aminosäuresubstitution an der Position 97 und eine Insertion von einer Aminosäure zwischen den Aminosäuren an Positionen 97 und 98 in der Zählung gemäß SEQ ID NO:2 aufweist, vorzugsweise ausgewählt aus S97A und/oder S97AD;
      iv) eine Aminosäuresequenz umfasst, die eine zu der in SEQ ID NO:3 angegebenen Aminosäuresequenz über deren Gesamtlänge zu mindestens 80%, bevorzugt zu mindestens 90 %, insbesondere zu 100 % identisch ist und die optional mindestens eine Aminosäuresubstitution an einer der folgenden Positionen 32, 33, 48-54, 58-62, 94-107, 116, 123-133, 150, 152-156, 158-161, 164, 169, 175-186, 197, 198, 203-216 in der Zählung gemäß SEQ ID NO:3 aufweist, insbesondere mindestens eine Aminosäuresubstitution an einer, zwei, drei oder vier der folgenden Positionen 116, 126, 127, 128 und 160 in der Zählung gemäß SEQ ID NO:3, vorzugsweise mindestens eine, noch bevorzugter mehrere, am bevorzugtesten jede der Aminosäuresubstitution G116V, S126L, P127Q und/oder S128A; oder
      v) eine Aminosäuresequenz umfasst, die eine alkalische Protease aus Bacillus lentus DSM 5483 umfasst, insbesondere eine, die eine Aminosäuresequenz aufweist, die zu der in SEQ ID NO:4 angegebenen Aminosäuresequenz über deren Gesamtlänge zu mindestens 80%, bevorzugt zu mindestens 90 %, insbesondere zu 100 % identisch ist und die optional mindestens eine Aminosäuresubstitution an einer, zwei, drei oder vier der folgenden Positionen 3, 4, 99 und 199 in der Zählung gemäß SEQ ID NO:4 aufweist, insbesondere die Aminosäuresubstitution R99E oder R99D, sowie optional zusätzlich mindestens eine oder zwei, vorzugsweise alle drei der Aminosäuresubstitutionen S3T, V4I und V199I sowie
   b2) optional ein Tensid, bevorzugt ein nichtionisches Tensid,
c) optional eine flüssige (20°C) von den Reinigungsmittelzubereitungen A und B verschiedene Reinigungsmittelzubereitung C, enthaltend
   c1) ein Acidifizierungsmittel,
   c2) einen Glaskorrosionsinhibitor,
   c3) optional ein nichtionisches Tensid,
   c4) optional ein Hydrotrop, und
   c5) optional weniger als 1 Gew.-%, bevorzugt weniger als 0,5 Gew.-%, insbesondere weniger als 0,1 Gew.-% Enzymzubereitung, und
d) ein Verpackungsmittel, in welchem die Reinigungsmittelzubereitungen A, B und optional C getrennt voneinander vorliegen.

Alles vorstehend für die flüssige Reinigungsmittelzubereitung Beschriebene gilt analog ebenfalls für die Reinigungsmittelzubereitung B der Reinigungsmittelkombination sowie der Reinigungsmittelangebotsform.

Alle Prozentangaben, die im Zusammenhang mit den hierin beschriebenen Zusammensetzungen gemacht werden, beziehen sich, sofern nicht explizit anders angegeben auf Gew.-%, jeweils bezogen auf die betreffende Mischung. Werden in der vorliegenden Anmeldung Aggregatzustände (fest, flüssig) genannt, so beziehen diese sich, wenn nicht anders angegeben, auf Raumtemperatur (20°C) bei Normaldruck von 1bar.

Die Reinigungsmittelzubereitung bzw. die Reinigungsmittelkombination und auch die Reinigungsmittelangebotsform ist dadurch gekennzeichnet, dass die Reinigungsmittelzubereitungen phosphatfrei sind, d.h. dass sie weniger als 1 Gew.-% Phosphat, vorzugsweise weniger als 0,5 Gew.-% Phosphat, besonders bevorzugt weniger als 0,1 Gew.-% Phosphat und insbesondere kein Phosphat enthalten.

Die Kombination einer solchen Zusammensetzung mit den erfindungsgemäßen Reinigungsmittelzubereitungen hat für den Verbraucher den Vorteil, dass er sich um die Erneuerung bzw. den Ersatz der Wirkstoffzusammensetzung und der Reinigungsmittelzubereitungen viele Gedanken machen muss. Er tauscht die Kombination gemeinsam aus und muss sich nicht separat um den Ersatz einzelner Produkte kümmern.

Überraschenderweise wurde gefunden, dass die erfindungsgemäße Reinigungsmittelangebotsform eine gute Reinigungsleistung, bevorzugt bei hartnäckigen Speiseresten auf dem Spülgut, beispielsweise Hackfleisch und Eigelb aufweist. Das Abspülen von Speiseresten vor Einsortierung in die Spülmaschine kann daher entfallen, was aus ökologischen und zeitlichen Gründen sowie hinsichtlich der zusätzlichen Wasserkosten besonders vorteilhaft ist. Somit muss der Verbraucher nicht die Spülmaschine übermäßig oft nutzen, um diese hartnäckigen Speisereste zeitnah zu entfernen, bis sie stark aufgetrocknet sind, sondern kann auch bei weniger Spülzyklen pro Woche der Spülmaschine Spülgut entnehmen, welches eine gute Reinheit insbesondere hinsichtlich solcher hartnäckigen Anschmutzungen aufweist.

"Variante", wie hierin verwendet, bezieht sich auf natürliche oder artifiziell erzeugte Variationen eines nativen Enzyms, die eine gegenüber der Referenzform abgewandelte Aminosäuresequenz aufweist. Zusätzlich zu den vorstehend erläuterten Aminosäureveränderungen können erfindungsgemäße Enzyme weitere Aminosäureveränderungen, insbesondere Aminosäuresubstitutionen, -insertionen oder -deletionen, aufweisen. Solche Enyzme sind z.B. durch gezielte genetische Veränderung, d.h. durch Mutageneseverfahren, weiterentwickelt und für bestimmte Einsatzzwecke oder hinsichtlich spezieller Eigenschaften (z.B. hinsichtlich ihrer katalytischen Aktivität, Stabilität, usw.) optimiert. Ferner können erfindungsgemäße Nukleinsäuren in Rekombinationsansätze eingebracht und damit zur Erzeugung völlig neuartiger Enzyme oder anderer Polypeptide genutzt werden. Das Ziel ist es, in die bekannten Moleküle gezielte Mutationen wie Substitutionen, Insertionen oder Deletionen einzuführen, um z.B. die Reinigungsleistung von Enzymen zu verbessern. Hierzu können insbesondere die Oberflächenladungen und/oder der isoelektrische Punkt der Moleküle und dadurch ihre Wechselwirkungen mit dem Substrat verändert werden. So kann z.B. die Nettoladung der Enzyme verändert werden, um darüber die Substratbindung insbesondere für den Einsatz in Wasch- und Reinigungsmitteln zu beeinflussen. Alternativ oder ergänzend kann durch eine oder mehrere entsprechende Mutationen die Stabilität oder katalytische Aktivität des Enzyms erhöht und dadurch seine Reinigungsleistung verbessert werden. Vorteilhafte Eigenschaften einzelner Mutationen, z.B. einzelner Substitutionen, können sich ergänzen. Eine hinsichtlich bestimmter Eigenschaften bereits optimierte Protease kann daher im Rahmen der Erfindung zusätzlich weiterentwickelt sein, z.B. hinsichtlich ihrer Stabilität gegenüber Tensiden und/oder Bleichmitteln und/oder anderen Komponenten.

Für die Beschreibung von Substitutionen, die genau eine Aminosäureposition betreffen (Aminosäureaustausche), wird hierin folgende Konvention angewendet: zunächst wird die natürlicherweise vorhandene Aminosäure in Form des international gebräuchlichen Einbuchstaben-Codes bezeichnet, dann folgt die zugehörige Sequenzposition und schließlich die eingefügte Aminosäure. Mehrere Austausche innerhalb derselben Polypeptidkette werden durch Schrägstriche voneinander getrennt. Bei Insertionen sind nach der Sequenzposition zusätzliche Aminosäuren benannt. Bei Deletionen ist die fehlende Aminosäure durch ein Symbol, z.B. einen Stern oder einen Strich, ersetzt oder vor der entsprechenden Position ein Δ angegeben. Beispielsweise beschreibt P14H die Substitution von Prolin an Position 14 durch Histidin, P14HT die Insertion von Threonin nach der Aminosäure Histidin an Position 14 und P14* oder ΔP14 die Deletion von Prolin an Position 14. Diese Nomenklatur ist dem Fachmann auf dem Gebiet der Enzymtechnologie bekannt.

Gemäß einer bevorzugten Ausführungsform beträgt der Gewichtsanteil aller Proteasen bezogen auf den Gehalt an aktivem Enzymprotein am Gesamtgewicht der Reinigungsmittelzubereitung B, 0,0005 bis 10,0 Gew.-%, bevorzugt 0,001 bis 7,0 Gew.-%, weiter bevorzugt 0,01 bis 4,0 Gew.-% und noch weiter bevorzugt 0,1 bis 2,0 Gew.-%.

Im Zusammenhang mit der vorliegenden Erfindung bedeutet das Merkmal, dass ein Enzym, insbesondere eine Protease die angegebenen Substitutionen aufweist, dass sie mindestens eine der entsprechenden Aminosäuren an den entsprechenden Positionen enthält, d.h. nicht alle der 10 Positionen anderweitig mutiert oder, beispielsweise durch Fragmentierung des Enzyms, insbesondere der Protease, deletiert sind.

Die Bestimmung der Identität von Nukleinsäure- oder Aminosäuresequenzen erfolgt durch einen Sequenzvergleich. Dieser Sequenzvergleich basiert auf dem im Stand der Technik etablierten und üblicherweise genutzten BLAST-Algorithmus (vgl. z.B. Altschul et al. (1990) "Basic local alignment search tool", J. Mol. Biol. 215:403-410 und Altschul et al. (1997) "Gapped BLAST and PSI-BLAST: a new generation of protein database search programs", Nucleic Acids Res., 25:3389-3402) und geschieht prinzipiell dadurch, dass ähnliche Abfolgen von Nukleotiden oder Aminosäuren in den Nukleinsäure- oder Aminosäuresequenzen einander zugeordnet werden. Eine tabellarische Zuordnung der betreffenden Positionen wird als Alignment bezeichnet. Ein weiterer im Stand der Technik verfügbarer Algorithmus ist der FASTA-Algorithmus. Sequenzvergleiche (Alignments), insbesondere multiple Sequenzvergleiche, werden mit Computerprogrammen erstellt. Häufig genutzt werden beispielsweise die Clustal-Serie (vgl. z.B. Chenna et al. (2003) "Multiple sequence alignment with the Clustal series of programs", Nucleic Acid Res. 31:3497-3500), T-Coffee (vgl. z.B. Notredame et al. (2000) "T-Coffee: A novel method for multiple sequence alignments", J. Mol. Biol. 302:205-217) oder Programme, die auf diesen Programmen beziehungsweise Algorithmen basieren. Ferner möglich sind Sequenzvergleiche (Alignments) mit dem Computer-Programm Vector NTI® Suite 10.3 (Invitrogen Corporation, 1600 Faraday Avenue, Carlsbad, Kalifornien, USA) mit den vorgegebenen Standardparametern, dessen AlignX-Modul für die Sequenzvergleiche auf ClustalW basiert. Soweit nicht anders angegeben, wird die hierin angegebene Sequenzidentität mit dem BLAST-Algorithmus bestimmt.

Ein weiterer Gegenstand der vorliegenden Erfindung ist daher eine Reinigungsmittelkombination bzw. Reinigungsmittelangebotsform, umfassend
a) eine flüssige (20°C) Reinigungsmittelzubereitung A, enthaltend Gerüststoff;
b) eine flüssige (20°C) von der Reinigungsmittelzubereitung A verschiedene Reinigungsmittelzubereitung B enthaltend
   b1) mindestens eine Protease,
      die bevorzugt eine von Subtilisinen abgeleitete Protease ist, und/oder insbesondere eine Protease gemäß (i) bis (v), wobei die mindestens eine Protease
      (ii) eine Aminosäuresequenz umfasst, die mindestens 70% Sequenzidentität mit der in SEQ ID NO:1 angegebenen Aminosäuresequenz über deren Gesamtlänge aufweist und die eine Aminosäuresubstitution an mindestens einer der Positionen, die den Positionen 12, 43, 122, 127, 154, 156, 160, 211, 212 oder 222, jeweils bezogen auf die Nummerierung gemäß SEQ ID NO:1, entsprechen, aufweist, insbesondere wobei die mindestens eine Aminosäuresubstitution aus der Gruppe bestehend aus Q12L, I43V, M122L, D127P, N154S, T156A, G160S, M211N, M211L, P212D, P212H oder A222S, jeweils bezogen auf die Nummerierung gemäß SEQ ID NO:1, ausgewählt ist, und/oder insbesondere bevorzugt eine der folgenden Aminosäuresubstitutionsvarianten, jeweils bezogen auf die Nummerierung gemäß SEQ ID NO:1, aufweist:
      i. I43V;
      j. M122L, N154S und T156A;
      k. M211N und P212D;
      I. M211L und P212D;
      m. G160S;
      n. D127P, M211L und P212D;
      o. P212H; oder
      p. Q12L, M122L und A222S;
      sowie
   b2) optional ein Tensid, bevorzugt ein nichtionisches Tensid,
c) optional eine flüssige (20°C) von den Reinigungsmittelzubereitungen A und B verschiedene Reinigungsmittelzubereitung C, enthaltend
   c1) ein Acidifizierungsmittel,
   c2) einen Glaskorrosionsinhibitor,
   c3) optional ein nichtionisches Tensid,
   c4) optional ein Hydrotrop, und
   c5) optional weniger als 1 Gew.-%, bevorzugt weniger als 0,5 Gew.-%, insbesondere weniger als 0,1 Gew.-% Enzymzubereitung, und
d) ein Verpackungsmittel, in welchem die Reinigungsmittelzubereitungen A, B und optional C getrennt voneinander vorliegen.

Gemäß einer weiteren bevorzugten Ausführungsform der vorliegenden Erfindung beträgt der Gewichtsanteil dieser Protease(n), bezogen auf die Menge an aktivem Enzymprotein, in der flüssigen Reinigungsmittelzubereitung B, bezogen auf deren Gesamtgewicht, 0,0005 bis 10,0 Gew.-%, bevorzugt 0,001 bis 7,0 Gew.-%, weiter bevorzugt 0,01 bis 4,0 Gew.-% und noch weiter bevorzugt 0,1 bis 2,0 Gew.-%.

Gemäß einer weiteren besonders bevorzugten Ausführungsform der vorliegenden Erfindung beträgt die Menge aller Proteasen, bezogen auf die Menge an aktivem Enzymprotein, welche pro Spülgang hinzudosiert wird, bevorzugt 0,001 bis 1000 mg/job, weiter bevorzugt 0,1 bis 600 mg/job und noch weiter bevorzugt 1,0 bis 400 mg/job.

Erfindungsgemäß bevorzugte flüssige Reinigungsmittelzubereitungen B enthalten, bezogen auf das Gesamtgewicht der Reinigungsmittelzubereitung B, 5 bis 50 Gew.-%, vorzugsweise 7 bis 40 Gew.-% und insbesondere 10 bis 30 Gew.-% Protease-Zubereitungen.

Der Gegenstand dieser Anmeldung sind weiterhin Reinigungsmittelkombinationen, die mindestns die zwei flüssigen Reinigungsmittelzubereitungen A und B umfassen.

Ein weiterer bevorzugter Gegenstand dieser Anmeldung sind entsprechende Reinigungsmittelangebotsformen, die durch die Kombination von zwei flüssigen Reinigungsmittelzubereitungen A und B erhalten werden. Die flüssigen Reinigungsmittelzubereitungen A und B und die Wirkstoffzusammensetzung unterscheiden sich hinsichtlich ihrer Zusammensetzung voneinander.

Die Reinigungsmittelzubereitung A enthält als ersten wesentlichen Bestandteil einen oder mehrere Gerüststoffe. Zu den Gerüststoffen zählen insbesondere Carbonate, organische Cobuilder und Silikate. Erfindungsgemäße Reinigungsmittelangebotsformen sind vorzugsweise dadurch gekennzeichnet, dass der Gerüststoff a1) ausgewählt ist aus der Gruppe der Carbonate, der Hydrogencarbonate, der Citrate, der Silikate, der polymeren Carboxylate und der Sulfonsäuregruppen-haltigen Polymeren.

Als Sulfongruppenhaltiges Polymer, wird vorzugsweise ein Sulfopolymer vorzugsweise ein copolymeres Polysulfonat, vorzugsweise ein hydrophob modifiziertes copolymeres Polysulfonat eingesetzt. Die Copolymere können zwei, drei, vier oder mehr unterschiedliche Monomereinheiten aufweisen. Bevorzugte copolymere Polysulfonate enthalten neben Sulfonsäuregruppen-haltigem(n) Monomer(en) wenigstens ein Monomer aus der Gruppe der ungesättigten Carbonsäuren.

Als ungesättigte Carbonsäure(n) wird/werden mit besonderem Vorzug ungesättigte Carbonsäuren der Formel R¹(R²)C=C(R³)COOH eingesetzt, in der R¹ bis R³ unabhängig voneinander für -H, -CH₃, einen geradkettigen oder verzweigten gesättigten Alkylrest mit 2 bis 12 Kohlenstoffatomen, einen geradkettigen oder verzweigten, ein- oder mehrfach ungesättigten Alkenylrest mit 2 bis 12 Kohlenstoffatomen, mit -NH2, -OH oder -COOH substituierte Alkyl- oder Alkenylreste wie vorstehend definiert oder für -COOH oder -COOR⁴ steht, wobei R⁴ ein gesättigter oder ungesättigter, geradkettigter oder verzweigter Kohlenwasserstoffrest mit 1 bis 12 Kohlenstoffatomen ist.

Besonders bevorzugte ungesättigte Carbonsäuren sind Acrylsäure, Methacrylsäure, Ethacrylsäure, α-Chloroacrylsäure, α-Cyanoacrylsäure, Crotonsäure, α-Phenyl-Acrylsäure, Maleinsäure, Maleinsäureanhydrid, Fumarsäure, Itaconsäure, Citraconsäure, Methylenmalonsäure, Sorbinsäure, Zimtsäure oder deren Mischungen. Einsetzbar sind selbstverständlich auch die ungesättigten Dicarbonsäuren.

Bei den Sulfonsäuregruppen-haltigen Monomeren sind solche der Formel R⁵(R⁶)C=C(R⁷)-X-SO₃H bevorzugt, in der R⁵ bis R⁷ unabhängig voneinander für -H, -CH₃, einen geradkettigen oder verzweigten gesättigten Alkylrest mit 2 bis 12 Kohlenstoffatomen, einen geradkettigen oder verzweigten, ein- oder mehrfach ungesättigten Alkenylrest mit 2 bis 12 Kohlenstoffatomen, mit-NH₂, -OH oder -COOH substituierte Alkyl- oder Alkenylreste oder für -COOH oder -COOR⁴ steht, wobei R⁴ ein gesättigter oder ungesättigter, geradkettigter oder verzweigter Kohlenwasserstoffrest mit 1 bis 12 Kohlenstoffatomen ist, und X für eine optional vorhandene Spacergruppe steht, die ausgewählt ist aus -(CH₂)ₙ- mit n = 0 bis 4, -COO-(CH₂)ₖ- mit k = 1 bis 6, -C(O)-NH-C(CH₃)₂-,-C(O)-NH-C(CH₃)₂-CH₂- und -C(O)-NH-CH(CH₃)-CH₂-.

Unter diesen Monomeren bevorzugt sind solche der Formeln
H₂C=CH-X-SO₃H, H₂C=C(CH₃)-X-SO₃H oder HO₃S-X-(R⁶)C=C(R⁷)-X-SO₃H,
in denen R⁶ und R⁷ unabhängig voneinander ausgewählt sind aus -H, -CH₃, -CH₂CH₃,-CH₂CH₂CH₃ und -CH(CH₃)₂ und X für eine optional vorhandene Spacergruppe steht, die ausgewählt ist aus -(CH₂)ₙ- mit n = 0 bis 4, -COO-(CH₂)ₖ- mit k = 1 bis 6, -C(O)-NH-C(CH₃)₂-,-C(O)-NH-C(CH₃)₂-CH₂- und -C(O)-NH-CH(CH₃)-CH₂-.

Gemäß einer besonders bevorzugten Ausführungsform enthält die Reinigungsmittelzubereitung A ein Polymer umfassend als sulfonsäuregruppenhaltiges Monomer Acrylamidopropansulfonsäuren, Methacrylamidomethylpropansulfonsäuren oder Acrylamidomethylpropansulfonsäure.

Besonders bevorzugte Sulfonsäuregruppen-haltige Monomere sind dabei 1-Acrylamido-1-propansulfonsäure, 2-Acrylamido-2-propansulfonsäure, 2-Acrylamido-2-methyl-1-propansulfonsäure, 2-Methacrylamido-2-methyl-1-propansulfonsäure, 3-Methacrylamido-2-hydroxy-propansulfonsäure, Allylsulfonsäure, Methallylsulfonsäure, Allyloxybenzolsulfonsäure, Methallyloxybenzolsulfonsäure, 2-Hydroxy-3-(2-propenyloxy)propansulfonsäure, 2-Methyl-2-propen1-sulfonsäure, Styrolsulfonsäure, Vinylsulfonsäure, 3-Sulfopropylacrylat, 3-Sulfopropylmethacrylat, Sulfomethacrylamid, Sulfomethylmethacrylamid sowie Mischungen der genannten Säuren oder deren wasserlösliche Salze. In den Polymeren können die Sulfonsäuregruppen ganz oder teilweise in neutralisierter Form vorliegen, das heißt dass das acide Wasserstoffatom der Sulfonsäuregruppe in einigen oder allen Sulfonsäuregruppen gegen Metallionen, vorzugsweise Alkalimetallionen und insbesondere gegen Natriumionen, ausgetauscht sein kann. Der Einsatz von teil- oder vollneutralisierten Sulfonsäuregruppen-haltigen Copolymeren ist erfindungsgemäß bevorzugt.

Die Monomerenverteilung der erfindungsgemäß bevorzugt eingesetzten Copolymere beträgt bei Copolymeren, die nur Carbonsäuregruppen-haltige Monomere und Sulfonsäuregruppen-haltige Monomere enthalten, vorzugsweise jeweils 5 bis 95 Gew.-%, besonders bevorzugt beträgt der Anteil des Sulfonsäuregruppen-haltigen Monomers 50 bis 90 Gew.-% und der Anteil des Carbonsäuregruppen-haltigen Monomers 10 bis 50 Gew.-%, die Monomere sind hierbei vorzugsweise ausgewählt aus den zuvor genannten. Die Molmasse der erfindungsgemäß bevorzugt eingesetzten Sulfo-Copolymere kann variiert werden, um die Eigenschaften der Polymere dem gewünschten Verwendungszweck anzupassen. Bevorzugte Reinigungsmittel sind dadurch gekennzeichnet, dass die Copolymere Molmassen von 2000 bis 200.000 g·mol⁻¹, vorzugsweise von 4000 bis 25.000 g·mol⁻¹ und insbesondere von 5000 bis 15.000 g·mol⁻¹ aufweisen.

In einer weiteren bevorzugten Ausführungsform umfassen die Copolymere neben Carboxylgruppen-haltigem Monomer und Sulfonsäuregruppen-haltigem Monomer weiterhin wenigstens ein nichtionisches, vorzugsweise hydrophobes Monomer. Durch den Einsatz dieser hydrophob modifizierten Polymere konnte insbesondere die Klarspülleistung erfindungsgemäßer Geschirrspülmittel verbessert werden.

Besonders bevorzugt umfasst die Reinigungsmittelzubereitung A weiterhin ein anionisches Copolymer, wobei als anionisches Copolymer ein Copolymer, umfassend
i) Carbonsäuregruppen-haltige Monomere
ii) Sulfonsäuregruppen-haltige Monomere
iii) optional nichtionische Monomere, insbesondere hydrophobe Monomere eingesetzt wird.

Als nichtionische Monomere werden vorzugsweise Monomere der allgemeinen Formel
R¹(R²)C=C(R³)-X-R⁴ eingesetzt, in der R¹ bis R³ unabhängig voneinander für -H, -CH₃ oder-C₂H₅ steht, X für eine optional vorhandene Spacergruppe steht, die ausgewählt ist aus -CH₂-,-C(O)O- und -C(O)-NH-, und R⁴ für einen geradkettigen oder verzweigten gesättigten Alkylrest mit 2 bis 22 Kohlenstoffatomen oder für einen ungesättigten, vorzugsweise aromatischen Rest mit 6 bis 22 Kohlenstoffatomen steht.

Besonders bevorzugte nichtionische Monomere sind Buten, Isobuten, Penten, 3-Methylbuten, 2-Methylbuten, Cyclopenten, Hexen, Hexen-1, 2-Methlypenten-1, 3-Methlypenten-1, Cyclohexen, Methylcyclopenten, Cyclohepten, Methylcyclohexen, 2,4,4-Trimethylpenten-1, 2,4,4-Trimethylpenten-2,2,3-Dimethylhexen-1, 2,4-Diemthylhexen-1, 2,5-Dimethlyhexen-1, 3,5-Dimethylhexen-1, 4,4-Dimethylhexan-1, Ethylcyclohexyn, 1-Octen, α-Olefine mit 10 oder mehr Kohlenstoffatomen wie beispielsweise 1-Decen, 1-Dodecen, 1-Hexadecen, 1-Oktadecen und C₂₂-α-Otefin, 2-Styrol, α-Methylstyrol, 3-Methylstyrol, 4-Propylstryol, 4-Cyclohexylstyrol, 4-Dodecylstyrol, 2-Ethyl-4-Benzylstyrol, 1-Vinylnaphthalin, 2-Vinylnaphthalin, Acrylsäuremethylester, Acrylsäureethylester, Acrylsäurepropylester, Acrylsäurebutylester, Acrylsäurepentylester, Acrylsäurehexylester, Methacrylsäuremethylester, *N*-(Methyl)acrylamid, Acrylsäure-2-Ethylhexylester, Methacrylsäure-2-Ethylhexylester, *N*-(2-Ethylhexyl)acrylamid, Acrylsäureoctylester, Methacrylsäureoctylester, *N*-(Octyl)acrylamid, Acrylsäurelaurylester, Methacrylsäurelaurylester, *N*-(Lauryl)acrylamid, Acrylsäurestearylester, Methacrylsäurestearylester, *N*-(Stearyl)acrylamid, Acrylsäurebehenylester, Methacrylsäurebehenylester und N-(Behenyl)acrylamid oder deren Mischungen, insbesondere Acrylsäure, Ethylacrylat, 2-Acrylamido-2-methylpropansulfonsäure (AMPS) sowie deren Mischungen.

Bevorzugte Reinigungsmittelangebotsformen umfassen eine Reinigungsmittelzubereitung A, die, bezogen auf ihr Gesamtgewicht, 2 bis 50 Gew.-%, vorzugsweise 6 bis 45 Gew.-% und insbesondere 10 bis 40 Gew.-% Gerüststoff enthält.

Besonders bevorzugt ist der Einsatz von Gerüststoffen a1) aus der Gruppe der Carbonate und/oder Hydrogencarbonate, vorzugsweise Alkalicarbonate, besonders bevorzugt Natriumcarbonat, in Mengen von 2 bis 30 Gew.-%, vorzugsweise von 4 bis 25 Gew.-% und insbesondere von 6 bis 20 Gew.-%, jeweils bezogen auf das Gewicht der Reinigungsmittelzubereitung A.

Als organische Cobuilder sind insbesondere Polycarboxylate / Polycarbonsäuren, polymere Carboxylate, Asparaginsäure, Polyacetale, Dextrine und organische Cobuilder zu nennen. Diese Stoffklassen werden nachfolgend beschrieben.

Brauchbare organische Gerüstsubstanzen sind beispielsweise die in Form der freien Säure und/oder ihrer Natriumsalze einsetzbaren Polycarbonsäuren, wobei unter Polycarbonsäuren solche Carbonsäuren verstanden werden, die mehr als eine Säurefunktion tragen. Beispielsweise sind dies Citronensäure, Adipinsäure, Bernsteinsäure, Glutarsäure, Äpfelsäure, Weinsäure, Maleinsäure, Fumarsäure, Zuckersäuren, sofern ein derartiger Einsatz aus ökologischen Gründen nicht zu beanstanden ist, sowie Mischungen aus diesen. Die freien Säuren besitzen neben ihrer Builderwirkung typischerweise auch die Eigenschaft einer Säuerungskomponente und dienen somit auch zur Einstellung eines niedrigeren und milderen pH-Wertes von Reinigungsmitteln. Insbesondere sind hierbei Citronensäure, Bernsteinsäure, Glutarsäure, Adipinsäure, Gluconsäure und beliebige Mischungen aus diesen zu nennen.

Besonders bevorzugte erfindungsgemäße Reinigungsmittelzubereitungen A enthalten als einen ihrer wesentlichen Gerüststoffe Citrat. Reinigungsmittelangebotsformen, dadurch gekennzeichnet, dass die Reinigungsmittelzubereitung A, bezogen auf ihr Gesamtgewicht 2 bis 40 Gew.-%, vorzugsweise 3 bis 25 Gew.-% und insbesondere 4 bis 20 Gew.-% Citrat enthalten, sind erfindungsgemäß bevorzugt. Citrat bzw. Citronensäure haben sich insbesondere in Kombination mit Phosphonat, insbesondere 1-Hydroxyethan-1,1-diphosphonsäure, (sofern regulatorisch zulässig) und/oder den Sulfonsäuregruppen-haltigen Polymeren als die in Bezug auf die Reinigungsleistung wie die Klarspülleistung und insbesondere Belagsinhibierung wirksamsten Gerüststoffe erwiesen.

Besonders bevorzugt sind Reinigungsmittelangebotsform bei denen, die Reinigungsmittelzubereitung A, jeweils bezogen auf ihr Gesamtgewicht, Citrat in Mengen von 3 bis 25 Gew%, insbesondere von 4 bis 20 Gew.-% sowie Carbonat in Mengen von 4 bis 25 Gew.-%, insbesondere 6 bis 20 Gew.-% enthält.

Als Gerüststoffe sind weiterhin polymere Polycarboxylate geeignet, dies sind beispielsweise die Alkalimetallsalze der Polyacrylsäure oder der Polymethacrylsäure, beispielsweise solche mit einer relativen Molekülmasse von 500 bis 70000 g/mol.

Geeignete Polymere sind insbesondere Polyacrylate, die bevorzugt eine Molekülmasse von 2000 bis 20000 g/mol aufweisen. Aufgrund ihrer überlegenen Löslichkeit können aus dieser Gruppe wiederum die kurzkettigen Polyacrylate, die Molmassen von 2000 bis 10000 g/mol, und besonders bevorzugt von 3000 bis 5000 g/mol, aufweisen, bevorzugt sein.

Geeignet sind weiterhin copolymere Polycarboxylate, insbesondere solche der Acrylsäure mit Methacrylsäure und der Acrylsäure oder Methacrylsäure mit Maleinsäure. Als besonders geeignet haben sich Copolymere der Acrylsäure mit Maleinsäure erwiesen, die 50 bis 90 Gew.-% Acrylsäure und 50 bis 10 Gew.-% Maleinsäure enthalten. Ihre relative Molekülmasse, bezogen auf freie Säuren, beträgt im allgemeinen 2000 bis 70000 g/mol, vorzugsweise 20000 bis 50000 g/mol und insbesondere 30000 bis 40000 g/mol.

Der Gehalt der maschinellen Geschirrspülmittel an (co-)polymeren Polycarboxylaten beträgt vorzugsweise 0,5 bis 20 Gew.-% und insbesondere 3 bis 10 Gew.-%.

Erfindungsgemäße maschinelle Geschirrspülmittel können als Gerüststoff kristalline schichtförmige Silikate der allgemeinen Formel NaMSiₓO₂ₓ₊₁ · yH₂O, worin M Natrium oder Wasserstoff darstellt, x eine Zahl von 1,9 bis 22, vorzugsweise von 1,9 bis 4, wobei besonders bevorzugte Werte für x 2, 3 oder 4 sind, und y für eine Zahl von 0 bis 33, vorzugsweise von 0 bis 20 steht.

Einsetzbar sind auch amorphe Natriumsilikate mit einem Modul Na₂O : SiO₂ von 1:2 bis 1:3,3, vorzugsweise von 1:2 bis 1:2,8 und insbesondere von 1:2 bis 1:2,6, welche vorzugsweise löseverzögert sind und Sekundärwascheigenschaften aufweisen.

In bevorzugten erfindungsgemäßen maschinellen Geschirrspülmitteln wird der Gehalt an Silikaten, bezogen auf das Gesamtgewicht des maschinellen Geschirrspülmittels, auf Mengen unterhalb 10 Gew.-%, vorzugsweise unterhalb 5 Gew.-% und insbesondere unterhalb 2 Gew.-% begrenzt. Besonders bevorzugte erfindungsgemäße maschinelle Geschirrspülmittel sind Silikat-frei.

Selbstverständlich können die erfindungsgemäßen maschinellen Geschirrspülmittel die vorgenannten Gerüststoffe sowohl in Form einzelner Substanzen als auch in Form von Substanzgemischen aus zwei, drei, vier oder mehr Gerüststoffen enthalten.

Besonders bevorzugte flüssige maschinelle Geschirrspülmittel sind dadurch gekennzeichnet, dass das Geschirrspülmittel mindestens zwei Gerüststoffe aus der Gruppe der Carbonate und Citrate, und der Sulfonsäuregruppen-haltigen Polymeren enthält, wobei der Gewichtsanteil dieser Gerüststoffe, bezogen auf sein Gesamtgewicht des maschinellen Geschirrspülmittels, bevorzugt 2 bis 50 Gew.-%, vorzugsweise 5 bis 45 Gew.-% und insbesondere 10 bis 40 Gew.-% beträgt. Die Kombination von zwei oder mehr Gerüststoffen aus der oben genannten Gruppe hat sich für die Reinigungs- und Klarspülleistung erfindungsgemäßer maschineller Geschirrspülmittel als vorteilhaft erwiesen.

Als weiteren Bestandteil kann die Reinigungsmittelzubereitung A einen Komplexbildner enthalten, der von den vorstehend genannten Gerüststoffen verschieden ist. Der Gewichtsanteil des Komplexbildners am Gesamtgewicht der Reinigungsmittelzubereitung A beträgt dabei vorzugsweise 2 bis 60 Gew.-%, vorzugsweise 4 bis 55 Gew.-% und insbesondere 8 bis 50 Gew.-%.

Eine weitere Gruppe bevorzugter Komplexbildner bilden die Phosphonate, sofern sie aus regulatorischen Gründen einsetzbar sind. Die komplexbildenden Phosphonate umfassen neben der 1-Hydroxyethan-1,1-diphosphonsäure eine Reihe unterschiedlicher Verbindungen wie beispielsweise Diethylentriaminpenta-(methylenphosphonsäure) (DTPMP). In dieser Anmeldung bevorzugt sind insbesondere Hydroxyalkan- bzw. Aminoalkanphosphonate. Unter den Hydroxyalkanphosphonaten ist das 1-Hydroxyethan-1,1-diphosphonat (HEDP) von besonderer Bedeutung als Cobuilder. Es wird vorzugsweise als Natriumsalz eingesetzt, wobei das Dinatriumsalz neutral und das Tetranatriumsalz alkalisch (pH 9) reagiert. Als Aminoalkanphosphonate kommen vorzugsweise Ethylendiamintetramethylenphosphonat (EDTMP), Diethylentriaminpentamethylenphosphonat (DTPMP) sowie deren höhere Homologe in Frage. Sie werden vorzugsweise in Form der neutral reagierenden Natriumsalze, z. B. als Hexanatriumsalz der EDTMP bzw. als Hepta- und Octa-Natriumsalz der DTPMP, eingesetzt. Als Builder wird dabei aus der Klasse der Phosphonate bevorzugt HEDP verwendet. Die Aminoalkanphosphonate besitzen zudem ein ausgeprägtes Schwermetallbindevermögen. Dementsprechend kann es, insbesondere wenn die Mittel auch Bleiche enthalten, bevorzugt sein, Aminoalkanphosphonate, insbesondere DTPMP, einzusetzen, oder Mischungen aus den genannten Phosphonaten zu verwenden.

Eine im Rahmen dieser Anmeldung bevorzugte Reinigungsmittelzusammensetzung A enthält ein oder mehrere Phosphonat(e) aus der Gruppe
a) Aminotrimethylenphosphonsäure (ATMP) und/oder deren Salze;
b) Ethylendiamintetra(methylenphosphonsäure) (EDTMP) und/oder deren Salze;
c) Diethylentriaminpenta(methylenphosphonsäure) (DTPMP) und/oder deren Salze;
d) 1-Hydroxyethan-1,1-diphosphonsäure (HEDP) und/oder deren Salze;
e) 2-Phosphonobutan-1,2,4-tricarbonsäure (PBTC) und/oder deren Salze;
f) Hexamethylendiamintetra(methylenphosphonsäure) (HDTMP) und/oder deren Salze;
g) Nitrilotri(methylenphosphonsäure) (NTMP) und/oder deren Salze.

Besonders bevorzugt werden Reinigungsmittelzusammensetzungen A, welche als Phosphonate 1-Hydroxyethan-1,1-diphosphonsäure (HEDP) oder Diethylentriaminpenta(methylenphosphonsäure) (DTPMP) enthalten.

Selbstverständlich können die erfindungsgemäßen maschinellen Geschirrspülmittel zwei oder mehr unterschiedliche Phosphonate (sofern regulatorisch zulässig) enthalten. Der Gewichtsanteil der Phosphonate am Gesamtgewicht erfindungsgemäßer Reinigungsmittelzusammensetzungen A beträgt vorzugsweise 1 bis 9 Gew.-%, vorzugsweise 1,2 bis 8 Gew.-% und insbesondere 1,5 bis 6 Gew.-%.

Besonders bevorzugte Reinigungsmittelangebotsformen sind dadurch gekennzeichnet, dass sie zusätzlich weitere Komplexbildner ausgewählt aus der Gruppe Hydroxyethylethylendiamintriessigsäure, Ethylendiamintetraessigsäure, Diethylentriaminpentaessigsäure, Iminodibernsteinsäure, Hydroxyimino-dibernsteinsäure, Asparaginsäurediessigsäure, Hydroxyethan-1,1-diphosphonsäure oder Diethylentriaminpenta(methylenphosphonsäure) sowie deren Salzen oder deren Mischungen enthalten können.

Gemäß einer besonders bevorzugten Ausführungsform enthält die Reinigungsmittelzubereitung A die Komplexbildner ausgewählt aus Phosphonaten, sofern aus regulatorischen Gründen zulässig, und/oder MGDA und deren jeweiligen Salzen. Insbesondere umfassen dann die Gerüststoffe Citrat sowie Carbonat und/oder Hydrogencarbonat sowie optional mindestens ein Sulfonsäuregruppenhaltiges Polymer.

Die Reinigungszusammensetzung A kann als Komplexbildner L-Glutaminsäure-*N,N*-diessigsäure und/oder das entsprechende Alkalisalz (GLDA), bevorzugt das Tetranatriumsalz, und/oder Methylglycindiessigsäure und/oder das entsprechende Alkalisalz (MGDA), bevorzugt das Trinatriumsalz, enthalten. Ganz besonders bevorzugt ist das Trinatriumsalz der Methylglycindiessigsäure enthalten. Die Bezeichnung Methylglycindiessigsäure bzw. L-Glutaminsäure-*N,N*-diessigsäure umfasst neben den freien Säuren auch deren Salze, beispielsweise deren Natrium- oder Kaliumsalze.

Bevorzugte Reinigungsmittelzubereitungen A sind dadurch gekennzeichnet, dass diese, bezogen auf das Gesamtgewicht der Reinigungsmittelzubereitungen A, 3 bis 30 Gew.-%, bevorzugt 10 bis 20 Gew.-%, vorzugsweise 11 bis 18 Gew.-% und insbesondere 12 bis 15,5 Gew.-%, ganz besonders bevorzugt 13 bis 15 Gew.-% Glutaminsäure-*N,N*-diessigsäure und/oder Methylglycindiessigsäure und/oder deren Salze, bevorzugt 10 bis 20 Gew.-%, vorzugsweise 11 bis 18 Gew.-% und insbesondere 12 bis 15,5 Gew.-%, ganz besonders bevorzugt 13 bis 15 Gew.-% Methylglycindiessigsäure und/oder deren Salze, insbesondere ihres Trinatriumsalzes enthält.

In einer bevorzugten erfindungsgemäßen Ausführungsform enthält eine der Reinigungsmittelzubereitung, bevorzugt Reinigungsmittelzubereitung B, weiterhin mindestens ein Tensid, insbesondere ausgewählt aus anionischen, nichtionischen, zwitterionischen und amphoteren Tensiden. Alternativ können die Tenside auch in einer von den Reinigungsmittelzubereitungen A und B verschiedenen Reinigungsmittelzubereitung enthalten sein. Tenside sind in einer erfindungsgemäßen Reinigungsmittelzubereitung B, soweit eingesetzt, vorzugsweise in einer Menge bis zu 40 Gew.-%, insbesondere 2 bis 40 Gew.-% oder 4 bis 35 Gew.-%, besonders bevorzugt in einer Menge von 5 bis 30 Gew.-%, insbesondere 8 bis 25 Gew.-%, enthalten.

Bevorzugt enthält Reinigungsmittelzubereitung A weniger als 2 Gew.-% Tensid, bevorzugt weniger als 1 Gew.-% Tensid, besonders bevorzugt weniger als 1 Gew.-% Tensid, insbesondere kein Tensid, jeweils bezogen auf das Gesamtgewicht der Reinigungsmittelzubereitung A.

Als nichtionische Tenside werden vorzugsweise alkoxylierte, vorteilhafterweise ethoxylierte, insbesondere primäre Alkohole mit vorzugsweise 8 bis 18 C-Atomen und durchschnittlich 1 bis 12 Mol Ethylenoxid (EO) pro Mol Alkohol eingesetzt, in denen der Alkoholrest linear oder bevorzugt in 2-Stellung methylverzweigt sein kann bzw. lineare und methylverzweigte Reste im Gemisch enthalten kann, so wie sie üblicherweise in Oxoalkoholresten vorliegen. Insbesondere sind jedoch Alkoholethoxylate mit linearen Resten aus Alkoholen nativen Ursprungs mit 12 bis 18 C-Atomen, zum Beispiel aus Kokos-, Palm-, Talgfett- oder Oleylalkohol, und durchschnittlich 2 bis 8 EO pro Mol Alkohol bevorzugt. Zu den bevorzugten ethoxylierten Alkoholen gehören beispielsweise C₁₂₋₁₄-Alkohole mit 3 EO, 4 EO oder 7 EO, C₉₋₁₁-Alkohol mit 7 EO, C₁₃₋₁₅-Alkohole mit 3 EO, 5 EO, 7 EO oder 8 EO, C₁₂₋₁₈-Alkohole mit 3 EO, 5 EO oder 7 EO und Mischungen aus diesen, wie Mischungen aus C₁₂₋₁₄-Alkohol mit 3 EO und C₁₂₋₁₈-Alkohol mit 7 EO. Die angegebenen Ethoxylierungsgrade stellen statistische Mittelwerte dar, die für ein spezielles Produkt eine ganze oder eine gebrochene Zahl sein können. Bevorzugte Alkoholethoxylate weisen eine eingeengte Homologenverteilung auf (narrow range ethoxylates, NRE). Zusätzlich zu diesen nichtionischen Tensiden können auch Fettalkohole mit mehr als 12 EO eingesetzt werden. Beispiele hierfür sind Talgfettalkohol mit 14 EO, 25 EO, 30 EO oder 40 EO. Auch nichtionische Tenside, die EO- und PO-Gruppen zusammen im Molekül enthalten, sind erfindungsgemäß einsetzbar. Hierbei können Blockcopolymere mit EO-PO-Blockeinheiten bzw. PO-EO-Blockeinheiten eingesetzt werden, aber auch EO-PO-EO-Copolymere bzw. PO-EO-PO-Copolymere. Selbstverständlich sind auch gemischt alkoxylierte Niotenside einsetzbar, in denen EO- und PO-Einheiten nicht blockweise, sondern statistisch verteilt sind. Solche Produkte sind durch gleichzeitige Einwirkung von Ethylen- und Propylenoxid auf Fettalkohole erhältlich.

Der Gehalt an nichtionischen Tensiden in der Reinigungszubereitung B beträgt in einer bevorzugten Ausführungsform 5 bis 30 Gew.-%, vorzugsweise 8 bis 25 Gew.-% und insbesondere 9 bis 15 Gew.-%, bezogen auf die Gesamtmenge der Reinigungszubereitung B.

Neben den nichtionischen Tensiden kann die Reinigungszubereitung B auch anionische Tenside enthalten. Als anionische Tenside werden beispielsweise solche vom Typ der Sulfonate und Sulfate eingesetzt. Als Tenside vom Sulfonat-Typ kommen dabei vorzugsweise C₉₋₁₃-Alkylbenzolsulfonate, Olefinsulfonate, d.h. Gemische aus Alken- und Hydroxyalkansulfonaten sowie Disulfonaten, wie man sie beispielsweise aus C₁₂₋₁₈-Monoolefinen mit end- oder innenständiger Doppelbindung durch Sulfonieren mit gasförmigem Schwefeltrioxid und anschließende alkalische oder saure Hydrolyse der Sulfonierungsprodukte erhält, in Betracht. Geeignet sind auch Alkansulfonate, die aus C₁₂₋₁₈-Alkanen beispielsweise durch Sulfochlorierung oder Sulfoxidation mit anschließender Hydrolyse bzw. Neutralisation gewonnen werden. Ebenso sind auch die Ester von α-Sulfofettsäuren (Estersulfonate), zum Beispiel die α-sulfonierten Methylester der hydrierten Kokos-, Palmkern- oder Talgfettsäuren geeignet. Die anionischen Tenside einschließlich der Seifen können in Form ihrer Natrium-, Kalium- oder Ammoniumsalze sowie als lösliche Salze organischer Basen, wie Mono-, Di- oder Triethanolamin, vorliegen. Vorzugsweise liegen die anionischen Tenside in Form ihrer Natrium- oder Kaliumsalze, insbesondere in Form der Natriumsalze vor.

Der Gehalt der Reinigungszubereitung B an anionischen Tensiden beträgt in einer bevorzugten Ausführungsform 0,1 bis 30 Gew.-%, vorzugsweise 2 bis 20 Gew.-%, bezogen auf die Gesamtmenge der Reinigungszubereitung A.

Ein bevorzugter pH-Wert erfindungsgemäßer Reinigungszubereitungen A liegt zwischen 9 und 14, insbesondere 9 und 12. Die Einstellung des pH-Wertes kann, falls erforderlich, durch entsprechende pH-Stellmittel, insbesondere Natrium- oder Kaliumhydroxid, erfolgen.

Die erfindungsgemäßen Reinigungsmittelzubereitungen B enthalten als ihren ersten wesentlichen Bestandteil mindestens ein reinigungsaktives Enzym. Der Gewichtsanteil der reinigungsaktiven Enzymzubereitung am Gesamtgewicht der Reinigungsmittelzubereitung B beträgt vorzugsweise zwischen 5 und 80 Gew.-%, bevorzugt zwischen 5 und 60 Gew.-%, besonders bevorzugt zwischen 10 und 50 Gew.-% und insbesondere zwischen 10 und 30 Gew.-%.

Zu den mit besonderem Vorzug eingesetzten Enzymen zählen dabei insbesondere Proteasen, Amylasen, Lipasen, Hemicellulasen, Cellulasen, Perhydrolasen oder Oxidoreduktasen, sowie vorzugsweise deren Gemische. Diese Enzyme sind im Prinzip natürlichen Ursprungs; ausgehend von den natürlichen Molekülen stehen für den Einsatz in Reinigungsmitteln verbesserte Varianten zur Verfügung, die entsprechend bevorzugt eingesetzt werden. Reinigungsmittel enthalten Enzyme vorzugsweise in Gesamtmengen von 1 x 10⁻⁶ bis 5 Gew.-% bezogen auf aktives Protein. Die Proteinkonzentration kann mit Hilfe bekannter Methoden, zum Beispiel dem BCA-Verfahren oder dem Biuret-Verfahren bestimmt werden.

Die erfindungsgemäße stabilisierende Wirkung wurde in besonderem Maße neben den Proteasen, bei den Amylasen, Cellulasen und Mannanasen beobachtet, weshalb erfindungsgemäße flüssige Reinigungsmittelzubereitungen B, dadurch gekennzeichnet, dass diese mindestens ein reinigungsaktives Enzym aus der Gruppe der Amylasen und/oder Proteasen und/oder Cellulasen und/oder Mannanasen, insbesondere aus der Gruppe der Amylasen und/oder Proteasen, enthalten, bevorzugt werden.

Beispiele für Amylasen sind die α-Amylasen aus *Bacillus licheniformis, Bacillus amyloliquefaciens* oder *Bacillus stearothermophilus* sowie insbesondere auch deren für den Einsatz in Wasch- oder Reinigungsmitteln verbesserte Weiterentwicklungen. Das Enzym aus *Bacillus licheniformis* ist von dem Unternehmen Novozymes unter dem Namen Termamyl® und von dem Unternehmen Danisco/Genencor unter dem Namen Purastar® ST erhältlich. Weiterentwicklungsprodukte dieser α-Amylase sind von dem Unternehmen Novozymes unter den Handelsnamen Duramyl® und Termamyl® ultra, von dem Unternehmen Danisco/Genencor unter dem Namen Purastar® OxAm und von dem Unternehmen Daiwa Seiko Inc. als Keistase® erhältlich. Die α-Amylase von *Bacillus amyloliquefaciens* wird von dem Unternehmen Novozymes unter dem Namen BAN® vertrieben, und abgeleitete Varianten von der α-Amylase aus *Bacillus stearothermophilus* unter den Namen BSG® und Novamyl®, ebenfalls von dem Unternehmen Novozymes. Des Weiteren sind für diesen Zweck die α-Amylase aus *Bacillus sp.* A 7- 7 (DSM 12368) und die Cyclodextrin-Glucanotransferase (CGTase) aus *Bacillus agaradherens* (DSM 9948) hervorzuheben. Ferner sind die amylolytischen Enzyme einsetzbar, die in den internationalen Patentanmeldungen WO2003002711, WO2003054177 und WO2007079938 offenbart sind, auf deren Offenbarung daher ausdrücklich verwiesen wird bzw. deren diesbezüglicher Offenbarungsgehalt daher ausdrücklich in die vorliegende Patentanmeldung mit einbezogen wird. Ebenso sind Fusionsprodukte aller genannten Moleküle einsetzbar. Darüber hinaus sind die unter den Handelsnamen Fungamyl® von dem Unternehmen Novozymes erhältlichen Weiterentwicklungen der α-Amylase aus *Aspergillus niger* und *A. oryzae* geeignet. Weitere vorteilhaft einsetzbare Handelsprodukte sind z.B. die Amylase-LT® sowie Stainzyme® oder Stainzyme ultra® bzw. Stainzyme plus® ebenfalls von dem Unternehmen Novozymes. Auch durch Punktmutationen erhältliche Varianten dieser Enzyme können erfindungsgemäß eingesetzt werden.

Die erfindungsgemäß in den Wasch- oder Reinigungsmitteln eingesetzte α-Amylase ist vorzugsweise ausgewählt aus:
a) einer α-Amylase, die eine Aminosäuresequenz umfasst, die zu der in SEQ ID NO:5 angegebenen Aminosäuresequenz über deren Gesamtlänge zu mindestens 80%, bevorzugt mindestens 81%, 82%, 83%, 84%, 85%, 86%, 87%, 88%, 89%, 90%, 90,5%, 91%, 91,5%, 92%, 92,5%, 93%, 93,5%, 94%, 94,5%, 95%, 95,5%, 96%, 96,5%, 97%, 97,5%, 98%, 98,5% und 98,8% identisch ist und optional mindestens eine Aminosäuresubstitution an einer der Positionen 172, 202, 208, 255 und 261 in der Zählung gemäß SEQ ID NO:5 aufweist, vorzugsweise ausgewählt aus der aus M202L, M202V, M202S, M202T, M202I, M202Q, M202W, S255N, R172Q und Kombinationen davon bestehenden Gruppe; und/oder
b) einer α-Amylase, die eine Aminosäuresequenz umfasst, die zu der in SEQ ID NO:6 angegebenen Aminosäuresequenz über deren Gesamtlänge zu mindestens 60%, bevorzugt mindestens 61%, 62%, 63%, 64%, 65%, 66%, 67%, 68%, 69%, 70% 71%, 72%, 73%, 74%, 75%, 76%, 77%, 78%, 79%, 80%, 81%, 82%, 83%, 84%, 85%, 86%, 87%, 88%, 89%, 90%, 90,5%, 91%, 91,5%, 92%, 92,5%, 93%, 93,5%, 94%, 94,5%, 95%, 95,5%, 96%, 96,5%, 97%, 97,5%, 98%, 98,5% und 98,8% identisch ist und optional mindestens eine Aminosäuresubstitution an einer der Positionen 9, 26, 30, 33, 82, 37, 106, 118, 128, 133, 149, 150, 160, 178, 182, 186, 193, 195, 202, 203, 214, 231, 256, 257, 258, 269, 270, 272, 283, 295, 296, 298, 299, 303, 304, 305, 311, 314, 315, 318, 319, 320, 323, 339, 345, 361, 378, 383, 419, 421, 437, 441, 444, 445, 446, 447, 450, 458, 461, 471, 482 und 484 und/oder eine Deletion an einer der Positionen 183 und 184 in der Zählung gemäß SEQ ID NO:6 aufweist, vorzugsweise mindestens eine Aminosäuresubstitution an einer der Positionen 9, 26, 149, 182, 186, 202, 257, 295, 299, 323, 339 and 345, besonders bevorzugt aus der aus R118K, D183*, G184*, N195F, R320K, R458K und Kombinationen davon bestehenden Gruppe ausgewählt; und/oder
c) einer α-Amylase, die eine Aminosäuresequenz umfasst, die zu der in SEQ ID NO:7 angegebenen Aminosäuresequenz über deren Gesamtlänge zu mindestens 90% identisch ist und optional mindestens eine Substitution und/oder Deletion an einer der Positionen 93, 116, 118, 129, 133, 134, 140, 142, 146, 147, 149, 151, 152, 169, 174, 183, 184, 186, 189, 193, 195, 197, 198, 200, 203, 206, 210, 212, 213, 235, 243, 244, 260, 262, 284, 303, 304, 320, 338, 347, 359, 418, 431, 434, 439, 447, 458, 469, 476 und 477 in der Zählung gemäß SEQ ID NO:7, aufweist.

In verschiedenen Ausführungsformen der Erfindung umfasst die Amylase eine Aminosäuresequenz umfasst, die zu der in SEQ ID NO:5 angegebenen Aminosäuresequenz über deren Gesamtlänge zu mindestens 80%, bevorzugt zu mindestens 81%, 82%, 83%, 84%, 85%, 86%, 87%, 88%, 89%, 90%, 90,5%, 91%, 91,5%, 92%, 92,5%, 93%, 93,5%, 94%, 94,5%, 95%, 95,5%, 96%, 96,5%, 97%, 97,5%, 98%, 98,5% und 98,8% identisch ist und optional mindestens eine Aminosäuresubstitution an einer der Positionen 172, 202, 208, 255 und 261 in der Zählung gemäß SEQ ID NO:5 aufweist, vorzugsweise ausgewählt aus der aus M202L, M202V, M202S, M202T, M202I, M202Q, M202W, S255N, R172Q und Kombinationen davon bestehenden Gruppe. Bevorzugt werden Amylasen eingesetzt, die an zwei, bevorzugt drei, der vorstehend genannten Positionen eine Aminosäuresubstitution aufweisen, insbesondere eine Substitution an Position 202 ausgewählt aus M202L, M202V, M202S, M202T, M202I, M202Q, M202W, eine Substitution an Position 255, insbesondere S255N, und eine Substitution an Position 172, insbesondere R172Q. Ganz besonders bevorzugt sind die M202L und M202T Mutanten.

In verschiedenen Ausführungsformen der Erfindung umfasst die Amylase eine Aminosäuresequenz, die zu der in SEQ ID NO:6 angegebenen Aminosäuresequenz über deren Gesamtlänge zu mindestens 60%, bevorzugt mindestens 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 90,5%, 91%, 91,5%, 92%, 92,5%, 93%, 93,5%, 94%, 94,5%, 95%, 95,5%, 96%, 96,5%, 97%, 97,5%, 98%, 98,5% und 98,8% identisch ist und optional mindestens eine Aminosäuresubstitution an einer der Positionen 9, 26, 30, 33, 82, 37, 106, 118, 128, 133, 149, 150, 160, 178, 182, 186, 193, 195, 202, 203, 214, 231, 256, 257, 258, 269, 270, 272, 283, 295, 296, 298, 299, 303, 304, 305, 311, 314, 315, 318, 319, 320, 323, 339, 345, 361, 378, 383, 419, 421, 437, 441, 444, 445, 446, 447, 450, 458, 461, 471, 482 und 484 und/oder eine Deletion an einer der Positionen 183 und 184 in der Zählung gemäß SEQ ID NO:6 aufweist, vorzugsweise mindestens eine Aminosäuresubstitution an einer der Positionen 9, 26, 149, 182, 186, 202, 257, 295, 299, 323, 339 and 345, besonders bevorzugt aus der aus R118K, D183*, G184*, N195F, R320K, R458K und Kombinationen davon bestehenden Gruppe ausgewählt. In verschiedenen bevorzugten Ausführungsformen weist die Amylase in der Zählung gemäß SEQ ID NO:6 Aminosäuresubstitutionen an drei oder mehr der Positionen 9, 26, 149, 182, 186, 202, 257, 295, 299, 323, 339 and 345 und optional eine oder mehrere, vorzugsweise alle, der Substitutionen und/oder Deletionen an den Positionen: 118, 183, 184, 195, 320 und 458, besonders bevorzugt R118K, D183*, G184*, N195F, R320K und/oder R458K, auf. In besonders bevorzugten Ausführungsformen weist die Amylase in der Zählung gemäß SEQ ID NO:6 folgende Aminosäuresubstitutionen und/oder Deletionen auf: M9L + M323T; M9L + M202L/T/V/I + M323T; M9L + N195F + M202L/T/V/I + M323T; M9L + R118K + D183* + G184* + R320K + M323T + R458K; M9L + R118K + D183* + G184* + M202L/T/V/I + R320K + M323T + R458K; M9L + G149A + G182T + G186A + M202L + T257I + Y295F + N299Y + M323T + A339S + E345R; M9L + G149A + G182T + G186A + M202I + T257I + Y295F + N299Y + M323T + A339S + E345R; M9L + R118K + G149A + G182T + D183* + G184* + G186A + M202L + T257I + Y295F + N299Y + R320K + M323T + A339S + E345R + R458K; M9L + R118K + G149A + G182T + D183* + G184* + G186A + N195F + M202L + T257I + Y295F + N299Y + R320K + M323T + A339S + E345R + R458K; M9L + R118K + G149A + G182T + D183* + G184* + G186A + M202I + T257I + Y295F + N299Y + R320K + M323T + A339S + E345R + R458K; M9L + R118K + D183* + D184* + N195F + M202L + R320K + M323T + R458K; M9L + R118K + D183* + D184* + N195F + M202T + R320K + M323T + R458K; M9L + R118K + D183* + D184* + N195F + M202I + R320K + M323T + R458K; M9L + R118K + D183* + D184* + N195F + M202V + R320K + M323T + R458K; M9L + R118K + N150H + D183* + D184* + N195F + M202L + V214T + R320K + M323T + R458K; oder M9L + R118K + D183* + D184* + N195F + M202L + V214T + R320K + M323T + E345N + R458K.

Eine besonders bevorzugte Amylase ist die Variante, die unter dem Handelnamen Stainzyme Plus™ kommerziell erhältlich ist (Novozymes A/S, Bagsvaerd, Dänemark).

In verschiedenen Ausführungsformen der Erfindung umfasst die Amylase eine Aminosäuresequenz umfasst, die zu der in SEQ ID NO:7 angegebenen Aminosäuresequenz über deren Gesamtlänge zu mindestens 90%, bevorzugt mindestens 90,5%, 91%, 91,5%, 92%, 92,5%, 93%, 93,5%, 94%, 94,5%, 95%, 95,5%, 96%, 96,5%, 97%, 97,5%, 98%, 98,5% und 98,8% identisch ist und optional mindestens eine Substitution und/oder Deletion an einer der Positionen 93, 116, 118, 129, 133, 134, 140, 142, 146, 147, 149, 151, 152, 169, 174, 183, 184, 186, 189, 193, 195, 197, 198, 200, 203, 206, 210, 212, 213, 235, 243, 244, 260, 262, 284, 303, 304, 320, 338, 347, 359, 418, 431, 434, 439, 447, 458, 469, 476 und 477 in der Zählung gemäß SEQ ID NO:7, aufweist. Diesbezüglich bevorzugte Aminosäuresubstitutionen umfassen: E260A/D/C/Q/L/M/F/P/S/W/V/G/H/I/K/N/R/T/Y, G304R/K/E/Q, W140Y/F, W189E/G/T, D134E, F262G/P, W284D/H/F/Y/R, W347H/F/Y, W439R/G, G476E/Q/R/K, G477E/Q/K/M/R, N195F/Y, N197F/L, Y198N, Y200F, Y203F, I206H/L/N/F/Y, H210Y, E212V/G, V213A, M116T, Q129L, G133E, E134Y, K142R, P146S, G147E, G149R, N151R, Y152H, Q169E, N174R, A186R, Y243F, S244Q, G303V, R320N, R359I, N418D und A447V.

In verschiedenen bevorzugten Ausführungsformen können erfindungsgemäße Wasch- oder Reinigungsmittel mindestens eine zweite Amylase und/oder mindestens eine zweite Protease enthalten, wobei die zweite Amylase unterschiedlich zur ersten Amylase ist und aus der oben genannten Gruppe ausgewählt ist und wobei die zweite Protease unterschiedlich zu ersten Protease ist.

Wenn zwei Amylasen enthalten sind, können diese vorzugsweise im Massenverhältnis von 50:1 bis 1:50, vorzugsweise 30:1 bis 1:10 (jeweils bezogen auf die Menge Aktivprotein Amylase 1 zu Amylase 2) eingesetzt werden. Es ist insbesondere bevorzugt, eine erste Amylase im Verhältnis zu einer zweiten Amylase von 20:1 bis 2:1, bevorzugt 15:1 bis 3:1, besonders bevorzugt 12:1 bis 5:1, beispielsweise 10:1 einzusetzen.

Weitere erfindungsgemäß bevorzugte flüssige Reinigungszubereitungen B enthalten, bezogen auf das Gesamtgewicht der Reinigungsmittelzubereitung, 0,1 bis 30 Gew.-%, vorzugsweise 1,0 bis 25 Gew.-% und insbesondere 2,0 bis 20 Gew.-% Cellulase-Zubereitungen.

Weitere erfindungsgemäß bevorzugte flüssige Reinigungszubereitungen B enthalten, bezogen auf das Gesamtgewicht der Reinigungsmittelzubereitung, 0,1 bis 30 Gew.-%, vorzugsweise 1,0 bis 25 Gew.-% und insbesondere 2,0 bis 20 Gew.-% Mannanase-Zubereitungen.

Erfindungsgemäß einsetzbar sind weiterhin Lipasen oder Cutinasen, insbesondere wegen ihrer Triglycerid-spaltenden Aktivitäten, aber auch, um aus geeigneten Vorstufen *in situ* Persäuren zu erzeugen. Hierzu gehören beispielsweise die ursprünglich aus *Humicola lanuginosa* (*Thermomyces lanuginosus*) erhältlichen, beziehungsweise weiterentwickelten Lipasen, insbesondere solche mit dem Aminosäureaustausch D96L. Desweiteren sind beispielsweise die Cutinasen einsetzbar, die ursprünglich aus *Fusarium solani pisi* und *Humicola insolens* isoliert worden sind. Einsetzbar sind weiterhin Lipasen, beziehungsweise Cutinasen, deren Ausgangsenzyme ursprünglich aus *Pseudomonas mendocina* und *Fusarium solanii* isoliert worden sind.

Weitere erfindungsgemäß bevorzugte flüssige Reinigungszubereitungen B enthalten, bezogen auf das Gesamtgewicht der Reinigungsmittelzubereitung, 0,1 bis 30 Gew.-%, vorzugsweise 1,0 bis 25 Gew.-% und insbesondere 2,0 bis 20 Gew.-% Lipase-Zubereitungen.

Weiterhin können Enzyme eingesetzt werden, die unter dem Begriff Hemicellulasen zusammengefaßt werden. Neben der bereits zuvor genannten Mannanase gehören hierzu beispielsweise Xanthanlyasen, Pektinlyasen (=Pektinasen), Pektinesterasen, Pektatlyasen, Xyloglucanasen (=Xylanasen), Pullulanasen und β-Glucanasen.

Zur Erhöhung der bleichenden Wirkung können erfindungsgemäß Oxidoreduktasen, beispielsweise Oxidasen, Oxygenasen, Katalasen, Peroxidasen, wie Halo-, Chloro-, Bromo-, Lignin-, Glucose- oder Mangan-peroxidasen, Dioxygenasen oder Laccasen (Phenoloxidasen, Polyphenoloxidasen) eingesetzt werden. Vorteilhafterweise werden zusätzlich vorzugsweise organische, besonders bevorzugt aromatische, mit den Enzymen wechselwirkende Verbindungen zugegeben, um die Aktivität der betreffenden Oxidoreduktasen zu verstärken (Enhancer) oder um bei stark unterschiedlichen Redoxpotentialen zwischen den oxidierenden Enzymen und den Anschmutzungen den Elektronenfluss zu gewährleisten (Mediatoren).

Reinigungsaktive Enzyme, insbesondere Proteasen und Amylasen, werden in der Regel nicht in Form des reinen Proteins sondern vielmehr in Form stabilisierter, lager- und transportfähiger Zubereitungen bereitgestellt. Zu diesen vorkonfektionierten Zubereitungen zählen beispielsweise die durch Granulation, Extrusion oder Lyophilisierung erhaltenen festen Präparationen oder, insbesondere bei flüssigen oder gelförmigen Mitteln, Lösungen der Enzyme, vorteilhafterweise möglichst konzentriert, wasserarm und/oder mit Stabilisatoren oder weiteren Hilfsmitteln versetzt. Alternativ können die Enzyme sowohl für die feste als auch für die flüssige Darreichungsform verkapselt werden, beispielsweise durch Sprühtrocknung oder Extrusion der Enzymlösung zusammen mit einem vorzugsweise natürlichen Polymer oder in Form von Kapseln, beispielsweise solchen, bei denen die Enzyme wie in einem erstarrten Gel eingeschlossen sind oder in solchen vom Kern-Schale-Typ, bei dem ein enzymhaltiger Kern mit einer Wasser-, Luft- und/oder Chemikalien-undurchlässigen Schutzschicht überzogen ist. In aufgelagerten Schichten können zusätzlich weitere Wirkstoffe, beispielsweise Stabilisatoren, Emulgatoren, Pigmente, Bleich- oder Farbstoffe aufgebracht werden. Derartige Kapseln werden nach an sich bekannten Methoden, beispielsweise durch Schüttel- oder Rollgranulation oder in Fluid-bed-Prozessen aufgebracht. Vorteilhafterweise sind derartige Granulate, beispielsweise durch Aufbringen polymerer Filmbildner, staubarm und aufgrund der Beschichtung lagerstabil.

Weiterhin ist es möglich, zwei oder mehrere Enzyme zusammen zu konfektionieren, so dass ein einzelnes Granulat mehrere Enzymaktivitäten aufweist.

Erfindungsgemäß besonders bevorzugte flüssige Reinigungsmittelzubereitungen B enthalten daher, bezogen auf das Gesamtgewicht der Reinigungsmittelzubereitung, 7 bis 40 Gew.-%, insbesondere 10 bis 30 Gew.-% Protease-Zubereitungen und 2 bis 20 Gew.-%, insbesondere 4,0 bis 16 Gew.-% Amylase-Zubereitungen eingesetzt, welche jeweils 0,4 bis 20 Gew.-%, insbesondere von 0,8 bis 10 Gew.-% aktives Protein enthalten.

Bevorzugt werden mehrere Enzyme und/oder Enzymzubereitungen, vorzugsweise flüssige Protease-Zubereitungen und/oder Amylase-Zubereitungen, sowie optional Cellulase-Zubereitungen und/oder Mannanase-Zubereitungen eingesetzt.

Ein bevorzugter pH-Wert erfindungsgemäßer Reinigungsmittelzubereitungen B liegt zwischen 6 und 9.

Die Reinigungsmittelzubereitungen B der erfindungsgemäßen Reinigungsmittelangebotsformen enthalten bevorzugt weniger als 2,5 Gew.-% Komplexbildner. Dabei enthalten sie bevorzugt weniger als 2,5 Gew.-% Komplexbildner und/oder Gerüststoffe. Die Absenkung des Komplexbildner-Gehalts unter diese Obergrenzen hat sich als für die Reinigungsleistung als vorteilhaft erwiesen. Durch eine weitere Absenkung des Gehaltes an Komplexbildner deutlich unterhalb die Obergrenzen ist überraschenderweise eine weitere Steigerung der Reinigungsleistung erfindungsgemäßer Reinigungsmittelangebotsformen realisierbar.

Entsprechend sind erfindungsgemäß bevorzugte Reinigungsmittelangebotsformen dadurch gekennzeichnet, dass die Reinigungsmittelzubereitung B weniger als 2,0 Gew.-% Komplexbildner, vorzugsweise weniger als 1,0 Gew.-% Komplexbildner, besonders bevorzugt weniger als 0,5 Gew.-% Komplexbildner und insbesondere keine Komplexbildner enthält.

Die Gesamtmenge des in der Reinigungsmittelzubereitung B enthaltenen Komplexbildners und/oder Gerüststoffe beträgt bevorzugt weniger als 10 Gew.-%, vorzugsweise weniger als 6 Gew.-%, besonders bevorzugt weniger als 2 Gew.-% und insbesondere 0 Gew.-%.

Ein optionaler Bestandteil der erfindungsgemäßen Reinigungsmittelzubereitungen, insbesondere der Reinigungsmittelzubereitung B, sind organische Lösungsmittel. Bevorzugte organische Lösungsmittel stammen aus der Gruppe der ein- oder mehrwertigen Alkohole, Alkanolamine oder Glykolether. Vorzugsweise sind die Lösungsmittel ausgewählt aus Ethanol, n- oder i-Propanol, Butanol, Glykol, Propan- oder Butandiol, Glycerin, Monoethanolamin, Diglykol, Propyl- oder Butyldiglykol, Hexylenglycol, Ethylenglykolmethylether, Ethylenglykolethylether, Ethylenglykolpropylether, Etheylenglykolmono-n-butylether, Diethylenglykolmethylether, Diethylenglykolethylether, Propylenglykolmethyl-, -ethyl- oder -propylether, Dipropylenglykolmethyl-, oder -ethylether, Methoxy-, Ethoxy- oder Butoxytriglykol, 1-Butoxyethoxy-2-propanol, 3-Methyl-3-methoxybutanol, Propylen-glykol-t-butylether sowie Mischungen dieser Lösungsmittel. Bevorzugte Lösungsmittel sind vorzugsweise ausgewählt aus Glycerin, 1,2-Propylenglycol, 1,3-Propylenglycol, Dipropylenglycol sowie Polyethylenglycolen, insbesondere solchen Polyethylenglycolen, welche mittleres Molekulargewicht zwischen 100 und 800, bevorzugt 200 und 600 g/mol aufweisen. Der Gewichtsanteil dieser organischen Lösungsmittel am Gesamtgewicht der jeweiligen erfindungsgemäßen Reinigungsmittelzubereitungen beträgt vorzugsweise 5 bis 80 Gew.-%, bevorzugt 10 bis 60 Gew.-% und insbesondere 20 bis 50 Gew.-%.

Ein besonders bevorzugtes und in Bezug auf die Stabilisierung der Reinigungsmittelzubereitung, insbesondere der Reinigungsmittelzubereitung B, besonders wirksames organisches Lösungsmittel ist das 1,2-Propylenglykol. Der Gewichtsanteil des 1,2-Propylenglykols am Gesamtgewicht der erfindungsgemäßen Reinigungsmittelzubereitungen B kann in weiten Grenzen variieren, jedoch haben sich solche Zubereitungen als besonders stabil erwiesen, die, bezogen auf das Gesamtgewicht der jeweiligen Reinigungsmittelzubereitung B, 5 bis 80 Gew.-%, vorzugsweise 10 bis 60 Gew.-% und insbesondere 20 bis 50 Gew.-% 1,2-Propylenglykol enthalten. Entsprechende Zubereitungen werden daher erfindungsgemäß bevorzugt.

Ein weiterer optionaler Bestandteil der erfindungsgemäßen Reinigungsmittelzubereitungen B ist eine Borsäure bzw. ein Borsäurederivat. Neben der Borsäure werden dabei vorzugsweise insbesondere die Boronsäuren oder deren Salze oder Ester eingesetzt, darunter vor allem Derivate mit aromatischen Gruppen, etwa ortho-, meta- oder para-substituierte Phenylboronsäuren, insbesondere 4-Formylphenyl-Boronsäure (4-FPBA), beziehungsweise die Salze oder Ester der genannten Verbindungen. Der Gewichtsanteil der Borsäure bzw. der Borsäurederivate am Gesamtgewicht erfindungsgemäßer Reinigungsmittelzubereitungen B beträgt vorzugsweise zwischen 0,001 bis 10 Gew.-%, bevorzugt 0,002 bis 6 Gew.-% und insbesondere 0,05 bis 3 Gew.-%.

Ein besonders bevorzugtes und in Bezug auf die Stabilisierung der Reinigungsmittelzubereitung besonders wirksames Borsäurederivat ist 4-Formylphenyl-Boronsäure. Der Gewichtsanteil der 4-Formylphenyl-Boronsäure am Gesamtgewicht der erfindungsgemäßen Reinigungsmittelzubereitungen kann in weiten Grenzen variieren, jedoch haben sich solche Zubereitungen als besonders stabil erwiesen, die, bezogen auf das Gesamtgewicht der Reinigungsmittelzubereitung B, 0,001 bis 10 Gew.-%, bevorzugt 0,002 bis 6 Gew.-% und insbesondere 0,05 bis 3 Gew.-% enthalten. Entsprechende Zubereitungen werden daher erfindungsgemäß bevorzugt.

Ein weiterer optionaler Bestandteil der erfindungsgemäßen Reinigungsmittelzubereitungen ist eine Ca- oder Mg-Ionenquelle. Der Gewichtsanteil der Ca- oder Mg-Ionenquelle am Gesamtgewicht erfindungsgemäßer Reinigungsmittelzubereitungen B beträgt vorzugsweise zwischen 0,01 bis 10 Gew.-%, bevorzugt 0,2 bis 8 Gew.-% und insbesondere 0,5 bis 5 Gew.-%.

Als besonders bevorzugte und in Bezug auf die Stabilisierung der Reinigungsmittelzubereitung B besonders wirksame Ca-Ionenquellen haben sich die organischen Calciumsalze erwiesen.

Der Gewichtsanteil der organischen Calciumsalze am Gesamtgewicht der erfindungsgemäßen Reinigungsmittelzubereitungen kann in weiten Grenzen variieren, jedoch haben sich solche Zubereitungen als besonders stabil erwiesen, die, bezogen auf das Gesamtgewicht der Reinigungsmittelzubereitung, 0,01 bis 10 Gew.-%, bevorzugt 0,2 bis 8 Gew.-% und insbesondere 0,5 bis 5 Gew.-% enthalten. Entsprechende Zubereitungen werden daher erfindungsgemäß bevorzugt.

Zur Enzymstabilisierung können in erfindungsgemäßen Reinigungsmittelzubereitungen B weiterhin auch Polyole enthalten sein, insbesondere Sorbitol.

Die flüssigen Reinigungsmittelzubereitungen B enthalten bezogen auf ihr Gesamtgewicht vorzugsweise 30 Gew.-% und weniger, vorzugsweise 25 Gew.-% und weniger, insbesondere 15 Gew.-% und weniger Wasser. In einer weiteren bevorzugten Ausführungsform enthalten die Reinigungsmittelzubereitungen B bezogen auf ihr Gesamtgewicht 0,5 bis 30 Gew.-%, bevorzugt 1,0 bis 25 Gew.-% und insbesondere 1,5 bis 30 Gew.-% Wasser.

In einer bevorzugten Ausführungsform umfasst die Reinigungsmittelangebotsform weiterhin eine flüssige Reinigungsmittelzubereitung C, wobei die Reinigungsmittelzubereitung C von den Reinigungsmittelzubereitungen A und B verschieden ist.

In dem erfindungsgemäßen maschinellen Geschirrspülverfahren werden die Reinigungsmittelzubereitungen A und B und die Wirkstoffzusammensetzung D in einer bevorzugten Ausführungsform in Kombination mit mindestens einer weiteren Reinigungsmittelzubereitung C eingesetzt. Bei Einsatz in einem Geschirrspülverfahren ist diese Reinigungsmittelzubereitung C vorzugsweise Tensid- und/oder Säure-haltig, bevorzugt Tensid- und Säurehaltig.

Durch den Einsatz einer Tensid- und/oder Säure-haltigen Reinigungsmittelzubereitung C kann die in den erfindungsgemäßen Geschirrspülverfahren erzielte Klarspülleistung verbessert werden. Dies gilt insbesondere für solche bevorzugten Verfahrensvarianten, bei denen die Dosierung der Reinigungsmittelzubereitungen A, B und C zeitversetzt erfolgt. Als tensidische Zusatzstoffe für die Reinigungsmittelzubereitung C eignen sich insbesondere die weiter oben beschriebenen nichtionischen Tenside. Vorzugsweise werden jedoch nichtionische Tenside der allgemeinen Formel R¹-CH(OH)CH₂O-(AO)_{w}-(A'O)ₓ-(A"O)_{y}-(A"'O)_{z}-R², in der
- R¹ für einen geradkettigen oder verzweigten, gesättigten oder ein- bzw. mehrfach ungesättigten C₆₋₂₄-Alkyl- oder -Alkenylrest steht;
- R² für einen linearen oder verzweigten Kohlenwasserstoffrest mit 2 bis 26 Kohlenstoffatomen steht;
- A, A', A" und A'" unabhängig voneinander für einen Rest aus der Gruppe -CH₂CH₂, -CH₂CH₂-CH₂, -CH₂-CH(CH₃), -CH₂-CH₂-CH₂-CH₂, -CH₂-CH(CH₃)-CH₂-, -CH₂-CH(CH₂-CH₃) stehen,
   - w, x, y und z für Werte zwischen 0,5 und 120 stehen, wobei x, y und/oder z auch 0 sein können
      eingesetzt. Als besonders wirkungsvoll haben sich hierbei die nichtionischen Tenside der allgemeine Formel R¹-CH(OH)CH₂O-(AO)_{w}-R² erwiesen, in der
      - R¹ für einen geradkettigen oder verzweigten, gesättigten oder ein- bzw. mehrfach ungesättigten C₆₋₂₄-Alkyl- oder -Alkenylrest steht;
      - R² für einen linearen oder verzweigten Kohlenwasserstoffrest mit 2 bis 26 Kohlenstoffatomen steht;
      - A für einen Rest aus der Gruppe CH₂CH₂, -CH₂CH₂-CH₂, -CH₂-CH(CH₃) steht, und
      - w für Werte zwischen 1 und 120, vorzugsweise 10 bis 80, insbesondere 20 bis 40 steht.

Zur Gruppe dieser nichtionischen Tenside zählen beispielsweise die C₄₋₂₂ Fettalkohol-(EO)₁₀₋₈₀-2-hydroxyalkylether, insbesondere auch die C₈₋₁₂ Fettalkohol-(EO)₂₂-2-hydroxydecylether und die C₄₋₂₂ Fettalkohot-(EO)₄₀₋₈₀-2-hydroxyalkylether.

Der Gewichtsanteil des nichtionischen Tensids am Gesamtgewicht der Reinigungsmittelzubereitung C beträgt vorzugsweise von 1,0 bis 20 Gew.-%, bevorzugt von 2,0 bis 18, besonders bevorzugt von 4,0 bis 15 Gew.-% und insbesondere von 6,0 bis 12 Gew.-%.

In einer weiteren besonders bevorzugten Ausführungsform enthält mindestens eine Reinigungsmittelzubereitung, insbesondere mindestens eine Reinigungsmittelzubereitung umfassend weiterhin ein nichtionisches Tensid, besonders bevorzugt zumindest die Reinigungsmittelzubereitung B und/oder D mindestens ein Hydrotrop (im weiteren auch als Lösevermittler bezeichnet). Bevorzugte Hydrotrope sind Xylolsulfonat, Cumolsulfonat, Harnstoff und/oder *N*-Methylacetamid, besonders bevorzugt Cumolsulfonat und/oder Xylolsulfonat, insbesondere Cumolsulfonat. Es wurde festgestellt, dass der Einsatz von Hydrotropen, insbesondere von Cumolsulfonat, die Phasenstabilität hinsichtlich der Temperaturschwankungen enorm verbessert. Insbesondere ist dies für solche Zubereitungen zu beobachten, welche mindestens ein nichtionisches Tensid enthalten. Insbesondere bevorzugt ist es, dass zumindest die Reinigungsmittelzubereitung C, insbesondere die Reinigungsmittelzubereitungen C und B mindestens ein Hydrotrop, bevorzugt Xylolsulfonat, Cumolsulfonat, Harnstoff und/oder N-Methylacetamid, besonders bevorzugt Cumolsulfonat und/oder Xylolsulfonat, insbesondere Cumolsulfonat., bevorzugt in einer Menge von 2 bis 25 Gew.-%, insbesondere von 4 bis 20 Gew.-% und besonders bevorzugt in einer Menge von 6 bis 15, beispielsweise von 7 bis 12 Gew.-%, bezogen auf das Gesamtgewicht der jeweiligen Reinigungsmittelzubereitung, enthält.

Bevorzugt beträgt das Gewichtsverhältnis des mindestens einen nichtionischen Tensids zu dem mindestens einen Hydrotrop, bevorzugt Xylolsulfonat, Cumolsulfonat, Harnstoff und/oder N-Methylacetamid, besonders bevorzugt Cumolsulfonat und/oder Xylolsulfonat, insbesondere Cumolsulfonat. 2:1 bis 1:2, insbesondere 1,6:1 bis 1:1.

In Ergänzung oder alternativ zu den nichtionischen Tensiden enthalten die erfindungsgemäßen Reinigungsmittelzubereitungen C bei Einsatz in einem Geschirrspülverfahren vorzugsweise mindestens ein Acidifizierungsmittel. Acidifizierungsmittel können den erfindungsgemäßen Reinigungsmittelzubereitungen C zugesetzt werden, um den pH-Wert der Flotte im Klarspülgang zu erniedrigen. Hier bieten sich sowohl anorganische Säuren als auch organische Säuren an, sofern diese mit den übrigen Inhaltsstoffen verträglich sind. Aus Gründen des Verbraucherschutzes und der Handhabungssicherheit sind insbesondere die festen Mono-, Oligo- und Polycarbonsäuren einsetzbar. Aus dieser Gruppe wiederum bevorzugt sind Ameisensäure, Citronensäure, Weinsäure, Bernsteinsäure, Malonsäure, Adipinsäure, Maleinsäure, Fumarsäure, Oxalsäure sowie Polyacrylsäure. Organische Sulfonsäuren wie Amidosulfonsäure sind ebenfalls einsetzbar. Kommerziell erhältlich und als Acidifizierungsmittel im Rahmen der vorliegenden Erfindung ebenfalls bevorzugt einsetzbar ist Sokalan® DCS (Warenzeichen der BASF), ein Gemisch aus Bernsteinsäure (max. 31 Gew.-%), Glutarsäure (max. 50 Gew.-%) und Adipinsäure (max. 33 Gew.-%). Reinigungsmittelzubereitungen C, die bezogen auf das Gesamtgewicht der Reinigungsmittelzubereitung C ein oder mehrere Acidifizierungsmittel, vorzugsweise Mono-, Oligo- und Polycarbonsäuren, besonders bevorzugt Ameisensäure, Weinsäure, Bernsteinsäure, Malonsäure, Adipinsäure, Maleinsäure, Fumarsäure, Oxalsäure sowie Polyacrylsäure und insbesondere Ameisensäure, Essigsäure und/oder Citronensäure in Mengen von 0,1 bis 12 Gew.-%, bevorzugt 0,2 bis 10 Gew.-% und insbesondere 0,3 bis 8,0 Gew.-% sind bevorzugte Ausführungsformen der vorliegenden Erfindung.

Der Einsatz von Ameisensäure ist bevorzugt, da sie neben der Säurefunktion zur Verbesserung des Klarspülergebnisses auch positiven Einfluss auf die Lagerstabilität der Reinigungszubereitung C hat, welche aufgrund der Lagerung im Innenraum der Spülmaschine, wie oben ausgeführt, starken Temperaturschwankungen unterworfen ist. Weiterhin weist sie eine desinfizierende Wirkung auf, so dass bei Anwendung von Ameisensäure im Klarspülgang, sowohl die Anzahl der Bakterien reduziert wird. Das gilt sowohl für solche Bakterien, welche sich in der Spülflotte des Klarspülgangs befinden als auch für solche, die während und nach dem Spülvorgang im Sumpf der Spülmaschine verbleibende Spülflotte als auch der Innenraum der Spülmaschine befinden, reduziert wird. Auch kann dadurch die Anzahl von Restkeimen auf dem gespülten Geschirr vermindert werden.

Besonders von Vorteil ist es, wenn eine Wirkstoffzusammensetzung D, insbesondere umfassend Duftstoffe und/oder Duftfänger, und gleichzeitig Ameisensäure als Acidifizierungsmittel in der Zubereitung C eingesetzt wird. Die Ameisensäure hat selbst einen leicht stechenden Geruch, der empfindlichen Verbrauchern unangenehm auffällt. Durch die abgetrennte Lagerung der Wirkstoffzusammensetzung und die Freisetzung des mindestens einen Wirkstoffs D, insbesondere wenn es sich dabei um ein oder mehrere Duftstoffe, insbesondere die vorstehend bevorzugten, und/oder ein oder mehrere Duftfänger, insbesondere z.B. Zinkricinoleat, handelt, entsteht weder während des Geschirrspülprozesses noch in der Zeit zwischen den Reinigungszyklen ein unangenehmer Geruch im Inneren der Spülmaschine.

Die zuvor beschriebenen Reinigungsmittelzubereitungen A, B und C unterscheiden sich hinsichtlich ihrer Zusammensetzung, sind also nicht identisch.

Weiterhin enthalten die erfindungsgemäßen Reinigungsmittelzubereitungen A, B und/oder C bei Einsatz in einem Geschirrspülverfahren vorzugsweise mindestens einen Glaskorrosionsinhibitor. Besonders bevorzugt enthalten die Zubereitung(en) A und/oder die Zubereitung(en) C eine entsprechende Menge an Glaskorrosionsinhibitor(en). Bevorzugt sind diese Glaskorrosionsinhibitoren ausgewählt aus wasserlöslichen Zinksalzen, bevorzugt Zinkchlorid, Zinksulfat und/oder Zinkacetat, besonders bevorzugt Zinkacetat, Polyalkyleniminien, insbesondere Polyethyleniminen.

Die erfindungsgemäßen Zubereitungen, insbesondere Zubereitungen A und/oder C, enthalten in einer bevorzugten Ausführungsform als weiteren Bestandteil mindestens ein Zinksalz, insbesondere anorganisch oder organisch, als Glaskorrosionsinhibitor. Das anorganische Zinksalz ist vorzugsweise ausgewählt aus der Gruppe bestehend aus Zinkbromid, Zinkchlorid, Zinkiodid, Zinknitrat und Zinksulfat. Das organische Zinksalz ist vorzugsweise ausgewählt aus der Gruppe bestehend aus Zinksalzen monomerer oder polymerer organischer Säuren, insbesondere aus der Gruppe Zinkacetat, Zinkacetylacetonat, Zinkbenzoat, Zinkformiat, Zinklactat, Zinkgluconat, Zinkricinoleat, Zinkabietat, Zinkvalerat und Zink-p-toluolsulfonat. In einer erfindungsgemäß besonders bevorzugten Ausführungsform wird als Zinksalz Zinkacetat eingesetzt.

Das Zinksalz ist in erfindungsgemäßen Reinigungsmittelzubereitungen vorzugsweise in einer Menge von 0,01 Gew.-% bis 5 Gew.-%, besonders bevorzugt in einer Menge von 0,05 Gew.-% bis 3 Gew.-%, insbesondere in einer Menge von 0,1 Gew.-% bis 2 Gew.-%, enthalten, bezogen auf das Gesamtgewicht der jeweiligen Reinigungsmittelzubereitung, insbesondere der jeweiligen Reinigungsmittelzubereitung A oder C.

Polyethylenimine, wie sie beispielsweise unter dem Namen Lupasol® (BASF) erhältlich sind, werden vorzugsweise in einer Menge von 0 bis 5 Gew.-%, insbesondere 0,01 bis 2 Gew.-%, bezogen auf das Gesamtgewicht der jeweiligen Zubereitung, als Glaskorrosionsinhibitoren eingesetzt werden.

Die Zusammensetzung einiger beispielhafter erfindungsgemäßer Reinigungsmittelkombinationen bzw. Reinigungsmittelangebotsformen, umfassend die Reinigungsmittelzubereitungen A, B und C kann der folgenden Tabelle entnommen werden.

**Tabelle 1:**

| | Rezeptur 1 | Rezeptur 2 | Rezeptur 3 | Rezeptur 4 |
|---|---|---|---|---|
| Inhaltsstoffe W.-u.R.-Zubereitung A | [Gew.-%] | [Gew.-%] | [Gew.-%] | [Gew.-%] |
| Gerüststoff | 2 bis 50 | 2 bis 50 | 2 bis 30 | 4 bis 30 |
| MGDA | 0bis 60 | 0 bis 30 | 0 bis 20 | 0 bis 15 |
| Phosphonate, sofern regulatorisch zulässig | 0 bis 10 | 1 bis 9 | 1,2 bis 8 | 1,5 bis 6 |
| Misc | ad 100 | ad 100 | ad 100 | ad 100 |

| Inhaltsstoffe W.-u.R.-Zubereitung B | | | | |
|---|---|---|---|---|
| Enzymzubereitung, mit erfindungsgemäßer Protease | mindestens 5 | mindestens 5 | mindestens 5 | mindestens 5 |
| Amylasezubereitung | mindestens 5 | mindestens 5 | mindestens 5 | mindestens 5 |
| Tenside | 2 bis 40 | 4 bis 40 | 5 bis 35 | 5 bis 35 |
| Komplexbildner | < 2,5 | < 2,5 | < 2,5 | < 2,5 |
| Misc | ad 100 | ad 100 | ad 100 | ad 100 |

| Inhaltsstoffe W.-u.R.-Zubereitung C | | | | |
|---|---|---|---|---|
| Tenside, bevorzugt nichtionische Tenside | 0-40 | 2,0-18 | 4,0-15 | 6,0-12,0 |
| Säure, bevorzugt Ameisensäure | 0,1-12 | 0,2-10 | 0,3-8,0 | 0,3-8,0 |
| Zinksalz | 0,01-5,0 | 0,05-3,0 | 0,05-3,0 | 0,1-2,0 |
| Hydrotrop, insbesondere Cumolsulfonat | 2-25 | 4-20 | 6-15 | 6-15 |
| Misc | ad 100 | ad 100 | ad 100 | ad 100 |

Die Konfektionierung der zuvor beschriebenen Kombination von Reinigungsmitteln erfolgt mittels eines Verpackungsmittels, in dem die Reinigungsmittelzubereitungen A und B bzw. A, B und C getrennt voneinander vorliegen. Diese Trennung kann beispielsweise durch voneinander getrennte Aufnahmekammern erreicht werden, wobei jede dieser Aufnahmekammern eines der miteinander kombinierten Reinigungsmittel enthält. Beispiele für derartige Konfektionsformen sind Kartuschen mit zwei, drei, vier oder mehr voneinander getrennten Aufnahmekammern, beispielsweise Zwei-, Drei-, Vier- oder Mehrkammerflaschen. Durch die Trennung der Reinigungsmittel unterschiedlicher Zusammensetzung können unerwünschte Reaktionen aufgrund chemischer Unverträglichkeit ausgeschlossen werden.

Bevorzugt beträgt die Viskosität aller Reinigungsmittelzubereitungen A und B bzw. A, B und C weniger als 120 mPas, insbesondere von 1 bis 100 mPas, insbesondere 10 bis 80 mPas, bevorzugt 20 bis 60 mPas (gemessen bei 20 °C mit einem Brookfield Instrument LVDV II+, Spindel 31, 100 rpm). Dies hat den Vorteil, dass die Reinigungsmittelzubereitungen aus dem Verpackungsmittel nur durch Öffnen eines Ventils auf der Unterseite des Verpackungsmittels (insbesondere der Kartusche) auf Basis der Schwerkraft, bevorzugt ohne die Beteiligung elektrischer oder elektronischer Mittel, wie z.B. Pumpen etc. dosiert werden können. Gleichzeitig entleeren sich die Kammern bevorzugt weitgehend vollständig, d.h. ohne größere Restmengen an den zu dosierenden Reinigungsmittelzusammensetzungen. Das ist für den Verbraucher und für die Umwelt vorteilhaft, weil nur geringe Mengen der Reinigungsmittelzubereitungen ungenutzt in den Kammern des Verpackungsmittels bzw. der Kartusche verbleiben.

Es ist ebenfalls erfindungsgemäß bevorzugt, dass das Verpackungsmaterial als wasserlöslicher oder wasserdispergierbarer Mehrkammerbeutel ausgestaltet ist, bei dem die Zubereitungen A und B sowie ggf. weitere Zubereitungen jeweils getrennt in den einzelnen Kammern vorliegen. Solche Mehrkammerbeutel und deren Herstellung sind im Stand der Technik hinlänglich beschrieben, beispielsweise in der WO2015/132279 A1, auf deren gesamte Offenbarung hiermit vollständig Bezug genommen wird. Die vorstehend und im weiteren genannten Ausführungsformen gelten ebenfalls für solche Mehrkammerbeutel enthaltend die flüssige Reinigungsmittelzubereitung allein sowie die Reinigungsmittelkombination aus Reinigungsmittelzubereitung A und Reinigungsmittelzubereitung B

Neben den Zubereitungen A und B befindet sich gemäß einer bevorzugten Ausführungsform in dem Verpackungsmittel zusätzlich mindestens eine, bevorzugt von den Reinigungsmittelzubereitungen, insbesondere von den Reinigungsmittelzubereitungen A und B, getrennt vorliegende Wirkstoffzusammensetzung befindet, die mindestens ein Trägermaterial, bevorzugt ein wasserunlösliches Trägermaterial, und mindestens einen Wirkstoff D enthält. Dabei handelt es sich bevorzugt um

In einer besonders bevorzugten Ausführungsform der vorliegenden Erfindung besteht das polymere Trägermaterial der Partikel wenigstens anteilsweise aus Ethylen/Vinylacetat-Copolymer. Ein weiterer bevorzugter Gegenstand der vorliegenden Anmeldung ist daher eine Reinigungsmittelangebotsform wie vorstehend beschrieben, dadurch gekennzeichnet, dass ein polymeres Trägermaterial mindestens 10 Gew.-%, vorzugsweise mindestens 30 Gew.-%, besonders bevorzugt mindestens 70 Gew.-% Ethylen/Vinylacetat-Copolymer enthält, vorzugsweise vollständig aus Ethylen/Vinylacetat-Copolymer hergestellt ist.

Ethylen/Vinylacetat-Copolymere ist die Bezeichnung für Copoylmere aus Ethylen und Vinylacetat. Die Herstellung dieses Polymers erfolgt grundsätzlich in einem der Herstellung von Polyethylen mit niedriger Dichte (LDPE; low density polyethylene) vergleichbaren Verfahren. Mit einem zunehmenden Anteil an Vinylacetat wird die Kristallinität des Polyethylens unterbrochen und auf diese Weise die Schmelz- und Erweichungspunkte bzw. die Härte der resultierenden Produkte herabgesetzt. Das Vinylacetat macht das Copolymer zudem polarer und verbessert damit dessen Adhäsion an polare Substrate.

Die vorstehend beschriebenen Ethylen/Vinylacetat-Copolymere sind kommerziell breit verfügbar, beispielsweise unter dem Warenzeichen Elvax® (Dupont). Im Rahmen der vorliegenden Erfindung besonders geeignete Polyvinylalkohole sind beispielsweise Elvax® 265, Elvax® 240, Elvax® 205 W, Elvax® 200 W sowie Elvax® 360. Weiter geeignet sind beispielsweise Produkte verfügbar unter dem Warenzeichen Evatane® (Arkema).

Im Rahmen der vorliegenden Erfindung, insbesondere im Bereich der Beduftung der Innenräume von maschinellen Geschirrspülmaschinen sind Wirkstoffzusammensetzungen besonders bevorzugt, in welchen als polymeres Trägermaterial Ethylen/Vinylacetat-Copolymer eingesetzt wird und dieses Copolymer 5 bis 50 Gew.-% Vinylacetat, vorzugsweise 10 bis 40 Gew.-% Vinylacetat und insbesondere 20 bis 30 Gew.-% Vinylacetat, jeweils bezogen auf das Gesamtgewicht des Copolymers, enthält.

Weitere geeignete Trägermaterialien sind die Cyclodextrine.

Alternativ oder in Ergänzung zu den vorgenannten Trägermaterialien werden mit Vorzug weiterhin anorganische Trägermaterialien eingesetzt. Insbesondere bevorzugt werden Reinigungsmittelangebotsformen, dadurch gekennzeichnet, dass es sich bei mindestens einem der Trägermaterialien um ein anorganisches Trägermaterial, vorzugsweise um ein Silikat, Phosphat oder Borat handelt.

Die Silikate, Phosphate oder Borate liegen dabei vorzugsweise in Form eines Glases, besonders bevorzugt in Form eines wasserlöslichen Glases vor. Besonders bevorzugtes Gläser sind Zink- und/oder bismuthhaltige Gläser, insbesondere Bismuthphosphathaltige und/oder Zinkphosphathaltige Gläser. In einem solchen Fall ist das Trägermaterial wasserlöslich und enthält in seiner Substanz bereits direkt den Wirkstoff, insbesondere die Glaskorrosionsinhibitoren Zink und/oder Bismuth) im Trägermaterial.

In einer bevorzugten Ausführungsform können die Reinigungsmittelangebotsformen solche Zink- oder Bismuthhaltigen Gläser, besonders bevorzugt Zinkphosphat-haltiges Glas, neben einer weiteren Wirkstoffzusammensetzung, umfassend ein Trägermaterial, bevorzugt ein wasserunlösliches Trägermaterial und mindestens einen Wirkstoff D enthalten. Diese können dann in einer gemeinsamen Kammer oder in getrennten Kammern, insbesondere in einer oder mehreren Kammern, welche Öffnungen aufweist, insbesondere solche Öffnungen, so dass die Spülflotte und/oder die Luft diese durchströmen können, enthalten sein. Bevorzugt sind solche Reinigungsmittelangebotsformen, die ein Zinkphosphat- oder Bismuthphosphathaltiges Glas enthalten und weiterhin mindestens eine, bevorzugt zwei, drei, oder mehrere Wirkstoffzusammensetzungen enthalten, die ein oder mehrere Duftstoffe und/oder ein oder mehrere Duftfänger als Wirkstoffe umfassen.

Im Rahmen der vorliegenden Anmeldung werden insbesondere thermoplastische Trägermaterialien bzw. Trägermaterialien, die sich unter Einwirkung der bei Gebrauch auftretenden Umgebungstemperaturen plastisch verformen, besonders bevorzugt. Durch die plastische Verformung der Trägermaterialien im Verlaufe einer oder mehrerer Anwendungen wird eine Änderung der Trägermaterialoberfläche, insbesondere eine Änderung der Größe der Trägermaterialoberfläche, erreicht, welche sich wiederum vorteilhaft auf das Freisetzungsprofil und die Freisetzungskinetik der in den Wirkstoffzusammensetzungen enthaltenen reinigungsaktiven Wirkstoffe auswirkt. Dosiervorrichtungen, dadurch gekennzeichnet, dass mindestens ein polymeres Trägermaterial einen Schmelz- oder Erweichungspunkt zwischen 40 und 125°C, vorzugsweise zwischen 60 und 100°C, besonders bevorzugt einen Schmelzpunkt von 70 bis 90°C und insbesondere zwischen 73 und 80°C aufweist (bevorzugte Bestimmungsmethode für den Schmelzpunkt gemäß ISO 11357-3), sind erfindungsgemäß bevorzugt.

Die erfindungsgemäßen Reinigungsmittelangebotsformen eignen sich insbesondere zur Mehrfachdosierung der in ihnen umfassten Wirkstoffe. Um eine solche Mehrfachdosierung über eine Vielzahl von Reinigungsverfahren zu gewährleisten, hat es sich als vorteilhaft erwiesen, ausschließlich wasserunlösliche Trägermaterialien einzusetzen. Diese wasserunlöslichen Trägermaterialien vereinfachen zudem die Herstellung erfindungsgemäßer Angebotsformen. Bevorzugte Angebotsformen sind daher dadurch gekennzeichnet, dass alle eingesetzten Trägermaterialien wasserunlöslich sind.

Die Wirkstoffzusammensetzungen können grundsätzlich alle, in Abhängigkeit von den chemischen und physikalischen Eigenschaften der Trägermaterialien realisierbaren Aggregatzustände und/oder Raumformen einnehmen. In einer weiteren Ausführungsform liegt mindestens eine der Wirkstoffzusammensetzungen als Gel vor.

In einer weiteren Ausführungsform liegt mindestens eine der Wirkstoffzusammensetzungen als Feststoff vor. Mit besonderem Vorzug werden Wirkstoffzusammensetzungen in Form einzelner, eine gesamte Wirkstoffzusammensetzungen umfassender Blöcke eingesetzt.

Bevorzugt können die Wirkstoffzusammensetzungen in partikulärer Form vorliegen, wobei die Wirkstoffzusammensetzungen, bei denen das Trägermaterial mindestens einer der Wirkstoffzusammensetzungen in Partikelform vorliegt, wobei diese Partikel vorzugsweise einen mittleren Durchmesser von 0,5 bis 20 mm, bevorzugt von 1 bis 10 mm und insbesondere von 3 bis 6 mm aufweisen, besonders bevorzugt werden.

Besonders bevorzugt werden Wirkstoffzusammensetzungen eingesetzt, die mindestens eine gefärbte Wirkstoffzusammensetzung umfassen. Durch die Einfärbung mindestens einer der Wirkstoffzusammensetzungen kann eine optische Differenzierung dieser Zusammensetzungen erreicht und der Mehrfachnutzen dieser unterschiedlichen Zusammensetzungen in einfacher Weise verdeutlicht werden. Weiterhin eignen sich die Farbstoffe aber auch als Indikator, insbesondere als Verbrauchsindikator für die eingefärbten Wirkstoffzusammensetzungen.

Bevorzugte Farbstoffe, deren Auswahl dem Fachmann keinerlei Schwierigkeit bereitet, besitzen eine hohe Lagerstabilität und Unempfindlichkeit gegenüber den übrigen Inhaltsstoffen der Mittel und gegen Licht sowie keine ausgeprägte Substantivität gegenüber den mit den farbstoffhaltigen Mitteln zu behandelnden Substraten wie beispielsweise Glas, Keramik oder Kunststoffgeschirr, um diese nicht anzufärben.

Bei der Wahl des Färbemittels muss beachtet werden, dass die Färbemittel eine hohe Lagerstabilität und Unempfindlichkeit gegenüber Licht sowie keine zu starke Affinität gegenüber Glas, Keramik oder Kunststoffgeschirr aufweisen. Gleichzeitig ist auch bei der Wahl geeigneter Färbemittel zu berücksichtigen, dass Färbemittel unterschiedliche Stabilitäten gegenüber der Oxidation aufweisen. Im allgemeinen gilt, dass wasserunlösliche Färbemittel gegen Oxidation stabiler sind als wasserlösliche Färbemittel. Abhängig von der Löslichkeit und damit auch von der Oxidationsempfindlichkeit variiert die Konzentration des Färbemittels in den Reinigungsmitteln. Bei gut wasserlöslichen Färbemitteln werden typischerweise Färbemittel-Konzentrationen im Bereich von einigen 10⁻² bis 10⁻³ Gew.-% gewählt. Bei den auf Grund ihrer Brillanz insbesondere bevorzugten, allerdings weniger gut wasserlöslichen Pigmentfarbstoffen liegt die geeignete Konzentration des Färbemittels in Reinigungsmitteln dagegen typischerweise bei einigen 10⁻³ bis 10⁻⁴ Gew.-%.

Gemäß einer bevorzugten Ausführungsform ist die Reinigungsmittelangebotsform dadurch gekennzeichnet, dass der mindestens eine Wirkstoff D ausgewählt ist aus der Gruppe der Duftstoffe, bevorzugt Linalylacetat, Dihydromyrcenol, Citronellonitrile, Menthylacetat, Methylphenylbutanol, Eucalyptol und deren Mischungen, Duftfänger, wie z.B. Zinkricinoleat, Cyclodextrine, 2-menthyl-5-cyclohexylpentanol und 1-Cyclohexylethanol, insbesondere Zinkricinoleat; Farbstoffe, Glaskorrosionsinhibitoren, antimikrobiellen Wirkstoffe, Germizide oder Fungizide sowie Mischungen davon, bevorzugt Mischungen aus mindestens einem Duftfänger, bevorzugt mit einem, zwei, drei oder mehr Duftstoffen und/oder mindestens einem Farbstoff. Weiter bevorzugt sind Mischungen aus mindestens einem Duftstoff, bevorzugt zwei, drei oder mehr Duftstoffen und mindestens einem Farbstoff.

Als Parfümöle bzw. Duftstoffe können im Rahmen der vorliegenden Erfindung einzelne Riechstoffverbindungen, z.B. die synthetischen Produkte vom Typ der Ester, Ether, Aldehyde, Ketone, Alkohole und Kohlenwasserstoffe verwendet werden. Bevorzugt werden jedoch Mischungen verschiedener Riechstoffe verwendet, die gemeinsam eine ansprechende Duftnote erzeugen. Solche Parfümöle können auch natürliche Riechstoffgemische enthalten, wie sie aus pflanzlichen Quellen zugänglich sind, z.B. Pine-, Citrus-, Jasmin-, Patchouly-, Rosen- oder Ylang-Ylang-Öl.

Um wahrnehmbar zu sein, muss ein Riechstoff flüchtig sein, wobei neben der Natur der funktionellen Gruppen und der Struktur der chemischen Verbindung auch die Molmasse eine wichtige Rolle spielt. So besitzen die meisten Riechstoffe Molmassen bis etwa 200 Dalton, während Molmassen von 300 Dalton und darüber eher eine Ausnahme darstellen. Auf Grund der unterschiedlichen Flüchtigkeit von Riechstoffen verändert sich der Geruch eines aus mehreren Riechstoffen zusammengesetzten Parfüms bzw. Duftstoffs während des Verdampfens, wobei man die Geruchseindrücke in "Kopfnote" (top note), "Herz- bzw. Mittelnote" (middle note bzw. body) sowie "Basisnote" (end note bzw. dry out) unterteilt. Da die Geruchswahrnehmung zu einem großen Teil auch auf der Geruchsintensität beruht, besteht die Kopfnote eines Parfüms bzw. Duftstoffs nicht allein aus leichtflüchtigen Verbindungen, während die Basisnote zum größten Teil aus weniger flüchtigen, d.h. haftfesten Riechstoffen besteht. Bei der Komposition von Parfüms können leichter flüchtige Riechstoffe beispielsweise an bestimmte Fixative gebunden werden, wodurch ihr zu schnelles Verdampfen verhindert wird. Bei der nachfolgenden Einteilung der Riechstoffe in "leichter flüchtige" bzw. "haftfeste" Riechstoffe ist also über den Geruchseindruck und darüber, ob der entsprechende Riechstoff als Kopf- oder Herznote wahrgenommen wird, nichts ausgesagt.

Die Duftstoffe können direkt verarbeitet werden, es kann aber auch vorteilhaft sein, die Duftstoffe auf Träger aufzubringen, die durch eine langsamere Duftfreisetzung für langanhaltenden Duft sorgen. Als solche Trägermaterialien haben sich beispielsweise Cyclodextrine bewährt, wobei die Cyclodextrin-Parfüm-Komplexe zusätzlich noch mit weiteren Hilfsstoffen beschichtet werden können.

Besonders bevorzugte Duftstoffe sind erfindungsgemäß Linalylacetat, Dihydromyrcenol, Citronellonitrile, Menthylacetat, Methylphenylbutanol und/oder Eucalyptol sowie deren Mischungen.

Als Duftfänger (oder wie im weiteren auch synonym verwendet Geruchsneutralisatoren oder Duftneutralisatoren, Agentien gegen Malodour oder Schlechtgerüche) sind beispielsweise die bekannten Ricenolate, insbesondere die Zinkricenoleate einsetzbar. Ebenfalls bevorzugt sind als Duftfänger 2-menthyl-5-cyclohexylpentanol und 1-Cyclohexylethanol. Mit besonderem Vorzug können weiterhin Aktivkohle und/oder Cyclodextrine und/oder Zeolithe, vorzugsweise sauer modifizierte Zeolithe, eingesetzt werden. Zinkricinoleat allein oder in Kombination mit einem oder mehreren der vorstehend bevorzugt genannten Duftstoffe und/oder Duftfänger, ist dabei besonders bevorzugt, da es sich auch positiv auf die Inhibierung der Glaskorrosion beim Spülprozess auswirkt.

Zur Bekämpfung von Mikroorganismen können alternativ oder zusätzlich zu den o.g. Duftstoffen und/oder Duftfängern antimikrobielle Wirkstoffe eingesetzt werden. Hierbei unterscheidet man je nach antimikrobiellem Spektrum und Wirkungsmechanismus zwischen Bakteriostatika und Bakteriziden, Fungistatika und Fungiziden usw. Wichtige Stoffe aus diesen Gruppen sind beispielsweise Benzalkoniumchloride, Alkylarlylsulfonate, Halogenphenole und Phenolmercuriacetat, wobei auch gänzlich auf diese Verbindungen verzichtet werden kann.

Die Wirkstoffe können in den Wirkstoffzubereitungen grundsätzlich in beliebigen Mengen enthalten sein. Besonders bevorzugt werden jedoch Dosiervorrichtungen, bei denen der Gewichtsanteil des/der Wirkstoffe 1 bis 70 Gew.-%, vorzugsweise 10 bis 60 Gew.-%, besonders bevorzugt 20 bis 50 Gew.-%, insbesondere 30 bis 40 Gew.-%, jeweils bezogen auf das Gesamtgewicht des/der Wirkstoffzusammensetzung(en) beträgt.

Wenn mehr als eine Wirkstoffzusammensetzung (eine Reinigungsmittelkombination) in der Reinigungsmittelangebotsform vorhanden ist, können diese getrennt voneinander oder nebeneinander in dem Verpackungsmittel der Reinigungsmittelangebotsform vorliegen. Die unterschiedlichen Wirkstoffzusammensetzungen können bevorzugt nebeneinander, das heißt in unmittelbarem Kontakt miteinander, in dem Verpackungsmittel der Reinigungsmittelangebotsform vorliegen.

Ein weiterer Gegenstand der vorliegenden Anmeldung ist eine Reinigungsmittelangebotsform, umfassend
a) eine erfindungsgemäße Reinigungsmittelzubereitung A in einer für die mindestens zweimalige, vorzugsweise mindestens viermalige und insbesondere mindestens achtmalige Durchführung eines maschinellen Geschirrspülverfahrens ausreichenden Menge;
b) mindestens eine weitere von A verschiedene Reinigungsmittelzubereitung B in einer für die mindestens zweimalige, vorzugsweise mindestens viermalige und insbesondere mindestens achtmalige Durchführung eines maschinellen Geschirrspülverfahrens ausreichenden Menge;
c) gegebenenfalls eine weitere von A und B verschiedene Reinigungsmittelzubereitung C in einer für die mindestens zweimalige, vorzugsweise mindestens viermalige und insbesondere mindestens achtmalige Durchführung eines maschinellen Geschirrspülverfahrens ausreichenden Menge;
d) gegebenenfalls mindestens eine weitere von A und B verschiedene Wirkstoffzusammensetzung in einer für die mindestens zweimalige, vorzugsweise mindestens viermalige und insbesondere mindestens achtmalige Durchführung eines maschinellen Geschirrspülverfahrens ausreichenden Menge, die mindestens ein Trägermaterial, bevorzugt ein wasserunlösliches Trägermaterial, und mindestens einen Wirkstoff D enthält;
e) eine Kartusche für die Reinigungsmittelzubereitungen A und B, bzw. A, B und C bzw. A, B, C und D, in welcher die Reinigungsmittelzubereitungen A und B, bzw. A, B, C bzw. A, B, C und D in voneinander getrennten Aufnahmekammern vorliegen.

Ein weiterer Gegenstand der vorliegenden Anmeldung ist ein Reinigungsmitteldosiersystem, umfassend
a) eine erfindungsgemäße Reinigungsmittelzubereitung A in einer für die mindestens zweimalige, vorzugsweise mindestens viermalige und insbesondere mindestens achtmalige Durchführung eines maschinellen Geschirrspülverfahrens ausreichenden Menge;
b) mindestens eine weitere von A verschiedene Reinigungsmittelzubereitung B in einer für die mindestens zweimalige, vorzugsweise mindestens viermalige und insbesondere mindestens achtmalige Durchführung eines maschinellen Geschirrspülverfahrens ausreichenden Menge;
c) gegebenenfalls eine weitere von A und B verschiedene Reinigungsmittelzubereitung C in einer für die mindestens zweimalige, vorzugsweise mindestens viermalige und insbesondere mindestens achtmalige Durchführung eines maschinellen Geschirrspülverfahrens ausreichenden Menge;
d) gegebenenfalls mindestens eine weitere von A und B verschiedene Wirkstoffzusammensetzung in einer für die mindestens zweimalige, vorzugsweise mindestens viermalige und insbesondere mindestens achtmalige Durchführung eines maschinellen Geschirrspülverfahrens ausreichenden Menge, die mindestens ein Trägermaterial, bevorzugt ein wasserunlösliches Trägermaterial, und mindestens einen Wirkstoff D enthält;
e) eine Kartusche für die Reinigungsmittelzubereitungen A, B und C bzw. A, B, C und D, in welcher die Reinigungsmittelzubereitungen A, B, C bzw. A, B, C und D in voneinander getrennten Aufnahmekammern vorliegen;
f) ein mit der Kartusche lösbar verbundenes Dosiergerät.

In einer bevorzugten Ausführungsform werden die zuvor beschriebenen Kartuschen der Reinigungsmittelangebotsformen mit einer von der Kartusche lösbaren Dosiergerät versehen. Ein solches Dosiergerät kann mit der Kartusche beispielsweise mittels einer Haft-, Rast-, Schnapp- oder Steckverbindung verbunden sein. Durch die Trennung von Kartusche und Dosiergerät wird beispielsweise die Befüllung der Kartusche vereinfacht. Alternativ ermöglich die lösbare Verbindung von Kartusche und Dosiergerät den Austausch der Kartuschen an dem Dosiergerät. Ein solcher Austausch kann beispielsweise bei einer Änderung des Reinigungsprogramms oder nach der vollständigen Leerung der Kartusche angezeigt sein.

Ein besonders bevorzugter Gegenstand dieser Anmeldung ist ein Reinigungsmitteldosiersystem, umfassend
a) eine erfindungsgemäße Reinigungsmittelangebotsform, umfassend eine für die mindestens zweimalige, vorzugsweise mindestens viermalige und insbesondere mindestens achtmalige Durchführung eines maschinellen Geschirrspülverfahrens ausreichende Menge an Reinigungsmittelzubereitungen A und B bzw. A, B und C bzw. A, B, C und D;
b) ein mit der Reinigungsmittelangebotsform lösbar verbundenes Dosiergerät.

Selbstverständlich sind auch Reinigungsmittelangebotsformen denkbar, bei denen die Kartusche und das Dosiergerät unlösbar miteinander verbunden sind.

Ein Gegenstand der vorliegenden Anmeldung ist weiterhin ein Reinigungsmitteldosiersystem, umfassend
a) eine erfindungsgemäße Reinigungsmittelangebotsform, umfassend eine für die mindestens zweimalige, vorzugsweise mindestens viermalige und insbesondere mindestens achtmalige Durchführung eines maschinellen Geschirrspülverfahrens ausreichende Menge an Reinigungsmittelzubereitungen A und B, bzw. A, B und C bzw. A, B, C und D;
b) ein mit der Reinigungsmittelangebotsform unlösbar verbundenes Dosiergerät

Die vorgenannten Reinigungsmitteldosiersysteme, umfassend die erfindungsgemäße Reinigungsmittelangebotsform (sowie optional ein oder zwei weitere, von dem erfindungsgemäßen Reinigungsmittelzubereitungen A und B bzw. A, B und C bzw. A, B, C sowie D verschiedene Zusammensetzungen, eine Kartusche und ein lösbar mit der Kartusche verbundenes Dosiergerät liegen in einer bevorzugten Ausführungsform in einer gemeinsamen Umverpackung vor, wobei die befüllte Kartusche und das Dosiergerät besonders bevorzugt getrennt voneinander in der Umverpackung enthalten sind. Die Umverpackung dient der Lagerung, dem Transport und der Präsentation der erfindungsgemäßen Reinigungsmitteangebotsform und schütz diese vor Verschmutzung, Schlag und Stoß. Insbesondere zum Zweck der Präsentation sollte die Umverpackung wenigstens anteilsweise transparent ausgestaltet sein.

Alternativ oder in Ergänzung zu einer Umverpackung besteht selbstverständlich die Möglichkeit, die erfindungsgemäße Reinigungsmittelangebotsform in Verbindung mit einer Geschirrspülmaschine zu vermarkten. Eine solche Kombination ist insbesondere in den Fällen vorteilhaft, in denen der Verlauf des maschinellen Geschirrspülverfahrens (z.B. Dauer, Temperaturverlauf, Wasserzufuhr) und die Reinigungsmittelrezeptur bzw. die Steuerelektronik des Dosiergeräts aufeinander abgestimmt sind.

Das erfindungsgemäße Dosiersystem besteht aus den Grundbauelementen einer mit dem erfindungsgemäßen Reinigungsmittel befüllten Kartusche und einem mit der Kartusche kuppelbarem Dosiergerät, welches wiederum aus weiteren Baugruppen, wie beispielsweise Bauelementträger, Aktuator, Verschlusselement, Sensor, Energiequelle und/oder Steuereinheit, gebildet ist.

Es ist bevorzugt, dass das erfindungsgemäße Dosiersystem beweglich ist. Beweglich im Sinne dieser Anmeldung bedeutet, dass das Dosiersystem nicht unlösbar mit einer wasserführenden Vorrichtung wie beispielsweise einer Geschirrspülmaschine, oder dergleichen verbunden ist, sondern beispielsweise aus einer Geschirrspülmaschine durch den Benutzer entnehmbar oder in einer Geschirrspülmaschine positionierbar, also eigenständig handhabbar, ist

Gemäß einer alternativen Ausgestaltung der Erfindung ist es auch denkbar, dass das Dosiergerät für den Benutzer nicht lösbar mit einer wasserführenden Vorrichtung wie beispielsweise einer Geschirrspülmaschine oder dergleichen verbunden ist und lediglich die Kartusche beweglich ist.

Da die zu dosierenden Zubereitungen je nach beabsichtigtem Verwendungszweck einen pH-Wert zwischen 2 und 14, insbesondere 2 und 12, aufweisen können, sollten alle Komponenten des Dosiersystems, die in Kontakt mit den Zubereitungen kommen, eine entsprechende Säure- und/oder Alkaliresistenz aufweisen. Ferner sollten diese Komponenten durch eine geeignete Materialauswahl weitestgehend chemisch inert, beispielsweise gegen nichtionische Tenside, Enzyme und/oder Duftstoffe sein.

Unter einer Kartusche im Sinne dieser Anmeldung wird ein Packmittel verstanden, das dazu geeignet ist, fließfähige oder streufähige Zubereitungen zu umhüllen oder zusammenzuhalten und das zur Abgabe der Zubereitung an ein Dosiergerät koppelbar ist. Insbesondere kann eine Kartusche auch mehrere Kammern umfassen, die mit voneinander verschiedenen Zusammensetzungen befüllbar sind. Auch ist es denkbar, dass eine Behältermehrzahl zu einer Kartuscheneinheit angeordnet wird.

Es ist vorteilhaft, dass die Kartusche wenigstens eine Auslassöffnung aufweist, die derart angeordnet ist, dass eine schwerkraftbewirkte Zubereitungsfreisetzung aus dem Behälter in der Gebrauchsstellung des Dosiergeräts bewirkt werden kann. Hierdurch werden keine weiteren Fördermittel zur Freisetzung von Zubereitung aus dem Behälter benötigt, wodurch der Aufbau des Dosiergeräts einfach und die Herstellungskosten niedrig gehalten werden können.

In einer bevorzugten Ausgestaltungsform der Erfindung ist wenigstens eine zweite Kammer zur Aufnahme wenigstens einer zweiten fließ- oder streufähigen Zubereitung vorgesehen, wobei die zweite Kammer wenigstens eine Auslassöffnung aufweist, die derart angeordnet ist, dass eine schwerkraftbewirkte Produktfreisetzung aus der zweiten Kammer in der Gebrauchsstellung des Dosiergeräts bewirkt wird. Die Anordnung einer zweiten Kammer ist insbesondere dann vorteilhaft, wenn in den voneinander getrennten Behältern Zubereitungen bevorratet sind, die üblicherweise nicht miteinander lagerstabil sind, wie beispielsweise Bleichmittel und Enzyme.

Des Weiteren ist es erfindungsmäß notwendig, dass mehr als zwei, insbesondere drei, vier oder fünf Kammern in bzw. an einer Kartusche vorgesehen sind. Insbesondere ist mindestens eine der Kammern zur Abgabe von Wirkstoff(en) D, wie etwa eines Glaskorrosionsinhibitors, eines Duftstoffs oder insbesondere eine Geruchsneutralisators an die Umgebung, so ausgestaltet, dass sie Öffnungen aufweist, die von der Spülflotte und/oder der Luft durchströmt werden können.

In einer weiteren Ausgestaltung der Erfindung ist die Kartusche einstückig ausgebildet. Hierdurch lassen sich die Kartuschen, insbesondere durch geeignete Blasformverfahren, kostengünstig in einem Herstellungsschritt ausbilden. Die Kammern einer Kartusche können hierbei beispielsweise durch Stege oder Materialbrücken voneinander separiert sein.

Die Kartusche kann auch mehrstückig durch im Spritzguss hergestellte und anschließend zusammengefügte Bauteile gebildet sein. Ferner ist es denkbar, dass die Kartusche in derart mehrstückig ausgeformt ist, dass wenigstens eine Kammer, vorzugsweise alle Kammern, einzeln aus dem Dosiergerät entnehmbar oder in das Dosiergerät einsetzbar sind. Hierdurch ist es möglich, bei einem unterschiedlich starken Verbrauch einer Zubereitung aus einer Kammer, eine bereits entleerte Kammer auszutauschen, während die übrigen, die noch mit Zubereitung befüllt sein können, in dem Dosiergerät verbleiben. Somit kann ein gezieltes und bedarfsgerechtes Nachfüllen der einzelnen Kammern bzw. deren Zubereitungen erreicht werden.

Die Kammern einer Kartusche können durch geeignete Verbindungsmethoden aneinander fixiert sein, so dass eine Behältereinheit gebildet ist. Die Kammern können durch eine geeignete formschlüssige, kraftschlüssige oder stoffschlüssige Verbindung lösbar oder unlösbar gegeneinander fixiert sein.

Insbesondere kann die Fixierung durch eine oder mehrere der Verbindungsarten aus der Gruppe der Snap-In Verbindungen, Klettverbindungen, Pressverbindungen, Schmelzverbindungen, Klebverbindungen, Schweißverbindungen, Lötverbindungen, Schraubverbindungen, Keilverbindungen, Klemmverbindungen oder Prellverbindungen erfolgen. Insbesondere kann die Fixierung auch durch einen Schrumpfschlauch (sog. Sleeve) ausgebildet sein, der in einem erwärmten Zustand über die gesamte oder Abschnitte der Kartusche gezogen wird und die Kammern bzw. die Kartusche im abgekühlten Zustand fest umschließt.

Um vorteilhafte Restentleerungseigenschaften der Kammern bereitzustellen, kann der Boden der Kammern trichterförmig zur Abgabeöffnung hin geneigt sein. Des Weiteren kann die Innenwand einer Kammer durch geeignete Materialwahl und/oder Oberflächenausgestaltung in derart ausgebildet sein, dass eine geringe Materialanhaftung der Zubereitung an der inneren Kammerwand realisiert ist. Auch durch diese Maßnahme lässt sich die Restentleerbarkeit einer Kammer weiter optimieren.

Die Kammern einer Kartusche können gleiche oder voneinander verschiedene Füllvolumina aufweisen. Bei einer Konfiguration mit zwei Kammern beträgt das Verhältnis der Behältervolumina bevorzugt 5:1, bei einer Konfiguration mit drei Kammern bevorzugt 4:1:1, wobei diese Konfigurationen insbesondere zur Verwendung in Geschirrspülmaschinen geeignet sind.

Wie oben erwähnt, besitzt die Kartusche vorzugsweise 3, 4, 5 oder 6 Kammern. Für den Einsatz einer derartigen Kartusche in einer Geschirrspülmaschine ist es insbesondere bevorzugt, dass die erste Kammer eine alkalische Reinigungszubereitung, die zweite Kammer eine enzymatische Zubereitung und die dritte Kammer einen Klarspüler beinhaltet, wobei das Volumenverhältnis der Kammern in etwa 4:1:1 beträgt. Die vierte Kammer beinhaltet dabei die mindestens eine Wirkstoffzusammensetzung, umfassend den mindestens einen Wirkstoff D und ein Trägermaterial, bevorzugt ein wasserunlösliches Trägermaterial.

In oder an einer Kammer kann eine Dosierkammer, in Fließrichtung der Zubereitung vor der Auslassöffnung ausgebildet sein. Durch die Dosierkammer wird die Zubereitungsmenge, die bei der Freisetzung von Zubereitung aus der Kammer an die Umgebung abgegeben werden soll, festgelegt. Dies ist insbesondere dann vorteilhaft, wenn das Verschlusselement des Dosiergeräts, das die Zubereitungsabgabe aus einer Kammer an die Umgebung bewirkt, nur in einen Abgabe- und einen Verschlusszustand ohne Kontrolle der Abgabemenge versetzt werden kann. Durch die Dosierkammer wird dann gewährleistet, dass ohne eine unmittelbare Rückkopplung der abgegebenen Zubereitungsmenge eine vordefinierte Menge an Zubereitung freigesetzt wird. Die Dosierkammern können einstückig oder mehrstückig ausgeformt sein.

Gemäß einer weiteren vorteilhaften Weiterentwicklung der Erfindung weist eine oder weisen mehrere Kammern neben einer Auslassöffnung jeweils eine flüssigkeitsdicht verschließbare Kammeröffnung auf. Durch diese Kammeröffnung ist es beispielsweise ermöglicht, in dieser Kammer aufbewahrte Zubereitung nachzufüllen.

Zur Belüftung der Kartuschenkammern können insbesondere im Kopfbereich der Kartusche Belüftungsmöglichkeiten vorgesehen sein, um einen Druckausgleich bei fallendem Befüllstand der Kammern zwischen dem Inneren der Kartuschenkammern und der Umgebung zu gewährleisten. Diese Belüftungsmöglichkeiten können beispielsweise als Ventil, insbesondere Silikonventil, Micro-Öffnungen in der Kartuschenwand oder dergleichen ausgebildet sein.

Sollte gemäß einer weiteren Ausgestaltung nicht die Kartuschenkammern direkt belüftet werden, sondern über das Dosiergerät oder keine Belüftung, z.B. bei der Verwendung flexibler Behältnisse, wie beispielsweise Beutel, vorgesehen sein, so hat dies den Vorteil, dass bei erhöhten Temperaturen im Laufe eines Spülzyklus eines Geschirrspülers durch die Erwärmung des Kammerinhalts ein Druck aufgebaut wird, der die zu dosierenden Zubereitungen in Richtung der Auslassöffnungen drückt, so dass hierdurch eine gute Restentleerbarkeit der Kartusche erreichbar ist. Ferner besteht bei einer derartigen, luftfreien Verpackung nicht die Gefahr einer Oxidation von Substanzen der Zubereitung, was eine Beutelverpackung oder auch Bag-In-Bottle-Verpackung insbesondere für oxidationsempfindliche Zubereitungen zweckmäßig erscheinen lässt.

Die Kartusche weist üblicherweise ein Füllvolumen von <5.000 ml, insbesondere <1.000 ml, bevorzugt <500ml, besonders bevorzugt <250 ml, ganz besonders bevorzugt < 50 ml auf.

Die Kartusche kann jede beliebige Raumform annehmen. Sie kann beispielsweise würfelartig, kugelförmig oder plattenartig ausgebildet sein.

Die Kartusche und das Dosiergerät können insbesondere derart bezüglich ihrer Raumform ausgestaltet sein, dass sie einen möglichst geringen Nutzvolumenverlust insbesondere in einer Geschirrspülmaschine gewährleisten.

Zur Verwendung des Dosiergeräts in Geschirrspülmaschinen ist es besonders vorteilhaft, das Gerät in Anlehnung an in Geschirrspülmaschinen zu reinigendem Geschirr auszuformen. So kann dieses beispielsweise plattenförmig, in etwa in den Abmessungen eines Tellers, ausgebildet sein. Hierdurch kann das Dosiergerät platzsparend z.B. im Unterkorb des Geschirrspülers positioniert werden. Ferner erschließt sich die richtige Positionierung der Dosiereinheit dem Benutzer unmittelbar intuitiv durch die tellerartige Formgebung. Bevorzugt weist die Kartusche ein Verhältnis von Höhe:Breite:Tiefe zwischen 5:5:1 und 50:50:1, insbesondere bevorzugt von etwa 10:10:1 auf. Durch die "schlanke" Ausbildung des Dosiergeräts und der Kartusche ist es insbesondere möglich, das Gerät in dem unteren Besteckkorb einer Geschirrspülmaschine in den für Teller vorgesehenen Aufnahmen zu positionieren. Dies hat den Vorteil, dass die aus dem Dosiergerät abgegeben Zubereitungen direkt in die Spülflotte gelangen und nicht an anderem Spülgut anhaften können.

Üblicherweise sind handelsübliche Haushaltsgeschirrspülmaschinen in derart konzipiert, dass die Anordnung von größerem Spülgut, wie etwa Pfannen oder große Teller, im unteren Korb der Geschirrspülmaschine vorgesehen ist. Um eine nicht optimale Positionierung des Dosiersystems durch den Benutzer im oberen Korb zu vermeiden, ist in einer vorteilhaften Ausgestaltung der Erfindung das Dosiersystem derart dimensioniert, dass eine Positionierung des Dosiersystems lediglich in den dafür vorgesehenen Aufnahmen des unteren Korbes ermöglicht ist. Hierzu können die Breite und die Höhe des Dosiersystems insbesondere zwischen 150mm und 300mm, besonders bevorzugt zwischen 175mm und 250mm gewählt sein.

Es ist jedoch auch denkbar, die Dosiereinheit in Becherform mit einer im Wesentlichen kreisrunden oder quadratischen Grundfläche auszubilden.

Um hitzeempfindliche Bestandteile einer in einer Kartusche befindlichen Zubereitung vor Wärmeeinwirkung zu schützen, ist es von Vorteil, die Kartusche aus einem Material mit einer geringen Wärmeleitfähigkeit herzustellen.

Eine weitere Möglichkeit zur Verminderung des Hitzeeinflusses auf eine Zubereitung in einer Kammer der Kartusche ist es, die Kammer durch geeignete Maßnahmen zu isolieren z.B. durch die Verwendung von Wärmedämmmaterialien wie etwa Styropor, die die Kammer oder die Kartusche in geeigneter Weise ganz oder teilweise umschließen.

In einer bevorzugten Ausführungsform der Erfindung, weist die Kartusche ein RFID-Etikett auf, dass zumindest Informationen über den Inhalt der Kartusche beinhaltet und das durch die Sensoreinheit auslesbar ist.

Diese Informationen können verwendet werden, um ein in der Steuereinheit gespeichertes Dosierprogramm auszuwählen. Hierdurch kann sichergestellt werden, dass stets ein für eine bestimmte Zubereitung optimales Dosierprogramm verwendet wird. Es kann auch vorgesehen sein, dass bei nicht Vorhandensein eines RFID-Labels oder bei einem RFID-Label mit einer falschen oder fehlerhaften Kennung, keine Dosierung durch das Dosiergerät erfolgt und stattdessen ein optisches oder akustisches Signal erzeugt wird, dass den Benutzer auf den vorliegenden Fehler hinweist.

Um einen Fehlgebrauch der Kartusche auszuschließen, können die Kartuschen auch strukturelle Elemente aufweisen, die mit korrespondierenden Elementen des Dosiergeräts nach dem Schlüssel-Schloss-Prinzip zusammenwirken, so dass beispielsweise nur Kartuschen eines bestimmten Typs an das Dosiergerät koppelbar sind. Ferner ist es durch diese Ausgestaltung möglich, dass Informationen über die an das Dosiergerät gekoppelten Kartusche an die Steuereinheit übertragen werden, wodurch eine auf den Inhalt des dementsprechenden Behälters abgestimmte Steuerung des Dosiergeräts erfolgen kann.

Die Kartusche ist insbesondere zur Aufnahme von fließfähigen Reinigungsmittel ausgebildet. Besonders bevorzugt weist eine derartige Kartusche eine Mehrzahl von Kammern zur räumlich separierten Aufnahme jeweils voneinander verschiedener Zubereitungen eines Reinigungsmittels auf. Die Kartusche kann so ausgebildet sein, dass sie lösbar oder fest in oder an der Geschirrspülmaschine angeordnet werden kann.

In dem Dosiergerät sind die zum Betrieb notwendige Steuereinheit, Sensoreinheit sowie wenigstens ein Aktuator integriert. Bevorzugt ist ebenfalls eine Energiequelle in dem Dosiergerät angeordnet.

Vorzugsweise besteht das Dosiergerät aus einem spritzwassergeschütztem Gehäuse, dass das Eindringen von Spritzwasser, wie es beispielsweise bei der Verwendung in einer Geschirrspülmaschine auftreten kann, in das Innere des Dosiergeräts verhindert.

Es ist besonders bevorzugt, dass das Dosiergerät wenigstens eine erste Schnittstelle umfasst, welche mit einer in oder an einem wasserführenden Gerät, insbesondere einem wasserführenden Haushaltsgerät, bevorzugt einer Geschirrspülmaschine ausgebildeten korrespondierenden Schnittstelle derart zusammenwirkt, dass eine Übertragung von elektrischer Energie von dem wasserführenden Gerät zum Dosiergerät verwirklicht wird.

In einer Ausgestaltung der Erfindung sind die Schnittstellen durch Steckverbinder ausgebildet. In einer weiteren Ausgestaltung können die Schnittstellen derart ausgebildet sein, dass eine drahtlose Übertragung von elektrischer Energie bewirkt wird.

In einer vorteilhaften Weiterentwicklung der Erfindung ist jeweils eine zweite Schnittstelle am Dosiergerät und dem wasserführenden Gerät, wie etwa einer Geschirrspülmaschine, zur Übertragung von elektromagnetischen Signalen, welche insbesondere Betriebszustands-, Mess- und/oder Steuerinformationen des Dosiergeräts und/oder des wasserführenden Geräts wie einer Geschirrspülmaschine repräsentieren, ausgebildet.

Durch einen Adapter kann eine einfache Kopplung des Dosiersystems mit einem wasserführenden Haushaltsgerät realisiert werden. Der Adapter dient der mechanischen und/oder elektrischen Verbindung des Dosiersystems mit dem wasserführenden Haushaltsgerät.

Der Adapter ist, bevorzugt fest, mit einer wasserführenden Leitung des Haushaltsgeräts verbunden. Es ist jedoch auch denkbar, den Adapter für eine Positionierung im oder am Haushaltsgerät vorzusehen, in der der Adapter vom Wasserfluss und/oder Sprühstrahl des Haushaltsgeräts erfasst ist.

Durch den Adapter wird es möglich, ein Dosiersystem sowohl für eine autarke als auch "build-in" Version auszuführen. Auch ist es möglich, den Adapter als eine Art Aufladestation für das Dosiersystem auszubilden, in der beispielsweise die Energiequelle des Dosiergeräts aufgeladen wird oder Daten zwischen dem Dosiergerät und dem Adapter ausgetauscht werden.

Der Adapter kann in einer Geschirrspülmaschine an einer der inneren Wände der Spülkammer, insbesondere an der inneren Seite der Geschirrspülmaschinentür, angeordnet sein. Es ist jedoch auch denkbar, dass der Adapter als solches nicht zugänglich für den Benutzer im wasserführenden Haushaltsgerät positioniert ist, so dass das Dosiergerät beispielsweise während der Montage des Haushaltsgeräts in den Adapter eingesetzt wird, wobei der Adapter, das Dosiergerät und das Haushaltsgerät derart ausgebildet sind, dass eine Kartusche vom Benutzer mit dem Dosiergerät gekoppelt werden kann.

Die erfindungsgemäßen Reinigungsmittelangebotsformen eignen sich für den Einsatz in der Geschirrreinigung, gleichwohl ist die Verwendung einer erfindungsgemäßen Reinigungsmittelangebotsform oder eines Reinigungsmitteldosiersystems zur Geschirreinigung in einem maschinellen Geschirrspülverfahren bevorzugt.

Wie eingangs ausgeführt, zeichnen sich die erfindungsgemäßen Reinigungsmittel durch eine besondere physikalische und chemische Stabilität, insbesondere gegenüber Temperaturschwankungen, aus. Die erfindungsgemäßen Reinigungsmittel eignen sich damit ausnehmend für die Dosierung mittels eines im Innenraum einer Geschirrspülmaschine befindlichen Dosiersystems. Ein derartiges Dosiersystem, das unbeweglich in den Innenraum der Geschirrspülmaschine integriert sein kann (Maschinen-integriertes Dosiergerät), aber selbstverständlich auch als bewegliche Vorrichtung in den Innenraum eingebracht werden kann (autarkes Dosiergerät), enthält die mehrfache zur Durchführung eines maschinellen Reinigungsverfahrens benötigte Menge des Reinigungsmittels.

Beweglich im Sinne dieser Anmeldung bedeutet, dass das Abgabe- und Dosiersystem nicht unlösbar mit einer Vorrichtung wie beispielsweise einer Geschirrspülmaschine oder dergleichen verbunden ist, sondern beispielsweise aus einer Geschirrspülmaschine entnehmbar oder in einer Geschirrspülmaschine positionierbar ist.

Die Verwendung einer erfindungsgemäßen Reinigungsmittelangebotsform zur Befüllung
i) einer unbeweglich in den Innenraum einer Geschirrspülmaschine integrierten Kartusche eines Dosiersystems oder
ii) einer für die Positionierung im Innenraum einer Geschirrspülmaschine vorgesehenen beweglichen Kartusche eines Dosiersystems
mit einer für die mindestens zweimalige, vorzugsweise mindestens viermalige und insbesondere mindestens achtmalige Durchführung eines maschinellen Geschirrspülverfahrens ausreichenden Menge dieser Reinigungsmittelangebotsform sind ebenfalls Gegenstände dieser Anmeldung.

Ein Beispiel für eine unbeweglich Kartusche ist ein unbeweglich in den Innenraum, beispielsweise in die Seitenwand oder die Innenverkleidung der Tür einer Geschirrspülmaschine integrierter Behälter. Ein Beispiel für eine bewegliche Kartusche ist ein Behälter, der vom Verbraucher in den Innenraum der Geschirrspülmaschine eingebracht wird und dort während des gesamten Verlaufs eines Reinigungsgangs verbleibt. Eine solche Kartusche ist, beispielsweise durch einfaches Einstellen in den Besteck- oder Geschirrkorb, in den Innenraum integrierbar kann jedoch vom Verbraucher auch wieder aus dem Innenraum der Geschirrspülmaschine entnommen werden.

Die Dosierung des Reinigungsmittels bzw. der Reinigungsmittelkombination aus der Kartusche in den Innenraum der Geschirrspülmaschine erfolgt wie weiter oben beschrieben vorzugsweise mittels eines von der Kartusche lösbaren Dosiergeräts. Ein solches Dosiergerät kann mit der Kartusche mittels einer Haft-, Rast-, Schnapp- oder Steckverbindung verbunden sein. Kartuschen mit unlösbar verbundenem Dosiergerät sind jedoch selbstverständlich auch einsetzbar.

Die Verwendung einer erfindungsgemäßen Reinigungsmittelangebotsform als Reinigungsmittelreservoir für i) ein unbeweglich in den Innenraum einer Geschirrspülmaschine integriertes Dosiergerät oder ii) ein für die Positionierung im Innenraum einer Geschirrspülmaschine vorgesehenes bewegliches Dosiergerät ist bevorzugt.

Die Verwendung eines erfindungsgemäßen Reinigungsmitteldosiersystems als Reinigungsmittelreservoir für eine Geschirrspülmaschine ist ein weiterer Gegenstand der vorliegenden Anmeldung.

Zwei weitere Gegenstände dieser Anmeldung sind die Verwendung einer erfindungsgemäßen Reinigungsmittelangebotsform, umfassend
a) eine erfindungsgemäße Reinigungsmittelzubereitung A in einer für die mindestens zweimalige, vorzugsweise mindestens viermalige und insbesondere mindestens achtmalige Durchführung eines maschinellen Geschirrspülverfahrens ausreichenden Menge;
b) mindestens eine weitere von A verschiedene Reinigungsmittelzubereitung B in einer für die mindestens zweimalige, vorzugsweise mindestens viermalige und insbesondere mindestens achtmalige Durchführung eines maschinellen Geschirrspülverfahrens ausreichenden Menge;
c) gegebenenfalls eine weitere von A und B verschiedene Reinigungsmittelzubereitung C in einer für die mindestens zweimalige, vorzugsweise mindestens viermalige und insbesondere mindestens achtmalige Durchführung eines maschinellen Geschirrspülverfahrens ausreichenden Menge;
d) gegebenenfalls mindestens eine weitere von A und B verschiedene Wirkstoffzusammensetzung in einer für die mindestens zweimalige, vorzugsweise mindestens viermalige und insbesondere mindestens achtmalige Durchführung eines maschinellen Geschirrspülverfahrens ausreichenden Menge, die mindestens ein Trägermaterial, bevorzugt wasserunlösliches Trägermaterial, und mindestens einen Wirkstoff D enthält;
e) eine Kartusche für die Reinigungsmittelzubereitungen A und B, bzw. A, B und C bzw. A, B, C und D, in welcher die Reinigungsmittelzubereitungen A, B, C bzw. A, B, C und D in voneinander getrennten Aufnahmekammern vorliegen als Reinigungsmittelreservoir für
   i) ein unbeweglich in den Innenraum einer Geschirrspülmaschine integriertes Dosiergerät oder
   ii) ein für die Positionierung im Innenraum einer Geschirrspülmaschine vorgesehenes bewegliches Dosiergerät.

Die erfindungsgemäßen Reinigungsmittel und Reinigungsmittelkombinationen werden, wie zuvor ausgeführt, vorzugsweise als maschinelle Geschirrspülmittel eingesetzt.

Maschinelles Geschirrspülverfahren unter Einsatz einer Reinigungsmittelangebotsform oder eines Reinigungsmitteldosiersystems nach einem der vorherigen Ansprüche, in dessen Verlauf aus einer im Innenraum der Geschirrspülmaschine befindlichen Kartusche
- eine Teilmenge a der in der Kartusche befindlichen Reinigungsmittelzubereitung A in den Innenraum der Geschirrspülmaschine dosiert wird, wobei eine Restmenge der in der Kartusche befindlichen Reinigungsmittelzubereitung bis zum Ende des Geschirrspülverfahrens in der Kartusche verbleibt, dadurch gekennzeichnet, dass diese Restmenge mindestens der doppelten, vorzugsweise mindestens der vierfachen und insbesondere mindestens der achtfachen Menge der Teilmenge a entspricht; und
- eine Teilmenge b der in der Kartusche befindlichen Reinigungsmittelzubereitung B in den Innenraum der Geschirrspülmaschine dosiert wird, wobei eine Restmenge der in der Kartusche befindlichen Reinigungsmittelzubereitung bis zum Ende des Geschirrspülverfahrens in der Kartusche verbleibt, dadurch gekennzeichnet, dass diese Restmenge mindestens der doppelten, vorzugsweise mindestens der vierfachen und insbesondere mindestens der achtfachen Menge der Teilmenge b entspricht; und
- gegebenenfalls eine Teilmenge c der in der Kartusche gegebenenfalls befindlichen Reinigungsmittelzubereitung C in den Innenraum der Geschirrspülmaschine dosiert wird, wobei eine Restmenge der in der Kartusche befindlichen Reinigungsmittelzubereitung bis zum Ende des Geschirrspülverfahrens in der Kartusche verbleibt, dadurch gekennzeichnet, dass diese Restmenge mindestens der doppelten, vorzugsweise mindestens der vierfachen und insbesondere mindestens der achtfachen Menge der Teilmenge c entspricht.
- gegebenenfalls eine Teilmenge d der in der Kartusche gegebenenfalls befindlichen Wirkstoffe D in den Innenraum der Geschirrspülmaschine dosiert wird, wobei eine Restmenge des in der Kartusche befindlichen Wirkstoffes bis zum Ende des Geschirrspülverfahrens in der Kartusche verbleibt, dadurch gekennzeichnet, dass diese Restmenge mindestens der doppelten, vorzugsweise mindestens der vierfachen und insbesondere mindestens der achtfachen Menge der Teilmenge d entspricht und

Gemäß einer bevorzugten Ausführungsform betrifft die Erfindung auch ein maschinelles Geschirrspülverfahren, bei dem die Wirkstoffzusammensetzung sich in der Kartusche befindet und durch Öffnungen von der Spülflotte und/oder der Luft durchströmt wird. Bei der Durchströmung mit Luft erfolgt insbesondere die Abgabe von Duftstoff(en) an die Luft im Innenraum der Spülmaschine und führt so neben einer Beduftung der Spülflotte während des Geschirrspülverfahrens auch zwischen den einzelnen Spülgängen zu einer für den Verbraucher angenehmen Geruchssituation beim Öffnen und Beladen der Spülmaschine.

In den erfindungsgemäßen Geschirrspülverfahren können selbstverständlich nicht nur die erfindungsgemäßen Reinigungsmittelangebotsformen, sondern auch die erfindungsgemäßen Reinigungsmitteldosiersysteme eingesetzt werden.

In einer bevorzugten Ausführungsform erfolgt die Dosierung der Reinigungsmittelzubereitung A und der Reinigungsmittelzubereitung B sowie gegebenenfalls der Reinigungsmittelzubereitung C zu unterschiedlichen Zeiten der Reinigungsgangs.

Ein weiterer bevorzugter Gegenstand dieser Anmeldung ist daher ein maschinelles Geschirrspülverfahren unter Einsatz einer erfindungsgemäßen Reinigungsmittelangebotsform oder eines erfindungsgemäßen Reinigungsmitteldosiersystems, in dessen Verlauf
a) zu einem Zeitpunkt t1 aus einer im Innenraum der Geschirrspülmaschine befindlichen Kartusche eine Teilmenge a der in der Kartusche befindlichen erfindungsgemäßen Reinigungsmittelzubereitung A in den Innenraum der Geschirrspülmaschine dosiert wird, wobei eine Restmenge des in der Kartusche befindlichen Reinigungsmittels bis zum Ende des Geschirrspülverfahrens in der Kartusche verbleibt, welche der mindestens doppelten, vorzugsweise der mindestens vierfachen und insbesondere mindestens der achtfachen Menge der Teilmenge a entspricht;
b) zu mindestens einem weiteren Zeitpunkt t2 ≠ t1 aus einer im Innenraum der Geschirrspülmaschine befindlichen Kartusche eine Teilmenge b der in der zweiten Kartusche befindlichen von der erfindungsgemäßen Reinigungsmittelzubereitung A unterschiedlichen Reinigungsmittelzubereitung B in den Innenraum der Geschirrspülmaschine dosiert wird, wobei eine Restmenge des in dieser Kartusche befindlichen Reinigungsmittels bis zum Ende des Geschirrspülverfahrens in der Kartusche verbleibt, welche mindestens der doppelten, vorzugsweise mindestens der vierfachen und insbesondere mindestens der achtfachen Menge der Teilmenge b entspricht;
c) gegebenenfalls zu mindestens einem weiteren Zeitpunkt t3 ≠ t2 ≠ t1 aus einer im Innenraum der Geschirrspülmaschine befindlichen Kartusche eine Teilmenge d der in einer weiteren Kartusche befindlichen von den erfindungsgemäßen Reinigungsmittelzubereitungen A und B unterschiedlichen Reinigungsmittelzubereitung C in den Innenraum der Geschirrspülmaschine dosiert wird, wobei eine Restmenge des in dieser Kartusche befindlichen Reinigungsmittels bis zum Ende des Geschirrspülverfahrens in der Kartusche verbleibt, welche mindestens der doppelten, vorzugsweise mindestens der vierfachen und insbesondere mindestens der achtfachen Menge der Teilmenge d entspricht.

In bevorzugten Ausführungsformen der zuvor beschriebenen maschinellen Geschirrspülverfahren mit zeitversetzter Dosierung der Reinigungsmittelzubereitungen A und B bzw. A, B und C liegt der Zeitpunkt t2 zeitlich mindestens 1 Minute, vorzugsweise mindestens 2 Minuten und insbesondere zwischen 3 und 30 Minuten, insbesondere zwischen 3 und 20 Minuten, vor oder nach, vorzugsweise vor dem Zeitpunkt t1. In bevorzugten Ausführungsformen der zuvor beschriebenen maschinellen Geschirrspülverfahren mit zeitversetzter Dosierung der Minute, vorzugsweise mindestens 2 Minuten und insbesondere zwischen 3 und 30 Minuten, insbesondere zwischen 3 und 20 Minuten, vor oder nach, vorzugsweise nach dem Zeitpunkt t1.

In einer bevorzugten Ausführungsform wird die Reinigungszubereitung B bei einer Temperatur von 20-35°C, anschließend die Reinigungszubereitung A bei einer Temperatur von 30-60°C und danach die Reinigungszubereitung C bei einer Temperatur unter 20°C in den Innenraum zudosiert.

### Ausführungsbeispiel:

### Reinigungsleistung:

Es wurden die Zubereitungen A und B in einem Verhältnis von 4:1 automatisch im Hauptspülgang in die Spülflotte dosiert. Dabei enthielt die Reinigungszubereitung B zusätzlich jeweils 30 mg/job Aktivprotein zwei unterschiedlicher Proteasen wie weiter unten beschrieben.

Die Reinigungsleistung der Reinigungsmittelkombination wurde nach der IKW-Methode in einer Miele GSL, Programm Eco 45°, bei 21 °dH, bestimmt.

**Tabelle 2:**

| | Rezeptur 4 |
|---|---|
| Inhaltsstoffe Zubereitung A | [Gew.-%] |
| Natriumcitrat | 20 |
| Natriumcarbonat | 15 |
| Reinigungsaktives Polymer | 10 |
| Misc (inkl. Wasser, Farbstoff) | ad 100 |

| Inhaltsstoffe W.-u.R.-Zubereitung B | [Gew.-%] |
|---|---|
| Amylasezubereitung (tq) | 10 |
| Tenside | 15 |
| Lösungsvermittler | 20 |
| Komplexbildner | 0 |
| Organisches Lösungsmittel | 35 |
| Misc (kein Wasser) | ad 100 |

**Tabelle 3: Reinigungsleistung:**

| | Reinigungsleistung | Eingebranntes Hackfleisch | Eigelb |
|---|---|---|---|
| E1 | Reinigungszubereitung A + Reinigungszubereitung B mit Protease 1 (30 mg/job) | 3,2 | 3,9 |
| E2 | Reinigungszubereitung A + Reinigungszubereitung B mit Protease 2 (30 mg/job) | 7,2 | 5,4 |

Reinigungszubereitung B (E1) enthält 30 mg Aktivprotein/job (entsprechend Gehalt an aktivem Enzymprotein/geeignet für eine Reinigungsanwendung) Protease 1, welche die Protease gemäß Seq ID No.2 der WO2013/060621 enthält. Reinigungszubereitung B (E2) enthält entsprechend die gleiche Menge Aktivprotein/job Protease 2, welche die Protease gemäß Seq ID No. 5 der WO2017/215925 enthält.

Dabei wurde überraschend festgestellt, dass beide Proteasen gute Ergebnisse auf proteasesensitiven Anschmutzungen lieferten, insbesondere bei Einsatz von Protease 2 ist eine besonders gute Reinigungsleistung insbesondere auf Eigelb und eingebranntes Hackfleisch zu beobachten.

Überraschenderweise wurde außerdem gefunden, dass die Reinigungsleistung der Proteasen, insbesondere auch der Protease 2, auch wenn die Reinigungsmittelzubereitung mehreren Reinigungszyklen in der Maschine durchlaufen hat, noch ausreichend erhalten ist.

## Patentansprüche

1. Flüssige Reinigungsmittelzubereitung (20°C), bevorzugt Geschirrspülmittelzubereitung, insbesondere maschinelles Geschirrspülmittelzubereitung, bevorzugt phosphatfreie maschinelle Geschirrspülmittelzubereitung, welche 30 Gew.-% und weniger, vorzugsweise 25 Gew.-% und weniger, insbesondere 15 Gew.-% und weniger Wasser, und mindestens eine Protease umfasst, die bevorzugt eine von Subtilisinen abgeleitete Protease ist, und/oder die insbesondere ausgewählt ist aus der Gruppe von Proteasen gemäß (i) bis (v), wobei die mindestens eine Protease
(i) eine Aminosäuresequenz umfasst, die mindestens 70% Sequenzidentität mit der in SEQ ID NO:1 angegebenen Aminosäuresequenz über deren Gesamtlänge aufweist und die eine Aminosäuresubstitution an mindestens einer der Positionen, die den Positionen 12, 43, 122, 127, 154, 156, 160, 211, 212 oder 222, jeweils bezogen auf die Nummerierung gemäß SEQ ID NO:1, entsprechen, aufweist, insbesondere wobei die mindestens eine Aminosäuresubstitution aus der Gruppe bestehend aus Q12L, I43V, M122L, D127P, N154S, T156A, G160S, M211N, M211L, P212D, P212H oder A222S, jeweils bezogen auf die Nummerierung gemäß SEQ ID NO:1, ausgewählt ist, und/oder insbesondere bevorzugt eine der folgenden Aminosäuresubstitutionsvarianten, jeweils bezogen auf die Nummerierung gemäß SEQ ID NO:1, aufweist:
a. I43V;
b. M122L, N154S und T156A;
c. M211N und P212D;
d. M211L und P212D;
e. G160S;
f. D127P, M211L und P212D;
g. P212H; oder
h. Q12L, M122L und A222S;
(ii) eine Aminosäuresequenz umfasst, die ein Subtilisin 309 aus *Bacillus lentus* umfasst, insbesondere eine, die eine Aminosäuresequenz umfasst, die zu der in SEQ ID NO:2 angegebenen Aminosäuresequenz über deren Gesamtlänge zu mindestens 80%, bevorzugt zu mindestens 90 %, insbesondere zu 100 % identisch ist und mindestens eine Aminosäuresubstitution an einer der Positionen 9, 15, 66, 212 und 239 in der Zählung gemäß SEQ ID NO:2 aufweist, vorzugsweise ausgewählt aus der Gruppe bestehend aus S9R, A15T, V66A, N212D und Q239R;
iii) eine Aminosäuresequenz umfasst, die ein Subtilisin 309 aus *Bacillus lentus* umfasst, insbesondere eine, die eine Aminosäuresequenz umfasst, die zu der in SEQ ID NO:2 angegebenen Aminosäuresequenz über deren Gesamtlänge zu mindestens 80% bevorzugt zu mindestens 90 %, insbesondere zu 100 % identisch ist und eine Aminosäuresubstitution an der Position 97 und eine Insertion von einer Aminosäure zwischen den Aminosäuren an Positionen 97 und 98 in der Zählung gemäß SEQ ID NO:2 aufweist, vorzugsweise ausgewählt aus S97A und/oder S97AD;
iv) eine Aminosäuresequenz umfasst, die eine zu der in SEQ ID NO:3 angegebenen Aminosäuresequenz über deren Gesamtlänge zu mindestens 80%, bevorzugt zu mindestens 90 %, insbesondere zu 100 % identisch ist und die optional mindestens eine Aminosäuresubstitution an einer der folgenden Positionen 32, 33, 48-54, 58-62, 94-107, 116, 123-133, 150, 152-156, 158-161, 164, 169, 175-186, 197, 198, 203-216 in der Zählung gemäß SEQ ID NO:3 aufweist, insbesondere mindestens eine Aminosäuresubstitution an einer, zwei, drei oder vier der folgenden Positionen 116, 126, 127, 128 und 160 in der Zählung gemäß SEQ ID NO:3, vorzugsweise mindestens eine, noch bevorzugter mehrere, am bevorzugtesten jede der Aminosäuresubstitution G116V, S126L, P127Q und/oder S128A; oder
v) eine Aminosäuresequenz umfasst, die eine alkalische Protease aus Bacillus lentus DSM 5483 umfasst, insbesondere eine, die eine Aminosäuresequenz aufweist, die zu der in SEQ ID NO:4 angegebenen Aminosäuresequenz über deren Gesamtlänge zu mindestens 80%, bevorzugt zu mindestens 90 %, insbesondere zu 100 % identisch ist und die optional mindestens eine Aminosäuresubstitution an einer, zwei, drei oder vier der folgenden Positionen 3, 4, 99 und 199 in der Zählung gemäß SEQ ID NO:4 aufweist, insbesondere die Aminosäuresubstitution R99E oder R99D, sowie optional zusätzlich mindestens eine oder zwei, vorzugsweise alle drei der Aminosäuresubstitutionen S3T, V4I und V199I.

2. Reinigungsmittel nach Anspruch 1, **dadurch gekennzeichnet, dass** der Gewichtsanteil aller Proteasen, bezogen auf den Gehalt an aktivem Enzymprotein, am Gesamtgewicht der Reinigungsmittelzubereitung, 0,0005 bis 10,0 Gew.-%, bevorzugt 0,001 bis 7,0 Gew.-%, weiter bevorzugt 0,01 bis 4,0 Gew.-% und noch weiter bevorzugt 0,1 bis 2,0 Gew.-% beträgt.

3. Reinigungsmittel nach Anspruch 1 oder 2, **dadurch gekennzeichnet, dass** die Menge aller Proteasen, bezogen auf die Menge an aktivem Enzymprotein, welche pro Spülgang hinzudosiert werden, bevorzugt 0,001 bis 1000 mg/job, weiter bevorzugt 0,1 bis 600 mg/job und noch weiter bevorzugt 1,0 bis 400 mg/job beträgt.

4. Reinigungsmittel nach einem der vorstehenden Ansprüche, **dadurch gekennzeichnet, dass** das Reinigungsmittel zusätzlich mindestens eine Amylase enthält, bevorzugt ausgewählt aus der Gruppe bestehend aus:
a) Amylase, die eine Variante einer Ausgangsamylase ist, wobei die Ausgangsamylase eine Sequenzidentität von mindestens 60 % zu SEQ ID N0:5 aufweist, und wobei die Amylasevariante zusätzlich mindestens eine Substitution an einer oder mehreren der folgenden Positionen gegenüber SEQ ID NO:5: 9, 26, 30, 33, 82, 37, 106, 118, 128, 133, 149, 150, 160, 178, 182, 186, 193, 195, 202, 203, 214, 231, 256, 257, 258, 269, 270, 272, 283, 295, 296, 298, 299, 303, 304, 305, 311, 314, 315, 318, 319, 320, 323, 339, 345, 361, 378, 383, 419, 421, 437, 441, 444, 445, 446, 447, 450, 458, 461, 471, 482, 484, umfasst und die bevorzugt auch Deletionen von D183* und G184* aufweist;
b) Amylase, die eine Variante einer Ausgangsamylase ist, wobei die Ausgangsamylase eine Sequenzidentität von mindestens 90% zu SEQ ID NO: 6 aufweist, und wobei die Amylasevariante Deletionen in den Positionen 183 und 184 und/oder mindestens eine Substitution an einer oder mehreren der folgenden Positionen gegenüber SEQ ID N06: 93, 116, 118, 129, 133, 134, 140, 142, 146, 147, 149, 151, 152, 169, 174, 186, 189, 193, 195, 197, 198, 200, 203, 206, 210, 212, 213, 235, 243, 244, 260, 262, 284, 303, 304, 320, 338, 347, 359, 418, 431, 434, 439, 447, 458, 469, 476 und 477 umfasst;
c) Amylase, die eine Variante einer Ausgangsamylase ist, wobei die Ausgangsamylase eine Sequenzidentität von mindestens 95% zu SEQ ID N0:7 aufweist, und weiterhin eine oder mehrere der folgenden Mutationen M202, M208, S255, R172 und/oder M261 umfasst; und
d) Mischungen davon.

5. Reinigungsmittel nach einem der vorherigen Ansprüche, **dadurch gekennzeichnet, dass** das Reinigungsmittel, bezogen auf das Gesamtgewicht des flüssigen enzymhaltigen Mittels, organisches Lösungsmittel, vorzugsweise ausgewählt aus Glycerin, 1,2-Propylenglycol, 1,3-Propylenglycol, Dipropylenglycol sowie Polyethylenglycolen, insbesondere 1,2-Propylenglycol enthält, wobei der Gewichtsanteil des 1,2-Propylenglycols, bezogen auf das Gesamtgewicht der flüssigen Reinigungsmittelzubereitung, vorzugsweise 5 bis 80 Gew.-%, bevorzugt 10 bis 60 Gew.-% und insbesondere 20 bis 50 Gew.-% beträgt.

6. Reinigungsmittelangebotsform, umfassend
a) eine flüssige (20°C) Reinigungsmittelzubereitung A, enthaltend mindestens einen Gerüststoff;
b) eine flüssige (20°C) von der Reinigungsmittelzubereitung A verschiedene Reinigungsmittelzubereitung B nach einem der Ansprüche 1 bis 5, welche zusätzlich
b2) optional ein Tensid, bevorzugt ein nichtionisches Tensid, und
b3) optional 2,5 Gew.-% oder weniger Komplexbildner enthält,
c) optional eine flüssige (20°C) von den Reinigungsmittelzubereitungen A und B verschiedene Reinigungsmittelzubereitung C, enthaltend
c1) ein Acidifizierungsmittel,
c2) optional einen Glaskorrosionsinhibitor,
c3) optional ein nichtionisches Tensid,
c4) optional ein Hydrotrop, und
c5) optional weniger als 1 Gew.-%, bevorzugt weniger als 0,5 Gew.-%, insbesondere weniger als 0,1 Gew.-% Enzymzubereitung, und
d) ein Verpackungsmittel, in welchem die Reinigungsmittelzubereitungen A, B und ggf. C getrennt voneinander vorliegen.

7. Reinigungsmittelangebotsform nach Anspruch 6, **dadurch gekennzeichnet, dass** die Reinigungsmittelzubereitung A, bezogen auf ihr Gesamtgewicht, 2 bis 50 Gew.-%, vorzugsweise 6 bis 45 Gew.-% und insbesondere 10 bis 40 Gew.-% Gerüststoff enthält und/oder der Gerüststoff a1) ausgewählt ist aus der Gruppe der Carbonate, der Hydrogencarbonate, der Citrate, der Silikate, der polymeren Carboxylate und der Sulfonsäuregruppen-haltige Polymere.

8. Reinigungsmittelangebotsform nach einem der Ansprüche 6 oder 7, **dadurch gekennzeichnet, dass** die Reinigungsmittelzubereitung A, jeweils bezogen auf ihr Gesamtgewicht, Citrat in Mengen von 3 bis 25 Gew%, insbesondere von 4 bis 20 Gew.-% sowie Carbonat in Mengen von 4 bis 25 Gew.-%, insbesondere 6 bis 20 Gew.-% enthält.

9. Reinigungsmittelangebotsform nach einem der Ansprüche 6 bis 8, **dadurch gekennzeichnet, dass** als Komplexbilder ein oder mehrere Phosphonate, bevorzugt in einer Menge von 1,2 bis 8 Gew.-%, insbesondere 1,5 bis 6 Gew.-%, eingesetzt werden.

10. Reinigungsmittelangebotsform nach einem der Ansprüche 6 bis 9, **dadurch gekennzeichnet, dass** die Reinigungsmittelzubereitung C das Acidifizierungsmittel c1) ausgewählt aus Ameisensäure, Weinsäure, Bernsteinsäure, Malonsäure, Adipinsäure, Maleinsäure, Fumarsäure, Oxalsäure und/oder Polyacrylsäure, insbesondere Ameisensäure, Essigsäure und/oder Citronensäure, und/oder die Reinigungsmittelzubereitung C das Acidifizierungsmittel c1) bevorzugt in Mengen von 0,1 bis 12 Gew.-%, besonders bevorzugt 0,2 bis 10 Gew.-% und insbesondere 0,3 bis 8,0 Gew.-%, jeweils bezogen auf das Gesamtgewicht der Reinigungsmittelzubereitung C, enthält.

11. Reinigungsmittelangebotsform nach einem der Ansprüche 6 bis 10, **dadurch gekennzeichnet, dass** Tenside in der Reinigungsmittelzubereitung B in einer Menge von 4 bis 35 Gew.-%, insbesondere 8 bis 25 Gew.-%, und in Reinigungsmittelzubereitung C in einer Menge von 4,0 bis 15,0 Gew.-%, insbesondere 6 bis 12,0 Gew.-%, bezogen auf das Gesamtgewicht der jeweiligen Zubereitung, enthalten sind.

12. Reinigungsmittelangebotsform nach einem der Ansprüche 6 bis 11, **dadurch gekennzeichnet, dass** die Reinigungsmittelzubereitung C der Glaskorrosionsinhibitor c2) ausgewählt ist aus Polyalkyleniminien, insbesondere Polyethyleniminen, und/oder wasserlöslichen Zinksalzen, bevorzugt Zinkchlorid, Zinksulfat und/oder Zinkacetat, besonders bevorzugt Zinkacetat, und/oder die Reinigungsmittelzubereitung C den Glaskorrosionsinhibitor c2) bevorzugt in Mengen von 0,01 Gew.-% bis 5 Gew.-%, besonders bevorzugt von 0,05 Gew.-% bis 3 Gew.-%, insbesondere von 0,1 Gew.-% bis 2 Gew.-%, bezogen auf das Gesamtgewicht der Reinigungsmittelzubereitung C, enthält.

13. Reinigungsmittelangebotsform nach einem der Ansprüche 6 bis 12, **dadurch gekennzeichnet, dass** die Reinigungsmittelzubereitung C das Hydrotrop c3) ausgewählt ist aus Xylolsulfonat, Cumolsulfonat, Harnstoff und/oder *N*-Methylacetamid, besonders bevorzugt Cumolsulfonat und/oder Xylolsulfonat, insbesondere Cumolsulfonat, und/oder die Reinigungsmittelzubereitung C das Hydrotrop c3) in einer Menge von 2 bis 25 Gew.-%, insbesondere von 4 bis 20 Gew.-% und besonders bevorzugt in einer Menge von 6 bis 15, beispielsweise von 7 bis 12 Gew.-%, bezogen auf das Gesamtgewicht der Reinigungsmittelzubereitung C, enthält.

14. Verwendung einer Reinigungsmittelangebotsform nach einem der Ansprüche 6 bis 13 als Reinigungsmittelreservoir für
i) ein unbeweglich in den Innenraum einer Geschirrspülmaschine integriertes Dosiergerät oder
ii) ein für die Positionierung im Innenraum einer vorgesehenes bewegliches Dosiergerät.

15. Verwendung einer Reinigungsmittelangebotsform nach einem der Ansprüche 6 bis 13 zur Befüllung
i) einer unbeweglich in den Innenraum einer Geschirrspülmaschine integrierten Kartusche eines Dosiersystems oder
ii) einer für die Positionierung im Innenraum einer Geschirrspülmaschine vorgesehenen beweglichen Kartusche eines Dosiersystems
mit einer für die mindestens zweimalige, vorzugsweise mindestens viermalige und insbesondere mindestens achtmalige Durchführung eines maschinellen Geschirrspülverfahrens bzw. maschinellen Textilwaschverfahrens ausreichenden Menge dieser Reinigungsmittelangebotsform.

16. Reinigungsmitteldosiersystem, umfassend
a) eine Reinigungsmittelangebotsform nach einem der Ansprüche 6 bis 13, umfassend eine für die mindestens zweimalige, vorzugsweise mindestens viermalige und insbesondere mindestens achtmalige Durchführung eines maschinellen Geschirrspülverfahrens ausreichende Menge an Reinigungsmittelzubereitungen A und B bzw. A, B und C;
b) ein mit der Reinigungsmittelangebotsform lösbar verbundenes Dosiergerät.

17. Verwendung einer Reinigungsmittelangebotsform nach einem der Ansprüche 6 bis 13, oder eines Reinigungsmitteldosiersystems nach Anspruch 16 zur Geschirrreinigung in einem maschinellen Geschirrspülverfahren.

18. Maschinelles Geschirrspülverfahren unter Einsatz einer Reinigungsmittelangebotsform nach einem der Ansprüche 6 bis 13, oder eines Reinigungsmitteldosiersystems nach Anspruch 16, in dessen Verlauf aus einer im Innenraum der Geschirrspülmaschine befindlichen Kartusche
- eine Teilmenge a der in der Kartusche befindlichen Reinigungsmittelzubereitung A in den Innenraum der Geschirrspülmaschine dosiert wird, wobei eine Restmenge der in der Kartusche befindlichen Reinigungsmittelzubereitung bis zum Ende des Geschirrspülverfahrens in der Kartusche verbleibt, **dadurch gekennzeichnet, dass** diese Restmenge mindestens der doppelten, vorzugsweise mindestens der vierfachen und insbesondere mindestens der achtfachen Menge der Teilmenge a entspricht; und
- eine Teilmenge b der in der Kartusche befindlichen Reinigungsmittelzubereitung B in den Innenraum der Geschirrspülmaschine dosiert wird, wobei eine Restmenge der in der Kartusche befindlichen Reinigungsmittelzubereitung bis zum Ende des Geschirrspülverfahrens in der Kartusche verbleibt, **dadurch gekennzeichnet, dass** diese Restmenge mindestens der doppelten, vorzugsweise mindestens der vierfachen und insbesondere mindestens der achtfachen Menge der Teilmenge b entspricht; und
- gegebenenfalls eine Teilmenge c der in der Kartusche gegebenenfalls befindlichen Reinigungsmittelzubereitung C in den Innenraum der Geschirrspülmaschine dosiert wird, wobei eine Restmenge der in der Kartusche befindlichen Reinigungsmittelzubereitung bis zum Ende des Geschirrspülverfahrens in der Kartusche verbleibt, **dadurch gekennzeichnet, dass** diese Restmenge mindestens der doppelten, vorzugsweise mindestens der vierfachen und insbesondere mindestens der achtfachen Menge der Teilmenge c entspricht.


SEQUENCE LISTING

<110>  Henkel AG & Co. KGaA

<120>  Reinigungsmittel mit Protease zur automatischen Dosierung

<130>  2019P00304EP

<160>  7

<170>  PatentIn version 3.5

<210>  1
<211>  269
<212>  PRT
<213>  Bacillus gibsonii

<400>  1

Gln Gln Thr Val Pro Trp Gly Ile Thr Arg Val Gln Ala Pro Thr Val
1               5                   10                  15

His Asn Arg Gly Ile Thr Gly Ser Gly Val Lys Val Ala Ile Leu Asp
            20                  25                  30

Thr Gly Ile Ala Gln His Ser Asp Leu Thr Ile Arg Gly Gly Ala Ser
        35                  40                  45

Phe Val Pro Gly Glu Ser Thr Thr Ala Asp Leu Asn Gly His Gly Thr
    50                  55                  60

His Val Ala Gly Thr Val Ala Ala Leu Asn Asn Ser Ile Gly Val Ile
65                  70                  75                  80

Gly Val Ala Pro Ser Ala Asp Leu Tyr Ala Val Lys Val Leu Gly Ala
                85                  90                  95

Asn Gly Arg Gly Ser Val Ser Gly Ile Ala Gln Gly Leu Glu Trp Ala
            100                 105                 110

Ala Thr Asn Asn Met His Ile Ala Asn Met Ser Leu Gly Ser Asp Ala
        115                 120                 125

Pro Ser Thr Thr Leu Glu Arg Ala Val Asn Tyr Ala Thr Ser Arg Gly
    130                 135                 140

Val Leu Val Ile Ala Ala Thr Gly Asn Asn Gly Thr Gly Ser Ile Gly
145                 150                 155                 160

Tyr Pro Ala Arg Tyr Ala Asn Ala Met Ala Val Gly Ala Thr Asp Gln
                165                 170                 175

Asn Asn Arg Arg Ala Ser Phe Ser Gln Tyr Gly Thr Gly Ile Asp Ile
            180                 185                 190

Val Ala Pro Gly Val Gly Ile Gln Ser Thr Tyr Leu Asn Asn Ser Tyr
        195                 200                 205

Ala Ser Met Pro Gly Thr Ser Met Ala Thr Pro His Val Ala Gly Val
    210                 215                 220

Ala Ala Leu Val Lys Gln Lys Asn Pro Ser Trp Asn Ala Thr Gln Ile
225                 230                 235                 240

Arg Asn His Leu Lys Asn Thr Ala Thr Asn Leu Gly Asn Ser Ser Gln
                245                 250                 255

Phe Gly Ser Gly Leu Val Asn Ala Asp Ala Ala Thr Arg
            260                 265

<210>  2
<211>  269
<212>  PRT
<213>  Bacillus lentus

<400>  2

Ala Gln Ser Val Pro Trp Gly Ile Ser Arg Val Gln Ala Pro Ala Ala
1               5                   10                  15

His Asn Arg Gly Leu Thr Gly Ser Gly Val Lys Val Ala Val Leu Asp
            20                  25                  30

Thr Gly Ile Ser Thr His Pro Asp Leu Asn Ile Arg Gly Gly Ala Ser
        35                  40                  45

Phe Val Pro Gly Glu Pro Ser Thr Gln Asp Gly Asn Gly His Gly Thr
    50                  55                  60

His Val Ala Gly Thr Ile Ala Ala Leu Asn Asn Ser Ile Gly Val Leu
65                  70                  75                  80

Gly Val Ala Pro Ser Ala Glu Leu Tyr Ala Val Lys Val Leu Gly Ala
                85                  90                  95

Ser Gly Ser Gly Ser Val Ser Ser Ile Ala Gln Gly Leu Glu Trp Ala
            100                 105                 110

Gly Asn Asn Gly Met His Val Ala Asn Leu Ser Leu Gly Ser Pro Ser
        115                 120                 125

Pro Ser Ala Thr Leu Glu Gln Ala Val Asn Ser Ala Thr Ser Arg Gly
    130                 135                 140

Val Leu Val Val Ala Ala Ser Gly Asn Ser Gly Ala Gly Ser Ile Ser
145                 150                 155                 160

Tyr Pro Ala Arg Tyr Ala Asn Ala Met Ala Val Gly Ala Thr Asp Gln
                165                 170                 175

Asn Asn Asn Arg Ala Ser Phe Ser Gln Tyr Gly Ala Gly Leu Asp Ile
            180                 185                 190

Val Ala Pro Gly Val Asn Val Gln Ser Thr Tyr Pro Gly Ser Thr Tyr
        195                 200                 205

Ala Ser Leu Asn Gly Thr Ser Met Ala Thr Pro His Val Ala Gly Ala
    210                 215                 220

Ala Ala Leu Val Lys Gln Lys Asn Pro Ser Trp Ser Asn Val Gln Ile
225                 230                 235                 240

Arg Asn His Leu Lys Asn Thr Ala Thr Ser Leu Gly Ser Thr Asn Leu
                245                 250                 255

Tyr Gly Ser Gly Leu Val Asn Ala Glu Ala Ala Thr Arg
            260                 265

<210>  3
<211>  269
<212>  PRT
<213>  Bacillus alkalophilus

<400>  3

Ala Gln Ser Val Pro Trp Gly Ile Ser Arg Val Gln Ala Pro Ala Ala
1               5                   10                  15

His Asn Arg Gly Leu Thr Gly Ser Gly Val Lys Val Ala Val Leu Asp
            20                  25                  30

Thr Gly Ile Ser Thr His Pro Asp Leu Asn Ile Arg Gly Gly Ala Ser
        35                  40                  45

Phe Val Pro Gly Glu Pro Ser Thr Gln Asp Gly Asn Gly His Gly Thr
    50                  55                  60

His Val Ala Gly Thr Ile Ala Ala Leu Asn Asn Ser Ile Gly Val Leu
65                  70                  75                  80

Gly Val Ala Pro Asn Ala Glu Leu Tyr Ala Val Lys Val Leu Gly Ala
                85                  90                  95

Ser Gly Ser Gly Ser Val Ser Ser Ile Ala Gln Gly Leu Glu Trp Ala
            100                 105                 110

Gly Asn Asn Gly Met His Val Ala Asn Leu Ser Leu Gly Ser Pro Ser
        115                 120                 125

Pro Ser Ala Thr Leu Glu Gln Ala Val Asn Ser Ala Thr Ser Arg Gly
    130                 135                 140

Val Leu Val Val Ala Ala Ser Gly Asn Ser Gly Ala Gly Ser Ile Ser
145                 150                 155                 160

Tyr Pro Ala Arg Tyr Ala Asn Ala Met Ala Val Gly Ala Thr Asp Gln
                165                 170                 175

Asn Asn Asn Arg Ala Ser Phe Ser Gln Tyr Gly Ala Gly Leu Asp Ile
            180                 185                 190

Val Ala Pro Gly Val Asn Val Gln Ser Thr Tyr Pro Gly Ser Thr Tyr
        195                 200                 205

Ala Ser Leu Asn Gly Thr Ser Met Ala Thr Pro His Val Ala Gly Ala
    210                 215                 220

Ala Ala Leu Val Lys Gln Lys Asn Pro Ser Trp Ser Asn Val Gln Ile
225                 230                 235                 240

Arg Asn His Leu Lys Asn Thr Ala Thr Ser Leu Gly Ser Thr Asn Leu
                245                 250                 255

Tyr Gly Ser Gly Leu Val Asn Ala Glu Ala Ala Thr Arg
            260                 265

<210>  4
<211>  269
<212>  PRT
<213>  Bacillus lentus

<400>  4

Ala Gln Ser Val Pro Trp Gly Ile Ser Arg Val Gln Ala Pro Ala Ala
1               5                   10                  15

His Asn Arg Gly Leu Thr Gly Ser Gly Val Lys Val Ala Val Leu Asp
            20                  25                  30

Thr Gly Ile Ser Thr His Pro Asp Leu Asn Ile Arg Gly Gly Ala Ser
        35                  40                  45

Phe Val Pro Gly Glu Pro Ser Thr Gln Asp Gly Asn Gly His Gly Thr
    50                  55                  60

His Val Ala Gly Thr Ile Ala Ala Leu Asn Asn Ser Ile Gly Val Leu
65                  70                  75                  80

Gly Val Ala Pro Ser Ala Glu Leu Tyr Ala Val Lys Val Leu Gly Ala
                85                  90                  95

Asp Gly Arg Gly Ala Ile Ser Ser Ile Ala Gln Gly Leu Glu Trp Ala
            100                 105                 110

Gly Asn Asn Gly Met His Val Ala Asn Leu Ser Leu Gly Ser Pro Ser
        115                 120                 125

Pro Ser Ala Thr Leu Glu Gln Ala Val Asn Ser Ala Thr Ser Arg Gly
    130                 135                 140

Val Leu Val Val Ala Ala Ser Gly Asn Ser Gly Ala Ser Ser Ile Ser
145                 150                 155                 160

Tyr Pro Ala Arg Tyr Ala Asn Ala Met Ala Val Gly Ala Thr Asp Gln
                165                 170                 175

Asn Asn Asn Arg Ala Ser Phe Ser Gln Tyr Gly Ala Gly Leu Asp Ile
            180                 185                 190

Val Ala Pro Gly Val Asn Val Gln Ser Thr Tyr Pro Gly Ser Thr Tyr
        195                 200                 205

Ala Ser Leu Asn Gly Thr Ser Met Ala Thr Pro His Val Ala Gly Ala
    210                 215                 220

Ala Ala Leu Val Lys Gln Lys Asn Pro Ser Trp Ser Asn Val Gln Ile
225                 230                 235                 240

Arg Asn His Leu Lys Asn Thr Ala Thr Ser Leu Gly Ser Thr Asn Leu
                245                 250                 255

Tyr Gly Ser Gly Leu Val Asn Ala Glu Ala Ala Thr Arg
            260                 265

<210>  5
<211>  485
<212>  PRT

<213>  Bacillus sp.

<400>  5

His His Asn Gly Thr Asn Gly Thr Met Met Gln Tyr Phe Glu Trp Tyr
1               5                   10                  15

Leu Pro Asn Asp Gly Asn His Trp Asn Arg Leu Asn Ser Asp Ala Ser
            20                  25                  30

Asn Leu Lys Ser Lys Gly Ile Thr Ala Val Trp Ile Pro Pro Ala Trp
        35                  40                  45

Lys Gly Ala Ser Gln Asn Asp Val Gly Tyr Gly Ala Tyr Asp Leu Tyr
    50                  55                  60

Asp Leu Gly Glu Phe Asn Gln Lys Gly Thr Val Arg Thr Lys Tyr Gly
65                  70                  75                  80

Thr Arg Ser Gln Leu Gln Ala Ala Val Thr Ser Leu Lys Asn Asn Gly
                85                  90                  95

Ile Gln Val Tyr Gly Asp Val Val Met Asn His Lys Gly Gly Ala Asp
            100                 105                 110

Ala Thr Glu Met Val Arg Ala Val Glu Val Asn Pro Asn Asn Arg Asn
        115                 120                 125

Gln Glu Val Thr Gly Glu Tyr Thr Ile Glu Ala Trp Thr Arg Phe Asp
    130                 135                 140

Phe Pro Gly Arg Gly Asn Thr His Ser Ser Phe Lys Trp Arg Trp Tyr
145                 150                 155                 160

His Phe Asp Gly Val Asp Trp Asp Gln Ser Arg Arg Leu Asn Asn Arg
                165                 170                 175

Ile Tyr Lys Phe Arg Gly His Gly Lys Ala Trp Asp Trp Glu Val Asp
            180                 185                 190

Thr Glu Asn Gly Asn Tyr Asp Tyr Leu Met Tyr Ala Asp Ile Asp Met
        195                 200                 205

Asp His Pro Glu Val Val Asn Glu Leu Arg Asn Trp Gly Val Trp Tyr
    210                 215                 220

Thr Asn Thr Leu Gly Leu Asp Gly Phe Arg Ile Asp Ala Val Lys His
225                 230                 235                 240

Ile Lys Tyr Ser Phe Thr Arg Asp Trp Ile Asn His Val Arg Ser Ala
            245                 250                 255

Thr Gly Lys Asn Met Phe Ala Val Ala Glu Phe Trp Lys Asn Asp Leu
        260                 265                 270

Gly Ala Ile Glu Asn Tyr Leu Gln Lys Thr Asn Trp Asn His Ser Val
    275                 280                 285

Phe Asp Val Pro Leu His Tyr Asn Leu Tyr Asn Ala Ser Lys Ser Gly
    290                 295                 300

Gly Asn Tyr Asp Met Arg Asn Ile Phe Asn Gly Thr Val Val Gln Arg
305                 310                 315                 320

His Pro Ser His Ala Val Thr Phe Val Asp Asn His Asp Ser Gln Pro
            325                 330                 335

Glu Glu Ala Leu Glu Ser Phe Val Glu Glu Trp Phe Lys Pro Leu Ala
        340                 345                 350

Tyr Ala Leu Thr Leu Thr Arg Glu Gln Gly Tyr Pro Ser Val Phe Tyr
    355                 360                 365

Gly Asp Tyr Tyr Gly Ile Pro Thr His Gly Val Pro Ala Met Arg Ser
    370                 375                 380

Lys Ile Asp Pro Ile Leu Glu Ala Arg Gln Lys Tyr Ala Tyr Gly Lys
385                 390                 395                 400

Gln Asn Asp Tyr Leu Asp His His Asn Ile Ile Gly Trp Thr Arg Glu
            405                 410                 415

Gly Asn Thr Ala His Pro Asn Ser Gly Leu Ala Thr Ile Met Ser Asp
        420                 425                 430

Gly Ala Gly Gly Ser Lys Trp Met Phe Val Gly Arg Asn Lys Ala Gly
    435                 440                 445

Gln Val Trp Ser Asp Ile Thr Gly Asn Arg Thr Gly Thr Val Thr Ile
    450                 455                 460

Asn Ala Asp Gly Trp Gly Asn Phe Ser Val Asn Gly Gly Ser Val Ser
465                 470                 475                 480

Ile Trp Val Asn Lys
            485

<210>  6
<211>  485
<212>  PRT
<213>  Bacillus sp.

<400>  6

His His Asn Gly Thr Asn Gly Thr Met Met Gln Tyr Phe Glu Trp Tyr
1               5                   10                  15

Leu Pro Asn Asp Gly Asn His Trp Asn Arg Leu Arg Ser Asp Ala Ser
            20                  25                  30

Asn Leu Lys Asp Lys Gly Ile Ser Ala Val Trp Ile Pro Pro Ala Trp
        35                  40                  45

Lys Gly Ala Ser Gln Asn Asp Val Gly Tyr Gly Ala Tyr Asp Leu Tyr
    50                  55                  60

Asp Leu Gly Glu Phe Asn Gln Lys Gly Thr Ile Arg Thr Lys Tyr Gly
65                  70                  75                  80

Thr Arg Asn Gln Leu Gln Ala Ala Val Asn Ala Leu Lys Ser Asn Gly
                85                  90                  95

Ile Gln Val Tyr Gly Asp Val Val Met Asn His Lys Gly Gly Ala Asp
            100                 105                 110

Ala Thr Glu Met Val Arg Ala Val Glu Val Asn Pro Asn Asn Arg Asn
        115                 120                 125

Gln Glu Val Ser Gly Glu Tyr Thr Ile Glu Ala Trp Thr Lys Phe Asp
    130                 135                 140

Phe Pro Gly Arg Gly Asn Thr His Ser Asn Phe Lys Trp Arg Trp Tyr
145                 150                 155                 160

His Phe Asp Gly Val Asp Trp Asp Gln Ser Arg Lys Leu Asn Asn Arg
                165                 170                 175

Ile Tyr Lys Phe Arg Gly Asp Gly Lys Gly Trp Asp Trp Glu Val Asp
            180                 185                 190

Thr Glu Asn Gly Asn Tyr Asp Tyr Leu Met Tyr Ala Asp Ile Asp Met
        195                 200                 205

Asp His Pro Glu Val Val Asn Glu Leu Arg Asn Trp Gly Val Trp Tyr
    210                 215                 220

Thr Asn Thr Leu Gly Leu Asp Gly Phe Arg Ile Asp Ala Val Lys His
225                 230                 235                 240

Ile Lys Tyr Ser Phe Thr Arg Asp Trp Ile Asn His Val Arg Ser Ala
                245                 250                 255

Thr Gly Lys Asn Met Phe Ala Val Ala Glu Phe Trp Lys Asn Asp Leu
            260                 265                 270

Gly Ala Ile Glu Asn Tyr Leu Asn Lys Thr Asn Trp Asn His Ser Val
        275                 280                 285

Phe Asp Val Pro Leu His Tyr Asn Leu Tyr Asn Ala Ser Lys Ser Gly
    290                 295                 300

Gly Asn Tyr Asp Met Arg Gln Ile Phe Asn Gly Thr Val Val Gln Arg
305                 310                 315                 320

His Pro Met His Ala Val Thr Phe Val Asp Asn His Asp Ser Gln Pro
                325                 330                 335

Glu Glu Ala Leu Glu Ser Phe Val Glu Glu Trp Phe Lys Pro Leu Ala
            340                 345                 350

Tyr Ala Leu Thr Leu Thr Arg Glu Gln Gly Tyr Pro Ser Val Phe Tyr
        355                 360                 365

Gly Asp Tyr Tyr Gly Ile Pro Thr His Gly Val Pro Ala Met Lys Ser
    370                 375                 380

Lys Ile Asp Pro Ile Leu Glu Ala Arg Gln Lys Tyr Ala Tyr Gly Arg
385                 390                 395                 400

Gln Asn Asp Tyr Leu Asp His His Asn Ile Ile Gly Trp Thr Arg Glu
                405                 410                 415

Gly Asn Thr Ala His Pro Asn Ser Gly Leu Ala Thr Ile Met Ser Asp
            420                 425                 430

Gly Ala Gly Gly Asn Lys Trp Met Phe Val Gly Arg Asn Lys Ala Gly
        435                 440                 445

Gln Val Trp Thr Asp Ile Thr Gly Asn Arg Ala Gly Thr Val Thr Ile
    450                 455                 460

Asn Ala Asp Gly Trp Gly Asn Phe Ser Val Asn Gly Gly Ser Val Ser

465                 470                 475                 480

Ile Trp Val Asn Lys
                485

<210>  7
<211>  485
<212>  PRT
<213>  Bacillus sp.

<400>  7

His His Asn Gly Thr Asn Gly Thr Met Met Gln Tyr Phe Glu Trp His
1               5                   10                  15

Leu Pro Asn Asp Gly Asn His Trp Asn Arg Leu Arg Asp Asp Ala Ser
            20                  25                  30

Asn Leu Arg Asn Arg Gly Ile Thr Ala Ile Trp Ile Pro Pro Ala Trp
        35                  40                  45

Lys Gly Thr Ser Gln Asn Asp Val Gly Tyr Gly Ala Tyr Asp Leu Tyr
    50                  55                  60

Asp Leu Gly Glu Phe Asn Gln Lys Gly Thr Val Arg Thr Lys Tyr Gly
65                  70                  75                  80

Thr Arg Ser Gln Leu Glu Ser Ala Ile His Ala Leu Lys Asn Asn Gly
                85                  90                  95

Val Gln Val Tyr Gly Asp Val Val Met Asn His Lys Gly Gly Ala Asp
            100                 105                 110

Ala Thr Glu Asn Val Leu Ala Val Glu Val Asn Pro Asn Asn Arg Asn
        115                 120                 125

Gln Glu Ile Ser Gly Asp Tyr Thr Ile Glu Ala Trp Thr Lys Phe Asp
    130                 135                 140

Phe Pro Gly Arg Gly Asn Thr Tyr Ser Asp Phe Lys Trp Arg Trp Tyr
145                 150                 155                 160

His Phe Asp Gly Val Asp Trp Asp Gln Ser Arg Gln Phe Gln Asn Arg
                165                 170                 175

Ile Tyr Lys Phe Arg Gly Asp Gly Lys Ala Trp Asp Trp Glu Val Asp
            180                 185                 190

Ser Glu Asn Gly Asn Tyr Asp Tyr Leu Met Tyr Ala Asp Val Asp Met

        195                 200                 205

Asp His Pro Glu Val Val Asn Glu Leu Arg Arg Trp Gly Glu Trp Tyr
    210                 215                 220

Thr Asn Thr Leu Asn Leu Asp Gly Phe Arg Ile Asp Ala Val Lys His
225                 230                 235                 240

Ile Lys Tyr Ser Phe Thr Arg Asp Trp Leu Thr His Val Arg Asn Ala
                245                 250                 255

Thr Gly Lys Glu Met Phe Ala Val Ala Glu Phe Trp Lys Asn Asp Leu
            260                 265                 270

Gly Ala Leu Glu Asn Tyr Leu Asn Lys Thr Asn Trp Asn His Ser Val
        275                 280                 285

Phe Asp Val Pro Leu His Tyr Asn Leu Tyr Asn Ala Ser Asn Ser Gly
    290                 295                 300

Gly Asn Tyr Asp Met Ala Lys Leu Leu Asn Gly Thr Val Val Gln Lys
305                 310                 315                 320

His Pro Met His Ala Val Thr Phe Val Asp Asn His Asp Ser Gln Pro
                325                 330                 335

Gly Glu Ser Leu Glu Ser Phe Val Gln Glu Trp Phe Lys Pro Leu Ala
            340                 345                 350

Tyr Ala Leu Ile Leu Thr Arg Glu Gln Gly Tyr Pro Ser Val Phe Tyr
        355                 360                 365

Gly Asp Tyr Tyr Gly Ile Pro Thr His Ser Val Pro Ala Met Lys Ala
    370                 375                 380

Lys Ile Asp Pro Ile Leu Glu Ala Arg Gln Asn Phe Ala Tyr Gly Thr
385                 390                 395                 400

Gln His Asp Tyr Phe Asp His His Asn Ile Ile Gly Trp Thr Arg Glu
                405                 410                 415

Gly Asn Thr Thr His Pro Asn Ser Gly Leu Ala Thr Ile Met Ser Asp
            420                 425                 430

Gly Pro Gly Gly Glu Lys Trp Met Tyr Val Gly Gln Asn Lys Ala Gly
        435                 440                 445

Gln Val Trp His Asp Ile Thr Gly Asn Lys Pro Gly Thr Val Thr Ile
    450                 455                 460

Asn Ala Asp Gly Trp Ala Asn Phe Ser Val Asn Gly Gly Ser Val Ser
465                 470                 475                 480

Ile Trp Val Lys Arg
                485